# FASCICULE DE BREVET EUROPEEN

(11) **EP 0 761 672 B1**
(45) Date de publication et mention de la délivrance du brevet: **07.11.2001**
(21) Numéro de dépôt: 96401777.6
(22) Date de dépôt: 13.08.1996
(51) Int. Cl.: C07D 501/24, C07C 59/64, C07C 69/734, C07D 209/48, C07D 277/20, C07C 239/20, A61K 31/545

(54) **Nouvelles céphalosporines comportant en position 7 un radical benzyloxyimino substitué, leurs procédés et intermédiaires de préparation, leur application comme médicaments**
Cephalosporine die im Stellung 7 ein substituiertes Benzyloxyiminoradikal enthalten, Verfahren zu ihrer Herstellung und Zwischenverbindungen zu ihrer Herstellung, ihre Anwendung als Medikamente
Cephalosporins containing in position 7 a substituted benzyloxyimino radical, processes for their preparation and intermediates for their preparation, their use as medicaments

(30) Priorité: 16.08.1995 FR 9509822
(43) Date de publication de la demande: 12.03.1997
(73) Titulaire: Aventis Pharma S.A., 92160 Antony (FR)
(72) Inventeur: Aszodi, Jozsef, 77340 Pontault Combault (FR); Fauveau, Patrick, 93190 Livry Gargan (FR); Humbert, Daniel, 94120 Fontenay sous Bois (FR)

(56) Documents cités:
- EP-A- 0 462 009
- EP-A- 0 551 034
- FR-A- 2 684 995

## Description

La présente invention concerne de nouvelles céphalosporines comportant sur la chaîne latérale en position 7, un radical benzy loxyimino substitué, leurs procédé et intermédiaires de préparation, leur application comme médicaments, les compositions les renfermant et les nouveaux intermédiaires obtenus.

Les documents EP-A-0462 009 et 0551 034 décrivent des composés antibiotiques de type céphalosporines, dont la chaîne latérale en position 7 comporte une fonction benzyloxyimino substituée sur le phényle par deux hydroxy ou des substituants choisis notamment parmi les radicaux hydroxy, halogéno, cyano, alkoxy ou encore nitro, et sur le carbone benzylique par un radical carboxy, en général libre ou salifié.

La présente invention concerne des composés qui diffèrent de ceux décrits dans l'art antérieur en général, en ce sens que le carbone benzylique n'est pas substitué par un radical carboxy.

L'invention a pour objet les produits de formule générale (I) : 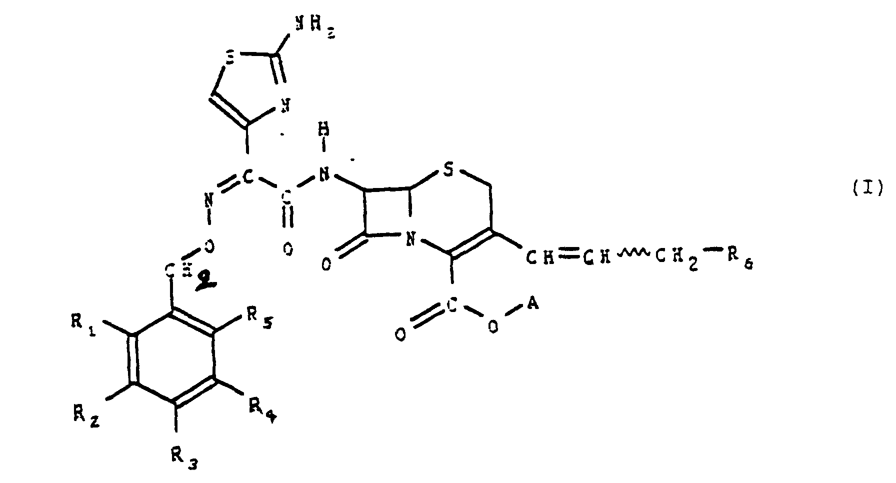 isomère syn, sous forme de sels internes ou de sels avec les acides minéraux ou organiques ou avec les bases, formule dans laquelle : R₁, R₂, R₃ et R₅, identiques ou différents, représentent un atome d'hydrogène, un atome d'halogène ou un radical choisi dans le groupe constitué par les radicaux hydroxy, alkyle renfermant de 1 à 4 atomes de carbone éventuellement substitué par un ou plusieurs atomes d'halogène, alkyloxy renfermant de 1 à 4 atomes de carbone, mercapto, alkylthio renfermant de 1 à 4 atomes de carbone, nitro, cyano, amino, alkylamino renfermant de 1 à 4 atomes de carbone, dialkylamino renfermant de 2 à 8 atomes de carbone, carbamoyle, (alkylamino) carbonyle renfermant de 2 à 5 atomes de carbone, (dialkylamino) carbonyle renfermant de 3 à 9 atomes de carbone, carboxy, alcoxycarbonyle renfermant de 2 à 5 atomes de carbone, acyloxy renfermant de 1 à 8 atomes de carbone et 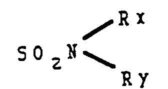 dans lequel Rx et Ry identiques ou différents représentent un atome d'hydrogène ou un radical alkyle renfermant de 1 à 4 atomes de carbone,
R₄ représente un radical hydroxy ou un radical acyloxy renfermant de 1 à 8 atomes de carbone,
A représente un atome d'hydrogène, un équivalent de métal alcalin, alcalino-terreux, de magnésium, d'ammonium ou d'une base organique aminée, ou A représente le reste d'un groupement ester facilement clivable, ou bien CO₂A représente CO₂^{^{⊖}}, le trait ondulé signifie que le groupement CH₂R₆ peut se trouver dans la position E ou Z et R₆ représente, sous forme d'ammonium quaternaire, un des radicaux suivants : 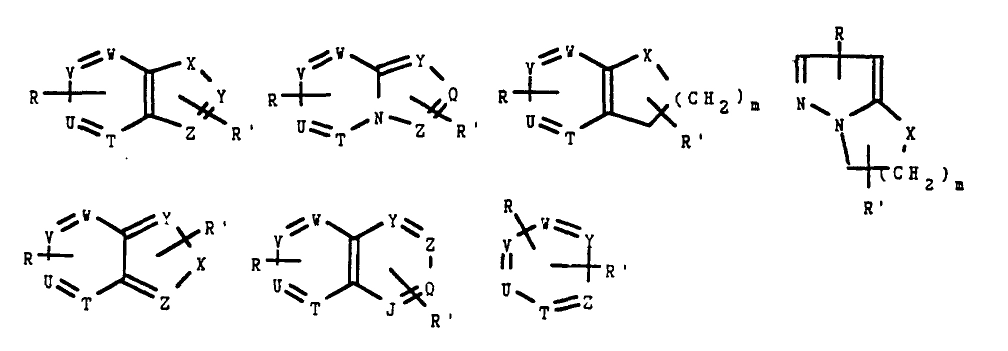 dans lesquels m est égal à 1, 2 ou 3, X représente CH₂, NH, O ou S ;
Q, J, Y, T, U, V, W et Z, identiques ou différents, représentent indépendamment les uns des autres CH ou N, étant entendu que chacun de ces radicaux cycliques contient de 1 à 5 hétéroatomes, qu'au moins un de ces hétéroatomes est l'atome d'azote et que ces radicaux cycliques peuvent être substitués par un ou plusieurs radicaux R ou R' ;
R et R', identiques ou différents, représentent un atome d'hydrogène, un radical alkyle renfermant de 1 à 4 atomes de carbone, un radical alkyloxy renfermant de 1 à 4 atomes de carbone, un atome d'halogène, un radical cyano, un radical CO₂-Q₁, CO-NQ₁(Q₂), NQ₁(Q₂), SO₂-NQ₁(Q₂), CS-NH₂, NH-CO-Q₁, CH=N-OH, CH=N-O-Q₁, CH₂-CN, CH₂-S-Q₁, S-Q₁, dans lesquels Q₁ et Q₂, identiques ou différents, représentent un atome d'hydrogène ou un radical alkyle renfermant de 1 à 4 atomes de carbone.

Par radical alkyle renfermant de 1 à 4 atomes de carbone, on entend le radical méthyle, éthyle, propyle, isopropyle, butyle, isobutyle, sec-butyle ou tert-butyle.

Par radical alkyloxy renfermant de 1 à 3 et 1 à atomes de carbone, on entend le radical méthoxy, éthoxy, propoxy, isopropoxy, ainsi que butoxy, isobutoxy, sec-butoxy ou tert-butoxy.

Par radical alkylthio renfermant de 1 à 4 atomes de carbone, on entend le radical méthylthio, éthylthio, propylthio, isopropylthio, butylthio, isobutylthio, sec-butylthio ou tert-butylthio.

Par radical alkylamino renfermant de 1 à 4 atomes de carbone, on entend le radical méthylamino, éthylamino, propylamino, isopropylamino, butylamino, isobutylamino, sec-butylamino ou tert-butylamino.

Par radical dialkylamino renfermant de 2 à 8 atomes de carbone, on entend notamment le radical diméthylamino, diéthylamino, dipropylamino, diisopropylamino, dibutylamino, diisobutylamino, éthyl méthylamino, propyl méthylamino, butyl méthylamino ou propyl éthylamino.

Par radical (alkylamino) carbonyle renfermant de 2 à 5 atomes de carbone, on entend notamment le radical (méthylamino) carbonyle, (éthylamino) carbonyle, (propylamino) carbonyle, (isopropylamino) carbonyle ou (butylamino) carbonyle.

Par radical (dialkylamino) carbonyle renfermant de 3 à 9 atomes de carbone, on entend notamment le radical (diméthylamino) carbonyle, (diéthylamino) carbonyle ou (dipropylamino) carbonyle.

Par radical alkoxycarbonyle renfermant de 2 à 5 atomes de carbone, on entend notamment le radical méthoxycarbonyle ou éthoxycarbonyle.

Par radical acyloxy renfermant de 1 à 8 atomes de carbone, on entend notamment le radical acétoxy, propionyloxy ou benzoyloxy.

Par atome d'halogène, on entend un atome de fluor, chlore, brome ou iode.

Lorsque R₄ représente un radical acyloxy, il s'agit notamment du radical acétoxy, propionyloxy ou benzoyloxy. Parmi les valeurs de A on peut citer un équivalent de sodium, de potassium, de lithium, de calcium, de magnésium ou d'ammonium. On peut citer encore un équivalent d'une base organique, par exemple la méthylamine, la propylamine, la triméthylamine, la diéthylamine, la triéthylamine, la N,N-diméthyléthanolamine, le tris[(hydroxyméthyl) amino] méthane, l'éthanolamine, la pyridine, la picoline, la dicyclohexylamine, la morpholine, la benzylamine, la procaine, la lysine, l'arginine, l'histidine, la N-méthylglucamine.

Les produits de formule (I) peuvent également se présenter sous forme de sel interne pur, sous forme salifiée ou sous forme associée aux acides de la solution.

Parmi les acides avec lesquels on peut salifier les produits de formule (I), on peut citer entre autres, les acides acétique, trifluoroacétique, fumarique, maléïque, tartrique, méthanesulfonique, benzènesulfonique, paratoluènesulfonique, phosphorique, sulfurique, chlorhydrique, bromhydrique, iodhydrique.

Parmi les bases avec lesquelles on peut salifier les produits de formule (I), on peut citer, entre autres, les bases correspondant aux sels mentionnés plus haut. Des exemples de salification par les bases sont fournis plus loin. Dans un mode préféré de l'invention CO₂A représente CO₂^{⊖}.

L'expression "sous forme d'ammonium quaternaire" indique que le radical R₆ est lié par le ou l'un des atomes d'azote qu'il comporte.

L'invention a particulièrement pour objet les produits de formule générale (I) telle que définie ci-dessus, dans laquelle R₆ représente un des radicaux suivants :

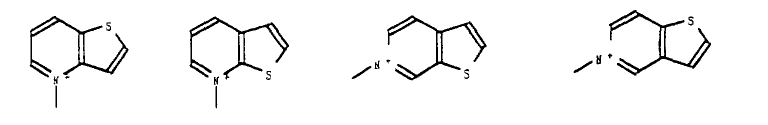

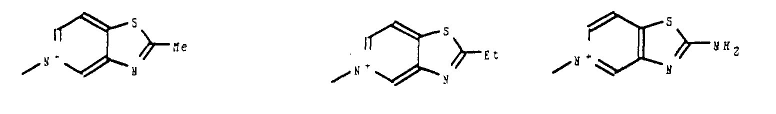

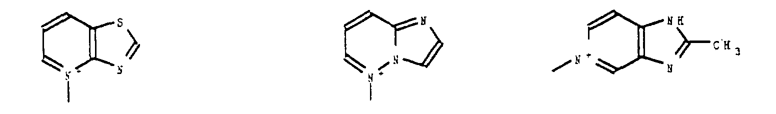

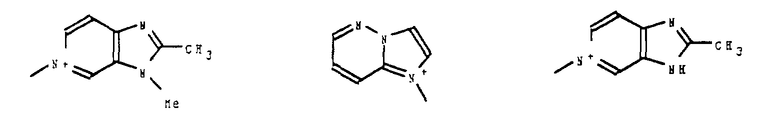

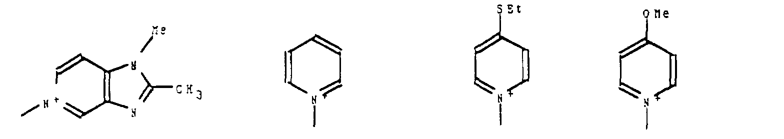

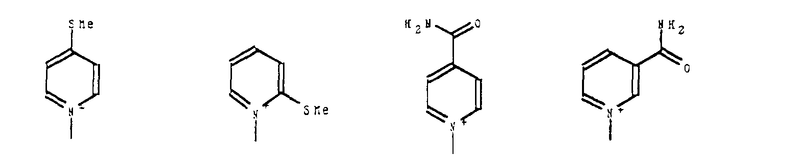

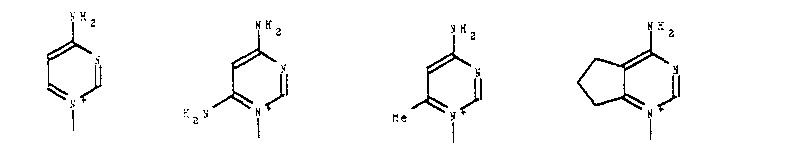

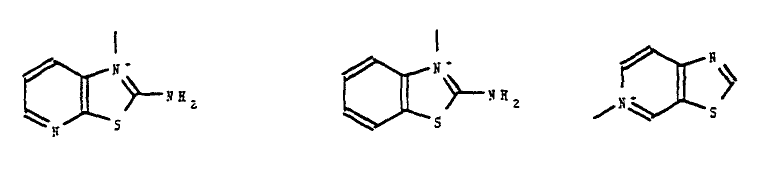

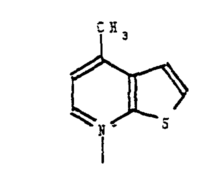

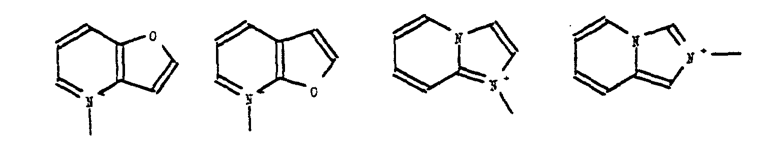

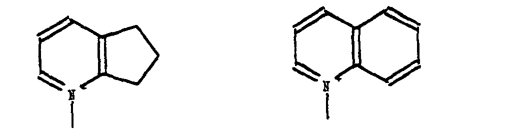

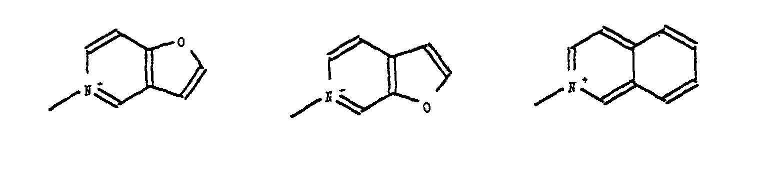

L'invention a plus particulièrement pour objet les produits de formule générale (I) telle que définie ci-dessus dans laquelle R₆ représente le radical quinoléinium, isoquinoléinium, 4-(méthylthio) pyridinium, thièno[2,3-b]pyridinium, imidazo(1,2-a)pyridinium ou le radical 6,7-dihydro-5H-1-pyrindinium, ceux dans laquelle R₃ et R₄ représentent chacun un radical hydroxy, ceux dans laquelle R₂ et R₅ représentent chacun un atome de chlore ou de fluor, ceux dans lesquels R₂ représente un atome de fluor et ceux dans lesquels R₂ représente un radical méthoxy et l'un de R₁ ou R₅ représente un atome de chlore.

L'invention a tout particulièrement pour objet les produits dont les noms suivent :
- le sel interne de (6R-(3-(E) 6alpha, 7béta-(Z))) 1-(3-(7-(((2-amino 4-thiazolyl) ((2,5-difluoro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium,
- le sel interne de (6R-(3-(E) 6alpha, 7béta-(Z))) 1-(3-(7-(((2-amino 4-thiazolyl) ((2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium,
- le sel interne de (6R-(3-(E) 6alpha, 7béta-(Z))) 1-(3-(7-(((2-amino 4-thiazolyl) ((2,5-difluoro 3,4-dihydroxyphényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium,
- le sel interne de (6R-(3-(E) 6alpha, 7béta-(Z))) 1-(3-(7-(((2-amino 4-thiazolyl) (((2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium,
- le sel interne de (6R-(3-(E) 6alpha, 7béta-(Z))) 1-(3-(7(((2-amino 4-thiazolyl) (((2,5-difluoro 3,4-dihydroxyphényl) méthoxy) imino) acétyl) amino) 2-carboxy 8-oxo 5-thia 1-azabicyclo[4,2,0]oct-2-èn-3-yl) 2-propényl) 6,7-dihydro 5H-1-pyrindinium,
- le sel interne de (6R-(3(E), 6alpha, 7béta(Z))) 1-(3-(7-(((2-amino 4-thiazolyl) ((2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) 6,7-dihydro 5H-1-pyrindinium,
- le sel interne de (6R-(3(E), 6alpha, 7béta(Z))) 1-(3-(7-(((2-amino 4-thiazolyl) ((5-fluoro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium.

L'invention a également pour objet un procédé de préparation des produits de-formule (I) telle que définie ci-dessus, caractérisé en ce que l'aldéhyde aromatique de formule (II) : 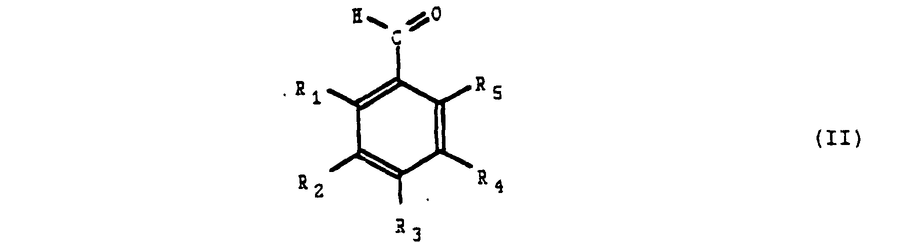 dans laquelle R₁, R₂, R₃, R₄ et R₅ sont tels que définis ci-dessus, est si nécessaire protégé en ses fonctions réactives et transformé ainsi en aldéhyde aromatique de formule (IIₚ) : 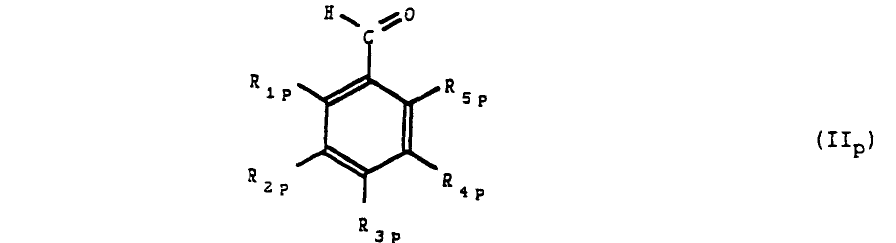 dans laquelle R₁ₚ, R₂ₚ, R₃ₚ, R₄ₚ et R₅ₚ représentent respectivement les valeurs de R₁, R₂, R₃, R₄ et R₅ telles que définies précédemment ou une fonction réactive protégée, ledit aldéhyde de formule (IIₚ) est traité par un agent réducteur, pour obtenir l'alcool de formule (III) : 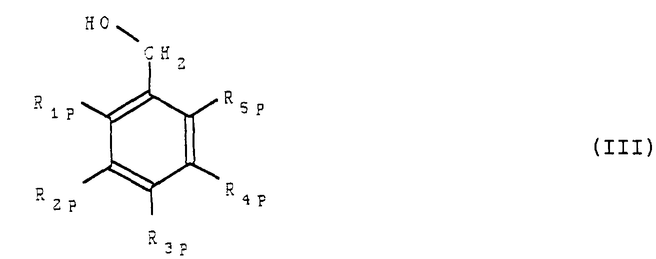 alcool de formule (III) que l'on traite par le N-hydroxy phtalimide, le cas échéant en présence d'un agent activant, pour obtenir le dérivé de formule (IV) : 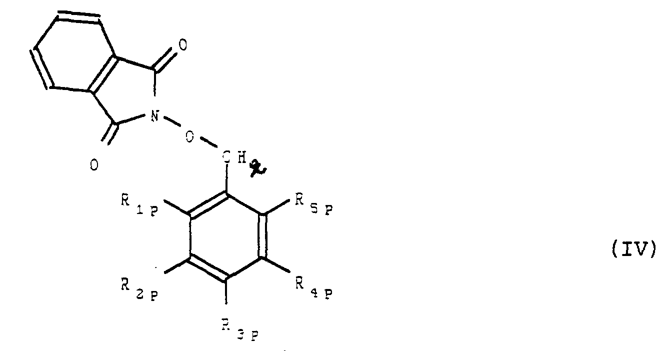 que l'on hydrolyse en hydroxylamine O-substituée de formule (V) : 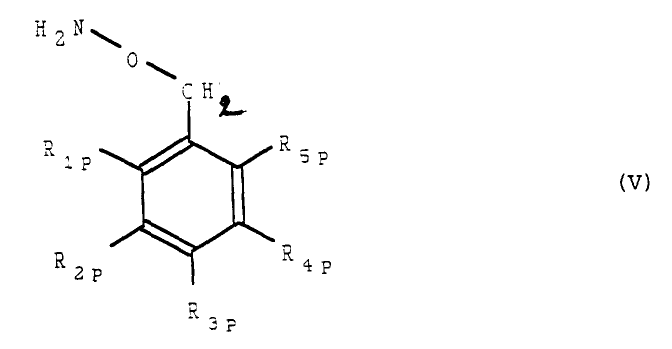 que l'on condense avec un dérivé de l'acide 2-(2-amino thiazol-4-yl) 2-oxo acétique de formule (VI) : 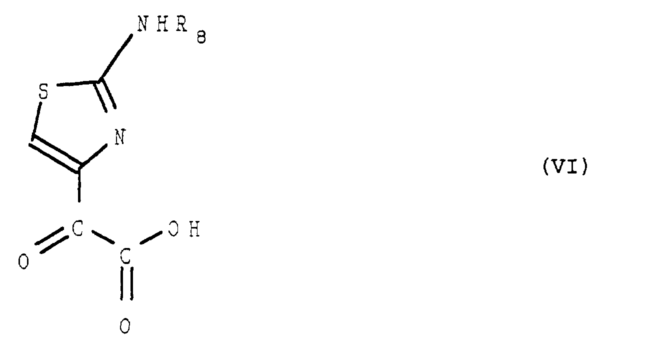 dans laquelle R₈ représente un atome d'hydrogène ou un groupe protecteur de la fonction amine, pour former le dérivé de l'acide alpha-alkoxyimino acétique "syn" de formule (VII) : 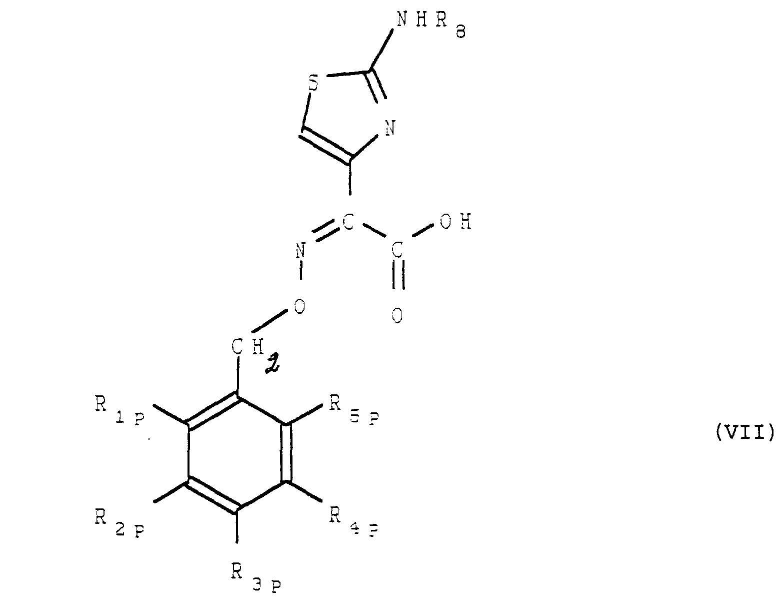 dont on prépare le cas échéant, un dérivé fonctionnel, produit de formule (VII) ou dérivé fontionnel que l'on amidifie avec un ester du chlorhydrate de l'acide 7-amino 3-(3-halo 1-propényl) 8-oxo 5-thia 1-azabicyclo[4,2,0]oct-2-èn-2-carboxylique de formule (VIII) : 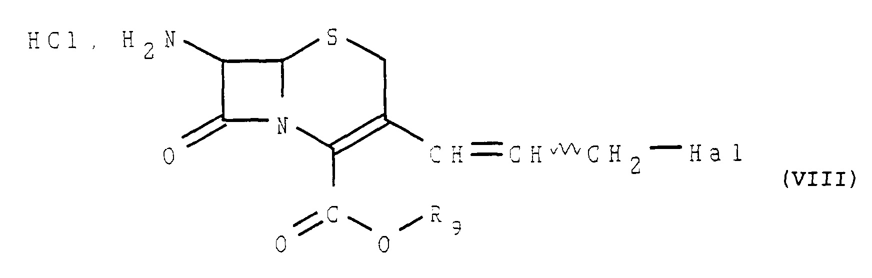 dans laquelle R₉ représente le reste d'un ester aisément clivable, pour conduire au dérivé de l'acide 7-(N-substitué amido) 3-(3-halo 1-propényl) 8-oxo 5-thia 1-azabicyclo-[4,2,0]oct-2-èn-2-carboxylique de formule (IX) : 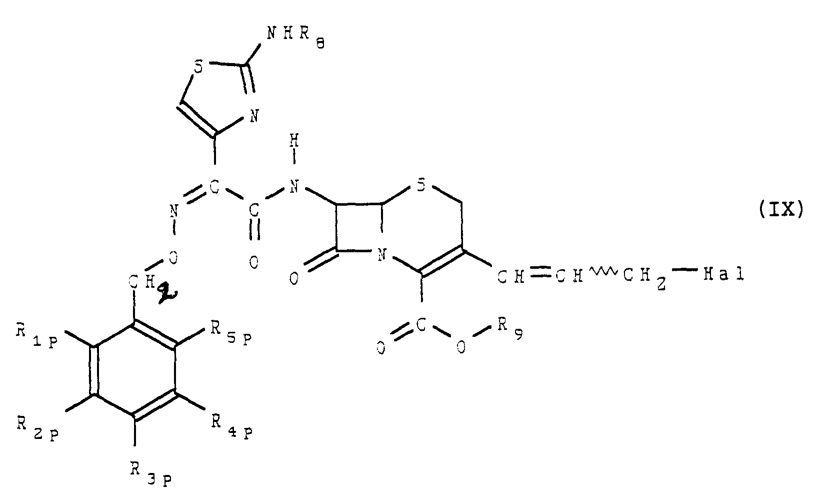 que l'on transforme, le cas échéant, en analogue 3-(3-iodo propényle) de formule (X) : 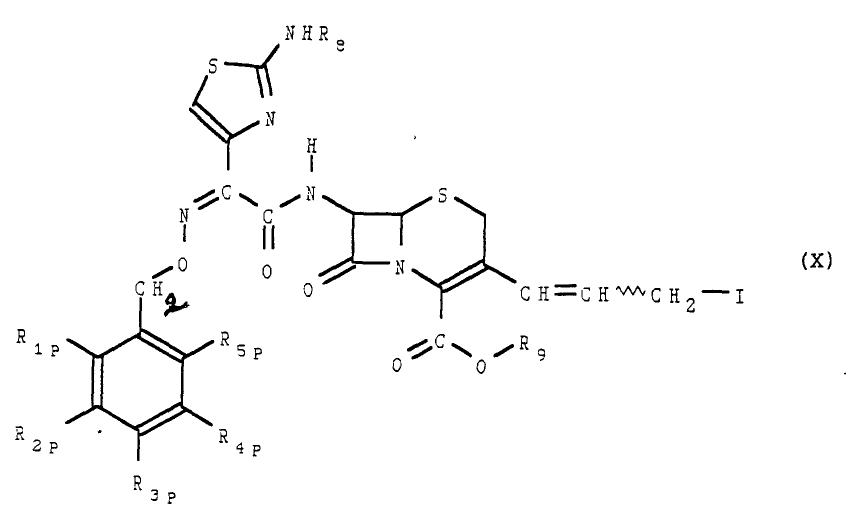 que l'on traite avec une base de formule R₆ pour obtenir le produit de formule (XI) : 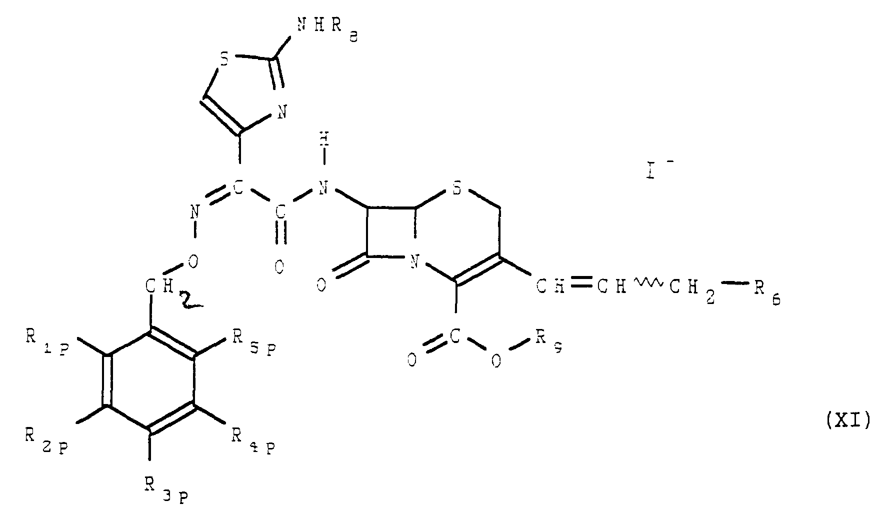 produit de formule (XI) à partir duquel le cas échéant, l'on isole les isomères (E) ou (Z) ou transforme les isomères (Z) en isomères (E), produit de formule (XI) que l'on soumet le cas échéant, à une ou plusieurs des réactions suivantes dans un ordre approprié :
a) coupure par hydrolyse ou par action de la thiourée de tout ou partie des groupements protecteurs esters et des groupements de protection du radical amino ou des radicaux hydroxyles,
b) estérification ou salification par une base du radical carboxylique,
c) salification par un acide du radical amino,
d) séparation des produits sous forme de mélange R,S en R et S.

L'invention a aussi pour objet une variante du procédé décrit ci-dessus, caractérisé en ce que l'hydroxylamine O-substituée de formule (V) est condensée au produit de formule (XII) : 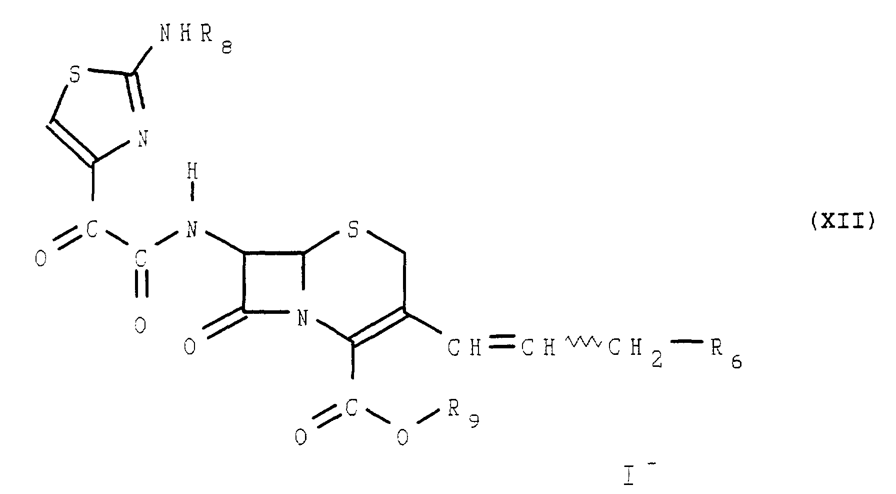 pour conduire au produit de formule (XI) telle que définie précédemment.

Les fonctions hydroxy protégées que peuvent représenter R₁ₚ, R₂ₚ, R₃ₚ, R₄ₚ et R₅ₚ, sont choisies parmi les groupes acyloxy tels que par exemple formyloxy, acétoxy, propionyloxy, chloroacétoxy, bromoacétoxy, dichloroacétoxy, trichloroacétoxy, trifluoroacétoxy, méthoxyacétoxy, phénoxyacétoxy, benzoyloxy, benzoylformoxy, p-nitro benzoyloxy. On peut citer également les groupements éthoxycarbonyloxy, méthoxycarbonyloxy, propoxycarbonyloxy, 2,2,2-trichloro éthoxycarbonyloxy, allyloxycarbonyloxy, triméthylsilyléthoxycarbonyloxy, benzyloxycarbonyloxy, tert-butoxycarbonyloxy, 1-cyclopropyl éthoxycarbonyloxy, phtaloyloxy, butyryloxy, isobutyryloxy, valéryloxy, isovaléryloxy, oxalyloxy, succinyloxy et pivaloyloxy, phénylacétoxy, phénylpropionyloxy, mésyloxy, chlorobenzoyloxy, para-nitrobenzoyloxy, para-tert-butyl benzoyloxy, caprylyloxy, acryloyloxy, méthylcarbamoyloxy, phénylcarbamoyloxy, naphtylcarbamoyloxy.

Elles peuvent encore être choisies parmi les radicaux phénoxy, 4-chloro phénoxy, tolyloxy ou tert-butyl phénoxy, tolylsulfonyloxy, tétrahydropyrannyloxy, tétrahydrothiopyrannyloxy, méthoxytétrahydropyrannyloxy, trityloxy, benzyloxy, 4-méthoxy benzyloxy, benzhydryloxy, trichloroéthoxy, 1-méthyl 1-méthoxyéthoxy, les radicaux alkoxy alkoxy-méthoxy tel que méthoxy éthoxy méthoxy ou encore les radicaux triméthylsilyléthoxyméthoxy ou triméthylsilyléthoxy.

Deux radicaux hydroxy adjacents peuvent encore être protégés en formant un radical méthylènedioxy, isopropylènedioxy, 1,1-cyclohexyl bis(oxy), diphénylméthylènedioxy, carbonate ou hydroxy borannylbis(oxy).

Les fonctions hydroxy protégées que peuvent représenter R₁ₚ, R₂ₚ, R₃ₚ, R₄ₚ et R₅ₚ, sont choisies de préférence parmi les groupes méthoxyéthoxyméthoxy, propionyloxyméthoxy, acétoxyméthoxy, butoxy, isobutoxy, tert-butoxy, pentoxy, hexyloxy, butyryloxyméthoxy, valéryloxyméthoxy, pivaloyloxyméthoxy, 2-acétoxy éthoxy, 2-propionyloxy éthoxy, 2-butyryloxy éthoxy, 2-iodoéthoxy, 2,2,2-trichloro éthoxy, vinyloxy, allyloxy, éthynyloxy, propynyloxy, benzyloxy, 4-méthoxy benzyloxy, 4-nitro benzyloxy, phényléthoxy, trityloxy, diphénylméthyloxy ou 3,4-diméthoxyphénoxy.

On préfère particulièrement le groupement 2-méthoxy éthoxyméthoxy (MEM-O).

Le reste de groupement ester facilement clivable que représente R₉ est choisi notamment parmi les groupements butyle, isobutyle, tert-butyle, pentyle, hexyle, méthoxyméthyle, éthoxyméthyle, isopropyloxyméthyle, alpha-méthoxy éthyle, alpha-éthoxy éthyle, méthylthiométhyle, éthylthiométhyle, isopropylthiométhyle, pivaloyloxyméthyle, acétoxyméthyle, propionyloxyméthyle, butyryloxyméthyle, isobutyryloxyméthyle, valéryloxyméthyle, isovaléryloxyméthyle, tert-butylcarbonyloxyméthyle, hexadécanoyloxyméthyle, pivaloyloxyméthyl, propionyloxyéthyle, isovaléryloxyéthyle, 1-acétoxy éthyle, 2-acétoxy éthyle, 1-propionyloxy éthyle, 2-propionyloxy éthyle, 1-butyryloxy éthyle, 2-butyryloxy éthyle, 1-(tert-butylcarbonyloxy) éthyle, 1-acétoxy propyle, 1-hexadécanoyloxy éthyle, 1-propionyloxy propyle, 1-méthoxycarbonyloxy éthyle, méthoxycarbonyloxyméthyle, 1-acétoxy butyle, 1-acétoxy hexyle, 1-acétoxy heptyle, phtalidyle, 5,6-diméthoxy phtalidyle, tert-butylcarbonylméthyle, vinyle, allyle, 2-chloro allyle, éthynyle, propynyle, méthoxycarbonylméthyle, benzyle, 4-méthoxy benzyle, 4-nitro benzyle, phénéthyle, trityle, diphényl méthyle, phényle, 4-chloro phényle, tolyle, tert-butyl phényle, 3,4-diméthoxy phényle, méthoxyéthoxyméthyle, diméthylaminoéthyle, cyanométhyle, tert-butoxycarbonylméthyle, 2,2-éthylènedioxy éthyle, cyanoéthyle, 2,2-diméthoxy éthyle, 2-chloro éthoxyméthyle, (2-hydroxy éthoxy) éthyle, 2,3-époxy propyle, 3-diméthylaminc 2-hydroxy propyle, 2-hydroxy éthyle, 2-méthylaminoéthoxyméthyle, (2-amino éthoxy) méthyle, 3-méthoxy 2,4-thiadiazol-5-yle, tétrahydropyrann-2-yle, 1-méthoxy 1-méthyl éthyle, 2-hydroxy 1-méthyl éthyle, isopropyle, carbamoylméthyle, chlorométhyle, 2-chloro éthyle, 2,2,2-trichloro éthyle, 2-iodo éthyle, acétyle, méthyle, 2-méthylthio éthyle, thiocyanatométhyle, 2-chloro 1-acétoxy éthyle, 2-bromo 1-acétoxy éthyle, 2-fluoro 1-acétoxy éthyle, 2-méthoxy 1-acétoxy éthyle, 2-méthyl 1-acétoxy propyle, 1-méthyl 1-acétoxy éthyle, 1-(méthoxyacétoxy) éthyle, 1-acétyl carbonyloxyéthyle, 1-hydroxy acétoxyéthyle, 1-(2-thiényl) carbonyloxyéthyle, 1-(2-furyl) carbonyloxyéthyle, 1-(5-nitro 2-furyl) carbonyloxyéthyle, 1-(2-pyrrolyl) carbonyloxyéthyle, 1-(propionyloxycarbonyloxy) éthyle, 1-(propoxycarbonyloxy) éthyle, 1-(isopropoxycarbonyloxy) éthyle, 1-(méthoxyéthoxycarbonyloxy) éthyle, 1-(allyloxycarbonyloxy) éthyle, isopropoxycarbonyl méthyle, 1-[(2,3-époxy propyl) oxycarbonyloxy] éthyle, 1-[(2-furyl) méthoxycarbonyloxy] éthyle, 1-[(2-fluoro éthoxy) carbonyloxy] éthyle, 1-(méthoxycarbonyloxy) propyle, 1-(méthoxycarbonyloxy) 1-méthyl éthyle, (méthoxycarbonyloxy) chlorométhyle, 1-(méthoxycarbonyloxy) 2-chloro éthyle, 1-(méthoxycarbonyloxy) 2-méthoxy éthyle, 1- (méthoxycarbonyloxy) allyle ou 5-méthyl 2-oxo 1,3-dioxol-4-yle.

Le radical diphénylméthyle est plus particulièrement préféré.

Le groupement protecteur du radical amino que peut représenter R₈ peut être par exemple un groupe carbamoyle, méthyl carbamoyle, phénylcarbamoyle, naphtylcarbamoyle, ainsi que les thiocarbamoyles correspondants, un radical alkyle de 1 à 6 atomes de carbone substitué ou non substitué tel que, préférentiellement, trichloroéthyle, tert-butyle ou tert-amyle, un radical aralkyle tel que benzyle, 4-méthoxy benzyle, phénéthyle, trityle, 3,4-diméthoxy benzyle ou benzhydryle, un radical acyle aliphatique, aromatique ou hétérocyclique substitué ou non, tel que par exemple formyle, acétyle, propionyle, butyryle, isobutyryle, valéryle, isovaléryle, chloroacétyle, dichloroacétyle, trichloroacétyle, bromoacétyle, trifluoroacétyle benzoyle, toluolyle, naphtoyle, chlorobenzoyle, para-nitro benzoyle, para-tert-butyl benzoyle, phénoxyacétyle, caprylyle, décanoyle, acryloyle, phtaloyle, mésyle, phénylacétyle, phénylpropionyle, oxalyle, succinyle, pivaloyle, un radical alcoxycarbonyle ou cycloalcoxycarbonyle inférieur tel que par exemple, méthoxycarbonyle, éthoxycarbonyle, propoxycarbonyle, 1-cyclopropyléthoxycarbonyle, isopropoxycarbonyle, butoxycarbonyle, tert-butoxycarbonyle, pentoxycarbonyle, hexyloxycarbonyle, trichloroéthoxy carbonyle, un groupe aralcoxycarbonyle, tel que benzyloxycarbonyle.

On préfère le groupement trityle.

La liste ci-dessus n'est pas limitative, il est évident que d'autres groupements protecteurs des amines, connus de l'homme du métier, groupements connus en particulier dans la chimie des peptides, peuvent également être utilisés.

L'agent réducteur que l'on fait agir sur l'aldéhyde de formule (IIp) est notamment un hydrure tel que le borohydrure de sodium ou l'hydrure d'aluminium-lithium ou encore l'hydrure de diisobutylaluminium.

L'estérification de l'alpha hydroxy acide de formule (III₁) peut être effectuée par les moyens connus de l'homme du métier, notamment par action du diazométhane en solution dans le chlorure de méthylène ou le tétrahydrofuranne.

L'agent activant en présence duquel on fait agir le N-hydroxyphtalimide peut être un agent de transfert de phase. De tels agents sont connus de l'homme du métier.

L'hydrolyse du phtalimide de formule (IV) est effectuée par action de l'hydrazine, de préférence sous forme d'hydrate.

Le dérivé fonctionnel de l'acide de formule (VII) peut être par exemple un halogénure, un anhydride symétrique ou mixte, l'amide, l'azide ou un ester activé.

Comme exemple d'anhydride mixte on peut citer par exemple celui formé avec le chloroformiate d'isobutyle et celui formé avec le chlorure de pivaloyle et les anhydrides mixtes carboxylique-sulfonique formés par exemple avec le chlorure de para-toluène sulfonyle.

Comme exemple d'ester activé, on peut mentionner l'ester formé avec le 2,4-dinitrophénol et celui formé avec l'hydroxybenzothiazole.

Comme exemple d'halogénure, on peut citer le chlorure ou le bromure.

L'anhydride peut être formé in situ par action de carbodiimide N,N'-disubstitué, par exemple le N,N-dicyclohexylcarbodiimide.

La réaction d'acylation est conduite de préférence dans un solvant organique tel que le chlorure de méthylène. On peut cependant utiliser d'autres solvants tels que le tétrahydrofuranne, le chloroforme ou le diméthylformamide.

Lorsqu'on utilise un halogénure d'acide et de manière générale lorsqu'une molécule d'acide est libérée au cours de la réaction, on réalise la réaction de préférence en présence d'une base telle que les carbonates et carbonates acides de sodium ou de potassium, l'acétate de sodium, la triéthylamine, la N,N-diisopropyl éthyl amine, la pyridine, la morpholine ou la N-méthylmorpholine.

La température de réaction est en général inférieure ou égale à la température ambiante.

On peut également faire agir directement un produit de formule (VII) avec un produit de formule (VIII) en présence d'un carbodiimide telle que le diisopropylcarbodiimide ou le 1-(3-diméthylamino propyl) 3-éthyl carbodiimide (EDC). Un exemple d'une telle préparation est donnée plus loin dans la partie expérimentale.

L'action des réactifs capable d'introduire le radical R₆ et conduire au produit de formule (XI) est effectuée dans les conditions suivantes :

Lorsque Hal représente par exemple un atome de chlore, on peut effectuer in situ ou séparément une substitution de l'atome de chlore par un atome d'iode en présence d'iodure de sodium puis ajouter ensuite le réactif désiré, en présence ou non d'un solvant organique tel que le dichlorométhane, l'acétonitrile, le tétrahydrofuranne, l'acétone ou la méthyléthylcétone.

On peut également faire agir directement sur le produit de formule (IX) ou (X), le réactif de formule R₆ approprié, en présence de tétrafluoroborate d'argent.

L'isomérie des produits de formule (XI) peut être différente de celle des produits de formule (IX) ou (X). Dans le cas où l'on isole l'isomère Z, on peut transformer cet isomère en isomère E selon les méthodes usuelles, notamment par action de l'iode.

Selon les valeurs de R₈, R₉, R₁ₚ, R₂ₚ, R₃ₚ, R₄ₚ et R₅ₚ, l'action sur le produit de formule (XI) d'un ou plusieurs agents d'hydrolyse, d'hydrogénolyse ou de la thiourée a pour but d'éliminer le radical R₈ lorsque celui-ci représente un groupement protecteur du radical amino, de transformer les radicaux R₁ₚ, R₂ₚ, R₃ₚ, R₄ₚ et R₅ₚ respectivement en radicaux R₁, R₂, R₃, R₄ et R₅ lorsque ceux-ci portent un groupement protecteur des radicaux hydroxyle et/ou d'éliminer le radical R₉ lorsque celui-ci représente, parmi les groupements esters facilement clivables, l'un de ceux que l'on désire éliminer.

Cependant, il est bien entendu possible d'éliminer R₈ et de transformer les radicaux R₁ₚ, R₂ₚ, R₃ₚ, R₄ₚ et R₅ₚ respectivement en radicaux R₁, R₂, R₃, R₄ et R₅ lorsque ceux-ci portent un groupement protecteur des radicaux hydroxyle sans toucher au substituant R₉ lorsque celui-ci doit être conservé. La nature des réactifs à mettre en jeu dans un tel cas est bien connu de l'homme du métier. On trouvera par exemple une description des différentes méthodes d'élimination des différents groupements protecteurs dans le brevet français B.F. 2.499.995. Des exemples de telles réactions sont donnés plus loin dans la partie expérimentale.

Etant donné la nature des groupements protecteurs préférés que l'on utilise : trityle pour R₈, 2-méthoxy éthoxy méthyle pour protéger les fonctions hydroxy et 4-méthoxy benzyle pour R₉, on utilise de préférence le mélange acide trifluoroacétique/anisole sans solvant ou dans un solvant tel que le chlorure de méthylène. On obtient alors un sel avec l'acide trifluoroacétique. On peut si désiré revenir à la base libre par action d'une base telle qu'un carbonate ou la triéthylamine.

La salification des produits peut être effectuée selon les méthodes usuelles ; elle peut par exemple être obtenue par action sur un produit sous forme acide ou sur un solvat, par exemple le solvat éthanolique, ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phosphate trisodique. On peut également faire appel à des sels d'acides organiques tels que par exemple, les sels de sodium d'acides carboxyliques aliphatiques, linéaires ou ramifiés, saturés ou insaturés de 1 à 18 et de préférence de 2 à 10 atomes de carbone. Les chaînes aliphatiques de ces acides peuvent être interrompues par un ou plusieurs hétéroatomes tels que l'oxygène ou le soufre ou substituées par des radicaux aryle tels que phényle, thiényle ou furyle, par un ou plusieurs radicaux hydroxyles ou par un ou plusieurs atomes d'halogène tels que fluor, chlore ou brome, préférentiellement chlore, par un ou plusieurs radicaux carboxyliques ou alkoxycarbonyles inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques, des acides aromatiques suffisamment solubles comme, par exemple, des acides benzoiques substitués, de préférence par des radicaux alkyles inférieurs.

Comme exemples de tels acides organiques, on peut mentionner les acides formique, acétique, acrylique, butyrique, adipique, isobutyrique, n-caproïque, isocaproïque, chloropropionique, crotonique, phényl acétique, (2-thiényl) acétique, (3-thiényl) acétique, (4-éthyl phényl) acétique, glutarique, l'ester monoéthylique de l'acide adipique, les acides hexanoïque, heptanoique, décanoique, oléïque, stéarique, palmitique, 3-hydroxy propionique, 3-méthoxy propionique, 3-méthyl thiobutyrique, 4-chloro butyrique, 4-phényl butyrique, 3-phénoxy butyrique, 4-éthyl benzoïque, 1-propyl benzoïque.

On utilise cependant de préférence comme sels de sodium, l'acétate de sodium, le 2-éthyl hexanoate de sodium ou le diéthyl acétate de sodium.

La salification peut également être obtenue par action d'une base organique comme la triéthylamine, la diéthylamine, la triméthylamine, la propylamine, la N,N-diméthyl éthanolamine, le tris[(hydroxyméthyl) amino] méthane, la méthylamine, l'éthanolamine, la pyridine, la picoline, la dicyclohexyl amine, la morpholine et la benzylamine.

Elle peut également être obtenue par action de l'arginine, de la lysine, de la procaine, de l'histidine, de la N-méthyl glucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus.

La salification des produits par les acides minéraux ou organiques est effectuée dans les conditions usuelles.

L'estérification éventuelle des produits est effectuée dans les conditions classiques. On opère en général en faisant réagir l'acide de formule (I) ou un dérivé fonctionnel avec un dérivé de formule :

Z-Re

dans laquelle Z représente un radical hydroxyle ou un atome d'halogène tel que le chlore, le brome, l'iode et Re désigne le groupement ester à introduire, groupement dont une liste non exhaustive figure ci-dessus. Dans certains cas, il peut être avantageux d'effectuer une estérification sur un produit dont l'amine et/ou les groupements réactionnels présents sur l'oxyimino sont bloqués avant d'enlever le groupement protecteur de l'amine et du groupement réactionnel présents sur l'oxyimino.

Les produits de formule (I) comportent plusieurs carbones asymétriques. Dans le noyau cephème, qui comporte deux carbones asymétriques, les deux carbones sont dans la configuration R. Par ailleurs, le radical présent sur la fonction oxyimino peut comporter également un carbone asymétrique qui peut être sous forme R ou S ou sous forme d'un mélange R + S. La séparation des deux diastéréoisomères peut être effectuée par les moyens connus de l'homme du métier, par exemple par chromatographie.

Les produits de formule générale (I) possèdent une très bonne activité antibiotique sur les bactéries gram^{⊕} telles que les staphylocoques, les streptocoques et notamment sur les staphylocoques pénicillino-résistants. Leur efficacité sur les entérobactéries productrices de β-lactamases chromosomales ou plasmidiques, est particulièrement remarquable.

Ces propriétés rendent aptes lesdits produits ainsi que leurs sels d'acides pharmaceutiquement acceptables à être utilisés comme médicaments dans le traitement des infections à germes sensibles et notamment dans celui des staphylococcies, telles que septicémies à staphylocoques, staphylococcies malignes de la face ou cutanée, pyodermites, plaies septiques ou suppurantes, anthrax, phlegmons, érésipèles, staphylococcies aiguës primitives ou post grippales, broncho-pneumonies, suppurations pulmonaires, méningites, ainsi que dans le traitement des infections chez les immunodéprimés.

Ces produits peuvent également être utilisés comme médicaments dans le traitement des colibacilloses et infections associées, dans les infections à proteus, à klebsiella et à salmonella et dans d'autres affections provoquées par des bactéries à gram^{⊖}.

La présente invention a donc également pour objet, à titre de médicaments et notamment de médicaments antibiotiques, les produits de formule (I) telle que définie ci-dessus dans laquelle R₁, R₂, R₃, R₄, R₅, R₆ et A sont tels que définis ci-dessus, ainsi que leurs sels d'acides pharmaceutiquement acceptables.

L'invention a particulièrement pour objet à titre de médicaments les produits préférés de formule (I) telle que décrite ci-dessus et notamment dans laquelle R₆ est choisi parmi les radicaux quinoléinium, isoquinoléinium, 4-(méthylthio) pyridinium, thièno[2,3-b]pyridinium, imidazo (1,2-a) pyridinium et 6,7-dihydro 5H-1-pyrindinium.

L'invention a plus particulièrement pour objet, à titre de médicaments et notamment de médicaments antibiotiques, les produits dont les noms suivent :
- le sel interne de (6R-(3-(E) 6alpha, 7béta-(Z))) 1-(3-(7-(((2-amino 4-thiazolyl) (((2,5-difluoro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium,
- le sel interne de (6R-(3-(E) 6alpha, 7béta-(Z))) 1-(3-(7(((2-amino 4-thiazolyl) (((2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium,
- le sel interne de (6R-(3-(E) 6alpha, 7béta-(Z))) 1-(3-(7(((2-amino 4-thiazolyl) (((2,5-difluoro 3,4-dihydroxyphényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium,
- le sel interne de (6R-(3-(E) 6alpha, 7béta-(Z))) 1-(3-(7-(((2-amino 4-thiazolyl) (((2,5-fluoro 3,4-dihydroxyphényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium,
- le sel interne de (6R-(3-(E) 6alpha, 7béta-(Z))) 1-(3-(7-(((2-amino 4-thiazolyl) (((2,5-fluoro 3,4-dihydroxyphényl) méthoxy) imino) acétyl) amino) 2-carboxy 8-oxo 5-thia 1-azabicyclo[4,2,0]oct-2-èn-3-yl) 2-propényl) 6,7-dihydro-5H-1-pyrindinium,
- le sel interne de (6R-(3(E), 6alpha, 7béta(Z))) 1-(3-(7-(((2-amino 4-thiazolyl) (((2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) 6,7-dihydro-5H-1-pyrindinium,
- le sel interne de (6R-(3(E), 6alpha, 7béta(Z))) 1-(3-(7-(((2-amino 4-thiazolyl) ((5-fluoro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium, ainsi que leurs sels pharmaceutiquement acceptables.

L'invention s'étend aux compositions pharmaceutiques renfermant, comme principe actif, au moins un des médicaments définis ci-dessus.

Ces compositions peuvent être administrées par voie buccale, rectale, parentérale, notamment intramusculaire ou par voie locale en application topique sur la peau et les muqueuses.

Les produits de formule (I) et notamment ceux dans laquelle A représente un ester clivable peuvent être administrés par voie orale.

Les compositions selon l'invention peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent notamment se présenter sous forme d'une poudre destinée à être dissoute extemporanément dans un véhicule approprié, par exemple, de l'eau stérile apyrogène.

La dose administrée est variable selon l'affection traitée, le sujet en cause, la voie d'administration et le produit considéré. Elle peut être, par exemple, comprise entre 0,250 g et 4 g par jour, par voie orale chez l'homme, avec le produit décrit à l'exemple 1 ou encore comprise entre 0,500 g et 1 g trois fois par jour par voie intramusculaire.

Les produits de formule (I) peuvent également être utilisés comme désinfectants des instruments chirurgicaux.

L'invention a enfin pour objet, à titre de produits industriels nouveaux et notamment à titre de produits intermédiaires nécessaires à la préparation des produits de formule (I), les produits de formules (IV), (V), (VII), (IX), (X) et (XI) telles que définies précédemment.

Les produits de formule (II) sont connus de manière générale et pour la plupart commercialisés ; d'autres peuvent être préparés à partir de produits commercialisés, selon les méthodes décrites dans la demande européenne 0551034. Pour la préparation des produits de formule (II), on peut aussi utiliser les méthodes décrites dans la littérature, notamment la réduction dite de Rosemund, la réduction des acides benzoïques, ou la formylation des cycles aromatiques comme par exemple la réaction de Vilsmeier-Haack, la réaction de Gatterman-Koch, la réaction de Reimer-Tiemann ou la réaction avec le fluorure de formyle (J. Am. Chem. Soc. 82, 2380 (1960)).

Les produits de formules (VI) et (VIII) sont également connus dans la littérature, notamment dans les demandes de brevets belge BE864828 et européenne EP0333154.

La préparation des produits de formule (XII) est décrite dans la demande européenne 0551034.

Les exemples suivants illustrent l'invention sans toutefois la limiter.

### EXEMPLE 1 : Sel interne de (6R(6R(3(E),6α,7β(Z))) 1-3-(7-(((2-amino-4-thiazolyl)(((2,5-dichloro-3,4-dihydroxyphényl)-méthoxy)imino)acétyl)amino)-2-carboxy-8-oxo-5-thia-1-azabicyclo(4.2.0)oct-2-en-3-yl)-2-propényl)-quinoléinium.

### Stade A : Alcool 2,5-dichloro 3,4-bis(2-méthoxyéthoxy)méthoxy benzylique.

A une solution de 16,1 g de 2,5-dichloro-3,4-bis- (2-méthoxyéthoxy)méthoxy benzaldéhyde dans 200 ml de méthanol, on ajoute, par portions à 0°C : 1,59 g de borohydrure de sodium. On agite 30 minutes à 0°C puis ajoute 2,4 ml d'acide acétique en laissant la température revenir à l'ambiance. On évapore à sec sous pression réduite et reprend le résidu avec 80 ml d'acétate d'éthyle, lave avec une solution d'eau salée, sèche, filtre et évapore à sec sous pression réduite on recueille ainsi 13,6 g de produit recherché utilisé tel quel pour le stade suivant.
Spectre I.R. CHCl₃
OH : 3610 cm⁻¹
Aromatique : 1555 cm⁻¹
Spectre R.M.N. CDCl₃
3,38 (m) : CH₃ de OMEM ; 3,58 (m) 4,00 (m) 5,24 (s) 5,25 (s) : les CH₂ de OMEM ; 4,78 (s) : : CH₂ de l'alcool ; 7,35 (s) : H aromatique.

### Stade B : (2,5 dichloro-3,4-bis(2-méthoxyéthoxy)méthoxy)-phényl)((1,3-dihydro 1,3 dioxo-2H-isoindol-2yl)oxy)méthyle.

A une solution, refroidie, à 0°C de 18,39 g de triphényl phosphine dans 150 ml de tétrahydrofuranne on ajoute goutte à goutte, sans dépasser + 5°C, 11,09 ml de azodicarboxylate de diéthyle, on agite 15 minutes et ajoute par fractions 11,43 g de N-hydroxyphtalimide. On refroidit à 0°C et ajoute goutte à goutte une solution de 13,6 g du produit obtenu au stade A ci-dessus dans 40 ml de tétrahydrofuranne, après 1 heure d'agitation à 0°C on amène à sec sous pression réduite. On reprend le résidu par 100 ml d'acétate d'éthyle, lave à l'eau, sèche, filtre et évapore à sec sous pression réduite. On obtient 50 g de produit que l'on chromatographie sur silice en éluant avec un mélange chlorure de méthylène-acétate d'éthyle 80-20. On recueille 17,9 g de produit recherché.
Spectre I.R. :
1790 cm⁻¹
1750 cm⁻¹ C=O
1736 cm⁻¹
1610 cm⁻¹ aromatique
1560 cm⁻¹
Spectre R.M.N. CDCl₃
3,38 (s) : CH₃ de OMEM ; 3,57-3,98-5,25-5,27 : les CH₂ de OMEM : 7,52 (s)-7,75 à 7,84 (m) : les aromatiques.

### Stade C : 2,5-dichloro-3,4-bis(2-méthoxyéthoxy)méthoxy oxyaminobenzyle.

A une solution, refroidie à 0°C, de 17,7 g de produit obtenu au stade B ci-dessus, dans 120 ml de dichlorométhane, on ajoute 3,24 ml d'hydrate d'hydrazine puis on agite pendant une heure à 0°C. On filtre l'insoluble et concentre à sec sous pression réduite, le résidu est repris par 50 ml d'éther éthylique, lave à l'eau, sèche et évapore à sec sous pression réduite. Le produit obtenu est chromatographié sur silice en éluant avec un mélange chlorure de méthylène-acétate d'éthyle 80-20. On recueille 9,6 g de produit que l'on reprend par 80 ml d'éther éthylique, lave à l'eau, sèche et évapore à sec sous pression réduite. On obtient 9,15 g de produit attendu. Spectre I.R. CHCl₃ :
3324 cm⁻¹ : CH₂-ONH₂
1583 cm⁻¹ : aromatique
1558 cm⁻¹ : NH₂
Spectre R.M.N. CDCl₃
3,38 (s) : CH₃ ; 3,58 (m)-4,00 (m)-5,25 (s) : les CH₂ de OMEM ; 4,74 (s) : CH₂-ONH₂ ; 5,55 (s,l) : NH₂ ; 7,28 : aromatique.

### Stade D : (Z) acide α (2,5-dichloro-3,4 bis((2-méthoxyéthoxy)méthoxy) phényl méthoxy imino)(2-((triphénylméthyl) amino)4-thiazoleacétique.

A une suspension de 8,34 g du produit obtenu au stade C avec 80 ml de méthanol, on ajoute, par portions à température ambiante, 9,5 g d'acide oxo-[2-[(triphénylméthyl) amino]-thiazol-4-yl]acétique (décrit dans la demande de brevet belge n° 864828). On agite une heure puis on évapore à sec. Le résidu est repris par du chlorure de méthylène, lavé avec de l'acide chlorhydrique 0,1 N, séché, filtré puis concentré à sec. On recueille 20 g de produit que l'on chromatographie sur silice en éluant avec un mélange chlorure de méthylène-méthanol. On chromatographie une seconde fois dans les mêmes conditions pour obtenir 13,51 g de produit recherché.
Spectre I.R. CHCl₃
3404 cm⁻¹ : =C-NH
1620 cm⁻¹ : COO⁻ / C=N
1592 cm⁻¹
1577 cm⁻¹ : hétérocycle
1529 cm⁻¹
1493 cm⁻¹ : aromatique
Spectre R.M.N. CDCl₃
3,35 (s) : CH₃ de OMEM ; 3,55 (m)-3,95 (m)-5,17 (s)-5,20 (s) : : les CH₂ de OMEM ; 5,13 (s,l) : CH₂-O-N ; 6,54 (s,l) : H thiazole ; 7,20 à 7,28 : aromatique.

### Stade E : [6R(3(E), 6α, 6β (Z))] 3-(3-chloro 1-propényl) 7-(((2,5-dichloro-3,4 bis((2-méthoxyéthoxy)méthoxy) phényl méthoxy) imino)(2-((triphénylméthyl)amino) 4-thiazolyl) acétyl) amino) 8-oxo 5-thia 1-azabicyclo(4.2.0)oct-2-ène-2-carboxylate de (4-méthoxyphényl)méthyle.

A une solution de 8,1 g du produit obtenu au stade D dans 40 ml de chlorure de méthylène, on ajoute 4,37 g de chlorhydrate de 7β-amino 3-[3-chloro 1-propényl) 8-oxo 5-thia 1-azabicyclo(4.2.0)oct-2-ène-2-carboxylate de (4-méthoxyphényl)méthyle (décrit dans la demande de brevet européen n° 0333154), on refroidit à -5°C et introduit 2,33 g de diméthylamino propyl-éthyl-carbodiimide, on agite 2 heures 30 à 0°C. On lave alors la solution avec 2 fois 10 ml de solution tampon phosphate à pH 7, puis à l'eau salée, sèche, filtre et évapore à sec sous pression réduite pour obtenir 10,54 g de produit que l'on chromatographie sur silice en éluant avec un mélange chlorure de méthylène-éther éthylique 90-10. On recueille 6,52 g de produit recherché.
Spectre R.M.N. CDCl₃
3,27 (d,j=18)-3,45 (d,j=18) : CH₂S ; 3,36-3,37 (s) : CH₃ de OMEM ; 3,57-3,98 (m) et 5,15 à 5,35 : les CH₂ de OMEM et CH₂-O-N ; 3,81 : méthoxy benzyle ; 5,01-5,05(d) : N-CH-CH-S ; 5,91-5,93 (dd) : N-CH-CH-S ; 3,72 et 3,92 (dd,j=12 & 8) : CH₂Cl ; 5,73 (dt,j = 11 & 8) et 6,24 (d,j = 11) : -CH=CH-CH₂Cl (delta Z) ; 6,02 (dt) et 7,04 (d,j = 15) : -CH=CH-CH₂Cl (delta E) ; 7,30 (m) : trityl ; 6,88 à 7,30 : les aromatiques ; 5,15 à 5,37 : O-CH₂-Φ.

### Stade F : [6R(3(E), 6α, 7β (Z))] 3-(3-iodo 1-propényl) 7-(((2,5 dichloro-3,4 bis ((2-méthoxyéthoxy)méthoxy) phényl méthoxy) imino) (2-((triphénylméthyl)amino) 4-thiazolyl) acétyl) amino) 8-oxo 5-thia 1-azabicyclo(4.2.0)oct-2-ène-2-carboxylate de (4-méthoxyphényl)méthyle.

A une solution de 3,1 g du produit obtenu au stade E, ci-dessus, dans 12,5 ml d'acétone, on ajoute 1,59 g d'iodure de sodium et un cristal d'iode. On agite 40 minutes à température ambiante, évapore à sec sous pression réduite et reprend avec 10 ml de chlorure de méthylène, lave avec une solution aqueuse de thiosulfate de sodium à 10% puis à l'eau salée, sèche, filtre et concentre à sec sous pression réduite. On obtient 3,79 g de produit recherché que l'on utilise tel quel pour le stade suivant.

### Stade G : (6R(3(E),6α,7β (Z))) iodure de 1-(3-(7-(((((2,5-dichloro-3,4-bis-((2-méthoxyéthoxy)méthoxy) phényl) méthoxy) imino)(2-((triphénylméthyl)amino) 4-thiazolyl) acétyl) amino) 2-(((4-méthoxyphényl) méthoxy) carbonyl) 8-oxo-5-thia-1-azabicyclo(4.2.0)oct-2-en-3-yl)-2-propényl)-quinoléinium.

A une solution du composé, obtenu au stade F, dans 3 à 4 ml de chlorure de méthylène, on ajoute 1,57 ml de quinoléine redistillée. On évapore le solvant sous pression réduite puis agite 1 heure à pression ordinaire. On ajoute de l'éther éthylique, agite 30 minutes, essore, lave à l'éther et sèche sous pression réduite. On recueille 4 g de produit que l'on chromatographie sur silice en éluant avec un mélange chlorure de méthylène-méthanol 95-05. On recueille 2,18 g de produit recherché.
Spectre I.R.
3404 cm-¹ : =C-NH
1788 cm-¹ : C=O
1721 cm-¹
1689 cm-¹
1628 cm-¹ : C=C
1613 cm-¹ : C=N
1595 cm-¹
1529 cm-¹ : aromatique
1517 cm-¹
1493 cm-¹ : amide II
Spectre R.M.N. CDCl₃
3,34 (s) : CH₃ de OMEM ; 3,55 (m)-3,96 (m) et 5,13 à 5,31 : les CH₂ de OMEM et CH₂-O-N= ; 6,86 (d, mobile) : CO-NH ; 6,70 (s) : H thiazole ; 7,27 (m) : trityl ; 4,97 (d,j=5) : N-CH-CH-S ; 5,88 (dd,d ap. éch.) : N-CH-CH-S ; 6,90 à 7,27 : 4 H aromatiques ; 3,78 (s) : OCH₃ du benzyle ; 5,13 à 5,31 : CH₂-Φ ; 3,47 (d)-3,64 (d) : S-CH₂- ; 6,51 (dt,j=15,5 & 6) : CH propényl ; 6,06 et 6,21 (ddl,j=15 & 6) : CH₂ propényl ; 7,98 à 10,45 : 7H quinoléine.

### Stade H : Sel interne de (6R(3(E),6α,7β(Z))) 1-3-(7-(((2-amino-4-thiazolyl)(((2,5-dichloro-3,4-dihydroxyphényl)-méthoxy)imino)acetyl)amino)-2-carboxy-8-oxo-5-thia-1-azabicyclo(4.2.0)oct-2-en-3-yl)-2-propényl)-quinoléinium.

On agite 10 minutes à 0°C une solution de 2,1 g du produit obtenu au stade G dans 20 ml d'une solution refroidie à 0°C d'acide trifluoroacétique à 10 % d'anisole, puis on laisse la température remonter et agite pendant 2 heures 30 à température ambiante. On filtre, lave avec quelques millilitres d'acide trifluoroacétique, refroidit à 0°C et ajoute 70 ml d'éther éthylique. On agite 10 minutes à 0°C puis 90 minutes à température ambiante. On filtre, lave par trois fois 10 ml d'éther éthylique et agite à nouveau le produit obtenu pendant une heure avec 30 ml d'éther éthylique. On filtre comme précédemment et sèche sous pression réduite, on recueille 1,076 g de produit recherché.
Spectre I.R. Nujol
Absorption OH/NH
1778 cm-¹ : C=O (béta lactame)
1666 cm-¹
1594 cm-¹ : hétérocycle
1544 cm-¹ : aromatique
1532 cm-¹ : amide II / COO⁻
Spectre R.M.N. Diméthylsulfoxyde
6,96 (s) : : 1H aromatique, 5,08 (s) : -CH₂-O-N= ; 9,74 (d,mob.) : NH ; 6,76 (s) : H thiazole ; 5,17 (d,j=5) : N-CH-CH-S ; 5,80 (dd,d ap. éch.) : N-CH-CH-S ; 3,51-3,72 (d,j=17,5)) : S-CH₂- ; 6,38 (dt,j=15,5 & 6) : CH propényl ; 7,00 : l'autre CH propényl ; 5,89 (m) : CH₂ propényl ; 8,25 à 9,59 : 7H quinoléine.

### EXEMPLE 2 : Sel interne de (6R(3(E),6α,7β(Z))) 1-3-(7-(((2-amino-4-thiazolyl) (((2,5-dichloro-3,4-dihydroxyphényl)-méthoxy)imino)acétyl)amino) -2-carboxy-8-oxo-5-thia-1-azabicyclo(4.2.0)oct-2-en-3-yl)-2-propényl)-imidazo(1,2-a)pyridinium.

### Stade A : [6R(3(E), 6α,7β (Z))] 3-(3-chloro 1-propényl) 7-(((2,5 dichloro-3,4-bis((2-méthoxyéthoxy)méthoxy) phényl méthoxy)imino)(2-((triphénylméthyl)amino) 4-thiazolyl) acétyl) amino) 8-oxo 5-thia 1-azabicyclo(4.2.0)oct-2-ène-2-carboxylate de (4-méthoxyphényl)méthyle.

En opérant comme au stade F de l'exemple 1 à partir de 3,2 g du produit obtenu au stade E de l'exemple 1, on obtient 3,55 g de produit recherché utilisé tel quel pour le stade suivant.

### Stade B : (6R(3(E),6α,7β (Z))) iodure de 1-(3-(7-(((((2,5-dichloro-3,4-bis((2-méthoxyéthoxy)méthoxy) phényl) méthoxy) imino)(2-((triphénylméthyl)amino) 4-thiazolyl) acétyl) amino) 2-(((4-méthoxyphényl) méthoxy) carbonyl) 8-oxo-5-thia-1-azabicyclo(4.2.0)oct-2-en-3-yl)-2-propényl)-imidazo (1,2 a) pyridinium

En opérant comme au stade G de l'exemple 1 à partir de 3,55 g du produit obtenu au stade A ci-dessus et en utilisant 0,69 ml d'imidazo (1,2 a) pyridine, on a obtenu 4,05 g du produit brut et après chromatographie sur silice (éluant chlorure de méthylène-méthanol 96-4) 2,05 g de produit recherché.
Spectre I.R.
3404 cm-¹ : =C-NH
1787 cm-¹ : C=O
1720 cm-¹
1689 cm-¹
1648 cm-¹ : C=C
1613 cm-¹ : C=N
1600 cm-¹
1587 cm-¹
1528 cm-¹ : aromatique
1517 cm-¹
1493 cm-¹ : amide II
Spectre R.M.N. CDCl₃
3,33-3,34 (s) : CH₃ de OMEM ; 3,56 (m)-3,95 (m) et 5,10 à 5,50 : les CH₂ de OMEM et CH₂-O-N= ; 6,86 (d,mobile) : CO-NH ; 6,70 (s) : H thiazole ; 7,25 à 7,33(m) : trityl ; 4,99 (d,j=5) N-CH-CH-S ; 5,88 (dd,d ap. éch.) : N-CH-CH-S ; 6,89 à 7,33 : 5 H aromatiques ; 3,78 (s) : OCH₃ du benzyle ; 5,10 à 5,50 : CH₂-Φ ; 3,47 (d)-3,60 (d,j=18) : S-CH₂- ; 6,27 (d,j=16 & 6,3) et 7,15 (d,j=16) : les CH propényl ; 5,10 à 5,50 : CH₂ propényl ; 7,44 à 9,21 : 7H imidazo pyridine.

### Stade C : Sel interne de (6R(3(E),6α,7β(Z))) 1-3-(7-(((2-amino-4-thiazolyl)(((2,5-dichloro-3,4-dihydroxyphényl)-méthoxy)imino)acétyl)amino)-2-carboxy-8-oxo-5-thia-1-azabicyclo(4.2.0)oct-2-en-3-yl)-2-propényl)-imidazo(1,2-a)pyridinium.

En opérant comme au stade H de l'exemple 1 à partir de 1,45 g du produit obtenu au stade B ci-dessus, et en utilisant 14 ml d'une solution d'acide trifluoroacétique à 10 % d'anisole, on obtient 0,678 g de produit recherché.
Spectre I.R. Nujol
Absorption OH/NH
1774 cm-¹ : C=O (béta lactame)
1674 cm-¹
1620 cm-¹ : aromatique
1528 cm-¹ : amide II / COO⁻
Spectre R.M.N. Diméthylsulfoxyde
6,96 (s) : 1H aromatique, 5,08 (s) : -CH₂-O-N= ; 9,72 (d,mob.) : NH ; 6,77 (s) : H thiazole ; 5,17 (d,j=5) : N-CH-CH-S ; 5,79 (dd,d ap. éch.) : N-CH-CH-S ; 3,53-3,72 (d,j=17,5)) : S-CH₂- ; 6,25 (dt,j=15,5 & 6)-6,88 (d,j=16) : les CH propényl ; 5,89 (m) : CH₂ propényl ; 7,57 à 8,96 : 6H imidazo pyridine.

### EXEMPLE 3 : Sel interne de (6R (3(E),6α,7β(Z))) 1-3-(7-(((2-amino-4-thiazolyl) (((2,5-dichloro-3,4-dihydroxyphényl)-méthoxy)imino)acétyl)amine)-2-carboxy-8-oxo-5-thia-1-azabicyclo(4.2.0)oct-2-en-3-yl)-2-propényl)-6,7 dihydro 5H-1-pyrindinium.

### Stade A : [6R(3(E), 6α, 7β (Z))] 3-(3-chloro 1-propényl) 7-(((2,5 dichloro-3,4-bis-((2-méthoxyéthoxy)méthoxy) phényl méthoxy) imino)(2-((triphénylméthyl)amino) 4-thiazolyl) acétyl) amino) 8-oxo 5-thia 1-azabicyclo(4.2.0)oct-2-ène-2-carboxylate de (4-méthoxyphényl)méthyle.

En opérant comme au stade F de l'exemple 1 à partir de 3,2 g du produit obtenu au stade E de l'exemple 1, on obtient 3,33 g de produit recherché utilisé tel quel pour le stade suivant.

### Stade B : (6R(3(E),6α,7β (Z))) iodure de 1-(3-(7-(((((2,5-dichloro-3,4-bis-((2-méthoxyéthoxy)méthoxy) phényl) méthoxy) imino)(2-((triphénylméthyl)amino) 4-thiazolyl) acétyl) amino) 2-(((4-méthoxyphényl) méthoxy) carbonyl) 8-oxo-5-thia-1-azabicyclo(4.2.0)oct-2-en-3-yl)-2-propényl)-6,7 dihydro 5H-1-pyrindinium.

En opérant comme au stade G de l'exemple 1 à partir de 3,33 g du produit obtenu au stade A ci-dessus et en utilisant 1,60 ml de (2,3)cyclo pentèno pyridine, on a obtenu 3,55 g du produit brut et après chromatographie sur silice (éluant chlorure de méthylène-méthanol (96-4)) 2,20 g de produit recherché.
Spectre I.R.
3404 cm-¹ : =C-NH
1788 cm-¹ : C=O
1720 cm-¹
1689 cm-¹
1614 cm-¹ : C=N/C=C
1600 cm-¹
1587 cm-¹
1527 cm-¹ : aromatique
1517 cm-¹
1492 cm-¹ : amide II
Spectre R.M.N. CDCl₃
3,34-3,35 (s) : CH₃ de OMEM ; 3,56 (m)-3,96 (m) et 5,13 (d)-5,33 (s,1) : les CH₂ de OMEM et CH₂-O-N= ; 6,89 : CO-NH ; 6,70 (s) : H thiazole ; 7,28 (m) : trityl ; 4,99 : N-CH-CH-S ; 5,89 (dd,d ap. éch.) : N-CH-CH-S ; 6,89 à 7,28 : 5 H aromatiques ; 3,78 (s) : OCH₃ du benzyle ; 5,13 à 5,33 : CH₂-Φ ; 3,55-3,68 (d,j=18) : S-CH₂- ; 6,37 (d,j=16 & 6,5) et 7,02 (d,j=16) : les CH propényl ; 5,49 à 5,60 : CH₂ propényl ; 2,41 à 9,14 : 9H pyrindinium.

### Stade C : Sel interne de (6R(3(E),6α,7β(Z))) 1-3-(7-(((2-amino-4-thiazolyl)(((2,5-dichloro-3,4-dihydroxyphényl)-méthoxy)imino)acétyl)amino)-2-carboxy-8-oxo-5-thia-1-azabicyclo(4.2.0)oct-2-en-3-yl)-2-propényl)-6,7 dihydro 5H-1-pyrindinium.

En opérant comme au stade H de l'exemple 1 à partir de 2,18 g du produit obtenu au stade B ci-dessus, et en utilisant 20 ml d'une solution d'acide trifluoroacétique à 10 % d'anisole, on obtient 1,094 g de produit recherché.
Spectre I.R. Nujol
Absorption OH/NH
1780 cm-¹ : C=O (béta lactame)
1674 cm-¹
Spectre R.M.N. Diméthylsulfoxyde
6,78 (s)-6,97 (s) : 1H aromatique , 5,08 (s) : -CH₂-O-N= ; 9,75 (d,mob.) : NH ; 6,78 (s)-6,97 (s) : H thiazole ; 5,19 (d,j=5) : N-CH-CH-S ; 5,82 (dd,d ap. éch.) : N-CH-CH-S ; 3,54-3,77 (d,j 18) : S-CH₂- ; 6,25 (dt)-6,88 (d,j=16) : les CH propényl ; 5,33 (d,l) : CH₂ propényl ; 2,23 à 8,76 : 9H pyrindinium.

### EXEMPLE 4 : Sel interne de (6R(3(E),6α,7β(Z))) 1-3-(7-(((2-amino-4-thiazolyl)(((2,5-difluoro-3,4-dihydroxyphényl)-méthoxy)imino)acétyl)amino)-2-carboxy-8-oxo-5-thia-1-azabicyclo(4.2.0)oct-2-en-3-yl)-2-propényl)-quinoléinium.

### Stade A : Alcool 2,5 difluoro 3,4-bis(2-méthoxyéthoxy)méthoxy benzylique.

En opérant comme au stade A de l'exemple 1, à partir de 21 g de 2,5 difluoro 3,4-bis(2-méthoxyethoxy)méthoxy benzaldéhyde et en utilisant 2,26 g de borohydrure de sodium, on a obtenu 20,53 g de produit recherché.
Spectre R.M.N. CDCl₃
3,36 (s) : CH₃ de OMEM ; 3,56 (m) 3,94 (m) 5,22(s) 5,24 (s) : les CH₂ de OMEM ; 4,67 (s) : CH₂ de l'alcool ; 5,96 (s) : H6 aromatique.

### Stade B : (2,5 difluoro-3,4 bis(2-méthoxyéthoxy)méthoxy)-phényl) ((1,3-dihydro 1,3-dioxo-2H-isoindol-2yl)oxy)méthyle.

En opérant comme au stade B de l'exemple 1, à partir de 2 g de l'alcool obtenu au stade A ci-dessus et en utilisant 2,98 g de triphényle phosphine, 1,79 ml de azodicarboxylate de diéthyle et 1,38 g de N-hydroxyphtalimide. On a obtenu 890 mg de produit recherché.
Spectre R.M.N. CDCl₃
3,30 (s) : CH₃ de OMEM ; 3,35-3,91-5,22-5,25 : les CH₂ de OMEM ; 5,20 (AB) : N-O-CH₂-Φ ; 7,14 (dd) : H6 ; 7,78 : les aromatiques.

### Stade C : 2,5 difluoro-3,4 bis(2-méthoxyéthoxy)méthoxy oxyaminobenzyle.

En opérant comme au stade C de l'exemple 1, à partir de 14,8 g de produit obtenu au stade B ci-dessus et 2,12 ml d'hydrate d'hydrazine on obtient, après chromatographie sur silice (éluant : mélange chlorure de méthylène-acétate d'éthyle 60-40) 7,2 g de produit attendu.
Spectre I.R. CHCl₃ :
3335 et 1585 cm⁻¹ : CH₂-O-NH₂
1630 et 1495 cm⁻¹ : aromatique
Spectre R.M.N. CDCl₃
3,37 O-CH₃ ; 3,57 (m)-3,95 (m)-5,24 (s) : les CH₂ de OMEM ; 4,68 (m) : CH₂-O-NH₂ ; 5,49 (s,l) : NH₂ ; 7,28 : H6 aromatique.

### Stade D : (Z) acide α (2,5-difluoro-3,4-bis((2-méthoxyéthoxy)méthoxy) phényl méthoxy imino)(2-((triphénylmethyl)amino)4-thiazoleacétique.

En opérant comme au stade D de l'exemple 1, à partir de 4 g du produit obtenu au stade C ci-dessus et en utilisant 4,92 g d'acide oxo-[2-[(triphénylméthyl)amino]thiazol-4-yl]acétique (décrit dans la demande de brevet belge n° 864828). On obtient, après chromatographie sur silice (éluant : mélange chlorure de méthylène-méthanol 90-10), 7,53 g de produit recherché.
Spectre I.R. CHCl₃
3404 cm⁻¹ : =C-NH
1607 cm⁻¹ : COO⁻ / hétérocycle / aromatique :
1597 cm⁻¹
1529 cm⁻¹
1495 cm⁻¹
Spectre R.M.N. CDCl₃
3,28 (s) : O-CH₃ de OMEM ; 3,50 (m)-3,87 (m)-5,15 (s) : les CH₂ de OMEM ; 4,93 (s,l) : CH₂-O-N ; 6,45 (s,l) : H thiazole ; 7,10 à 7,25 : tityle ; 6,88 (dd) : H6 aromatique.

### Stade E : [6R(3(E), 6α, 7β (Z))] 3-(3-chloro 1-propényl) 7-(((2,5 difluoro-3,4-bis((2-méthoxyéthoxy)méthoxy) phényl méthoxy) imino)(2-((triphénylméthyl)amino) 4-thiazolyl) acétyl) amino) 8-oxo 5-thia 1-azabicyclo(4.2.0)oct-2-ène-2-carboxylate de (4-méthoxyphényl)méthyle.

En opérant comme au stade E de l'exemple 1, à partir de 1,78 g du produit obtenu au stade D, et 780 mg de chlorhydrate de 7β-amino 3-[3-chloro 1-propényl) 8-oxo 5-thia 1-azabicyclo(4.2.0)oct-2-ène-2-carboxylate de (4-méthoxyphényl)méthyle (décrit dans la demande de brevet européen n° 0333154), on obtient après chromatographie sur silice (éluant : mélange chlorure de méthylène-acétate d'éthyle 80-20) 1 g de produit recherché.
Spectre R.M.N. CDCl₃
3,30-3,44 (s) : CH₂S ; 3,33-3,35 (s) : CH₃ de OMEM ; 3,55-3,92 (m) et 3,74 (dd) : les CH₂ de OMEM, CH=CH-CH₂-X ; 5,15-5,21 (AB)-5,31 (AB) : O-CH₂-O de OMEM, CH₂-O-N, O-CH₂-Φ. ; 3,80 : méthoxy benzyle ; 7,30 (m) : trityl ; 6,88 : les aromatiques ; 5,91 (dd) : H7 ; 6,75 : H5 thizole ; 6,71 (d) : CO-NH-CH ; 6,98 (s) : NH ;
pour ΔZ : 5,09 (d) : H6 ; 5,72 (dt) : -CH=CH-CH₂ ; 6,74 (d,j=8,5) : -CH=CH-CH₂
pour ΔE : 4,99 : H6 ; 6,85 : -CH=CH-CH₂

### Stade F : [6R(3(E), 6α, 7β (Z))] 3-(3-iodo 1-propényl) 7-(((2,5 difluoro-3,4-bis((2-méthoxyéthoxy)méthoxy)phényl méthoxy) imino)(2-((triphénylméthyl)amino) 4-thiazolyl) acétyl) amino) 8-oxo 5-thia 1-azabicyclo(4.2.0)oct-2-ène-2-carboxylate de (4-méthoxyphényl)méthyle.

En opérant comme au stade F de l'exemple 1 à partir de 6 g du produit obtenu comme au stade E ci-dessus, et en utilisant 3,15 g d'iodure de sodium, on a obtenu 6,45 g de produit recherché que l'on utilise tel quel pour le stade suivant.

### Stade G : (6R(3(E),6α,7β (Z))) iodure de 1-(3-(7-(((((2,5-difluoro-3,4-bis((2-méthoxyéthoxy)méthoxy) phényl) méthoxy) imino)(2-((triphénylméthyl)amino) 4-thiazolyl) acétyl) amino) 2- (((4-méthoxyphényl) méthoxy) carbonyl) 8-oxo-5-thia-1-azabicyclo(4.2.0)oct-2-en-3-yl)-2-propényl)-quinoléinium.

En opérant comme au stade G de l'exemple 1 à partir de 2 g du composé obtenu au stade F, en utilisant 0,954 ml de quinoléine, on obtient après chromatographie sur silice (éluant chlorure de méthylène-méthanol 92-08) 684 mg de produit recherché.

### Stade H : Sel interne de (6R(3 (E),6α,7β(Z))) 1-3-(7-(((2-amino-4-thiazolyl)(((2,5-difluoro-3,4-dihydroxyphényl)-méthoxy)imino)acetyl)amino)-2-carboxy-8-oxo-5-thia-1-azabicyclo(4.2.0)oct-2-en-3-yl)-2-propényl)-quinoléinium.

En opérant comme au stade H de l'exemple 1 à partir de 663 mg du produit obtenu au stade G, en utilisant 6 ml d'acide trifluoroacétique à 10 % d'anisole, on recueille 321 mg de produit recherché.
Spectre R.M.N. Diméthylsulfoxyde
3,53-3,72 (d,j=17,5) : CH₂S ; 5,04 (s) : Φ-CH₂-O ; 5,16 (d,j=5) : H6 ; 5,78 (dd,j=5 & 8) : H7 ; 5,88 (AB) : =CH-CH2-N⁺ ; 6,38 (dt,j=16 & 6) : =CH-CH2-N⁺ ; 6,69 (dd,j=6 & 11) : H6'' (cycle difluoro) ; 6,75(s) : H thiazole 6,98 (d,j=16) : =C-CH=CH- ; 8,06 à 9,58 : 7H' quinoléine ; 9,69 (dj=8, mobile) : CO-NH-CH ; 7,30-9,60 : H mobiles.

### EXEMPLE 5 : Sel interne de (6R(3(E), 6α,7β (Z))) 1-3-(7-(((2-amino-4-thiazolyl) (((2,5-difluoro-3,4-dihydroxyphényl) méthoxy)imino)acétyl)amino)-2-carboxy-8-oxo-5-thia-1-azabicyclo(4.2.0)oct-2-en-3-yl)-2-propényl)-imidazo(1,2-a)pyridinium.

### Stade A : (6R(3(E),6α,7β(Z))) iodure de 1-(3-(7-(((((2,5-difluoro-3,4-bis((2-méthoxyéthoxy)méthoxy) phényl) méthoxy) imino)(2-((triphénylméthyl)amino) 4-thiazolyl) acétyl) amino) 2-(((4-méthoxyphényl) méthoxy) carbonyl) 8-oxo-5-thia-1-azabicyclo(4.2.0)oct-2-en-3-yl)-2-propényl)-imidazo (1,2 a) pyridinium

En opérant comme au stade G de l'exemple 1 à partir de 2 g du produit obtenu au stade F de l'exemple 4 et en utilisant 0,492 ml d'imidazo (1,2 a) pyridine, on a obtenu après chromatographie sur silice (éluant chlorure de méthylène-méthanol (96-4)) 950 mg du produit recherché.

### Stade B : Sel interne de (6R(3(E),6α,7β(Z))) 1-3-(7-(((2-amino-4-thiazolyl)(((2,5-difluoro-3,4-dihydroxyphényl)-méthoxy)imino)acétyl)amino)-2-carboxy-8-oxo-5-thia-1-azabicyclo(4.2.0)oct-2-en-3-yl)-2-propényl)-imidazo(1,2-a)pyridinium.

En opérant comme au stade H de l'exemple 1 à partir de 940 mg du produit obtenu au stade A, ci-dessus, et en utilisant 9 ml d'une solution d'acide trifluoroacétique à 10 % d'anisole, on obtient 0,351 g de produit recherché.
Spectre R.M.N. Diméthylsulfoxyde
3,53-3,72 (d) : CH₂S ; 5,05 (s) : Φ -CH₂-O ; 5,17 (d) : H6 ; 5,78 (dd) : H7 ; 5,28 (m) : =CH-CH2-N⁺ ; 6,25 (dt,j=16 & 6) : =CH-CH2-N⁺ ; 6,70 (dd,j=6 & 11) : H6" (cycle difluoro) ; 6,76 (s) : H thiazole ; 6,98 (d,j=16) : =C-CH=CH- ; 7,57 à 8,44 : 6H' quinoléine ; 9,48 et 9,70 (d) : CO-NH-CH ; 7,30-9,65 : H mobiles.

### EXEMPLE 6 : Sel interne de (6R(3(E),6α,7β(Z))) 1-3-(7-(((2-amino-4-thiazolyl) (((2,5-difluoro-3,4-dihydroxyphényl)-méthoxy)imino)acétyl)amino)-2-carboxy-8-oxo-5-thia-1-azabicyclo(4.2.0)oct-2-en-3-yl)-2-propényl)-6,7-dihydro 5H-1-pyrindinium.

### Stade A : (6R(3(E),6α,7β (Z))) iodure de 1-(3-(7-(((((2,5-difluoro-3,4-bis((2-méthoxyéthoxy)méthoxy) phényl) méthoxy) imino)(2-((triphénylméthyl)amino) 4-thiazolyl) acétyl) amino) 2-(((4-méthoxyphényl) méthoxy) carbonyl) 8-oxo-5-thia-1-azabicyclo(4.2.0)oct-2-en-3-yl)-2-propényl)-6,7-dihydro 5H-1-pyrindinium.

En opérant comme au stade G de l'exemple 1 à partir de 2 g du produit obtenu au stade F de l'exemple 4 et en utilisant 0,595 ml de (2,3)cyclo pentèno pyridine, on a obtenu après chromatographie sur silice (éluant chlorure de méthylène-méthanol (96-4)), 899 mg de produit recherché.

### Stade B : Sel interne de (6R(3(E),6α,7β(Z))) 1-3-(7-(((2-amino-4-thiazolyl) (((2,5-difluoro-3,4-dihydroxyphényl)-méthoxy)imino)acétyl)amino)-2-carboxy-8-oxo-5-thia-1-azabicyclo(4.2.0)oct-2-en-3-yl)-2-propényl)-6,7 dihydro 5H-1-pyrindinium.

En opérant comme au stade H de l'exemple 1 à partir de 899 mg du produit obtenu au stade A ci-dessus, et en utilisant 8 ml d'une solution d'acide trifluoroacétique à 10 % d'anisole, on obtient 420 mg de produit recherché.
Spectre R.M.N. Diméthylsulfoxyde
2,24 (m)-3,14 (t)-3,37 (t) : pyrindinium ; 3,55-3,78 (d) : CH₂S ; 5,06 (s) : Φ-CH₂-O ; 5,19 (d) : H6 ; 5,81 (dd) H7 ; 5,34(m) : =CH-CH₂-N⁺ ; 6,29 (dt)-6,87 (d,j=16) : CH=CH (Δ E) ; 6,72 (dd,j=6,9 & 18) : H6'' (cycle difluoro) ; 6,78 (s) : H thiazole ; 7,92 (dd)-8,42 (d)-8,77 (d) : H'5,H'4 et H'6 ; 9,74 (d) : CO-NH-CH.

### EXEMPLE 7 : Sel interne de (6R(3(E),6α,7β(Z))) 1-3-(7-(((2-amino-4-thiazolyl) (((3,4-dihydroxy-5-fluorophényl) méthoxy) imino) acétyl) amino)-2-carboxy-8-oxo-5-thia-1-azabicyclo (4.2.0)oct-2-en-3-yl)-2-propényl)-quinoléinium.

### Stade A : Alcool 5-fluoro 3,4 bis(2-méthoxyéthoxy) méthoxy benzylique.

On opère comme au stade A de l'exemple 1 à partir de 9,2 g de 5-fluoro-3,4-dihydroxyphényl) méthoxy benzaldéhyde en utilisant 1,02 g de borohydrure de sodium. On recueille ainsi 7,7 g de produit recherché utilisé tel quel pour le stade suivant.

### Stade B : (5-fluoro-3,4 bis(2-méthoxyéthoxy) méthoxy) phényl) ((1,3-dihydro 1,3-dioxo-2H-isoindol-2yl) oxy) méthyle.

On opère comme au stade B de l'exemple 1 en utilisant 2,98 g de triphényl phosphine et 1,79 ml de azodicarboxylate de diéthyle, 1,38 g de N hydroxyphtalimide, à partir de 1,9 g du produit obtenu au stade A ci-dessus. On recueille 1,87 g de produit recherché.
Spectre I.R. :
1794cm⁻¹
1737 cm⁻¹ C=O
1620 cm⁻¹
1601 cm⁻¹ aromatique
1510 cm⁻¹
Spectre R.M.N. CDCl₃
3,36 (s)-3,38(s) : CH₃ de OMEM ; 3,56-3,89-3,96-5,12 : les CH₂ de OMEM : 7,01(s) à 7,75 : les aromatiques.

### Stade C : 5-fluoro 3,4-bis(2-méthoxyéthoxy) méthoxy oxyaminobenzyle.

On opère comme au stade C de l'exemple 1 à partir de 1,3 g de produit obtenu au stade B ci-dessus, en utilisant 0,2 ml d'hydrate d'hydrazine. Après chromatographie sur silice (éluant avec un mélange chlorure de méthylène-acétate d'éthyle 60-40), on recueille 710 mg de produit attendu. Spectre I.R. CHCl₃ :
3330cm⁻¹ : CH₂-ONH₂
1618, 1594, 1583 et 1509 cm⁻¹ : aromatique + NH₂ def. Spectre R.M.N. CDCl₃
3,37(s) et 3,38(s) : CH₃ ; 3,57(m)-3,84(m)-3,98(m)-5,21(s)-5,30(s) : les CH₂ de OMEM ; 4,59(s) : CH₂- ONH₂ ; 5,45(s,1) : NH₂ ; 6,81(dd)-6,98(m) : aromatique.

### Stade D : (Z) acide α (5-fluoro-3,4-bis((2-méthoxyéthoxy) méthoxy) phényl méthoxy imino)(2-((triphénylméthyl)amino) 4-thiazoleacétique.

On opère comme au stade D de l'exemple 1 à partir de 745 mg du produit obtenu au stade C et en utilisant 650 mg d'acide oxo-[2-[(triphénylméthyl) amino] thiazol-4-yl] acétique (décrit dans la demande de brevet belge n° 864828) pour obtenir 1,18 g de produit recherché.
Spectre I.R. CHCl₃
3404 cm⁻¹ : =C-NH
1593, 1577, 1530 et 1510 : Système conjugué + aromatique
Spectre R.M.N. CDCl₃
3,36(s)-3,38(s) : CH₃ de OMEM ; 3,58(m)-3,80(m) - 3,95(m) : les CH₂ de OMEM ; 5,18(s)-5,19(s)-5,25(s) : CH₂-O-N et O-CH2-O ; 6,62(s) : H thiazole ; 6,78 à 7,31 : aromatique ; 10,35 H mobile : CO2H.

### Stade E : [6R(3(E), 6α, 6β (Z))] 3-(3-chloro 1-propényl) 7-(((5-fluoro 3,4-bis ((2-méthoxyéthoxy) méthoxy) phényl méthoxy) imino)(2-((triphénylméthyl) amino) 4- thiazolyl) acétyl) amino) 8-oxo 5-thia 1-azabicyclo(4.2.0)oct-2-ène-2-carboxylate de (4-méthoxyphényl) méthyle.

On opère comme au stade E de l'exemple 1 à partir de 1,1 g du produit obtenu au stade D et en utilisant 699 mg de chlorhydrate de 7β-amino 3-[3-chloro 1-propényl) 8-oxo 5-thia 1-azabicyclo(4.2.0)oct-2-ène-2-carboxylate de (4-méthoxy phényl) méthyle (décrit dans la demande de brevet européen n° 0333154), et 336 mg de diméthylamino propyl éthyl carbodiimide, après chromatographie sur silice (éluant chlorure de méthylène-éther éthylique 85-15), on recueille 860 mg de produit recherché utilisé tel quel pour le stade suivant.

### Stade F : [6R(3(E), 6α, 7β (Z))] 3-(3-iodo 1-propényl) 7-(((5-fluoro 3,4-bis((2-méthoxyéthoxy)méthoxy) phényl méthoxy) imino)(2-((triphénylméthyl)amino) 4-thiazolyl) acétyl) amino) 8-oxo 5-thia 1-azabicyclo(4.2.0)oct-2-ène-2-carboxylate de (4-méthoxyphényl)méthyle.

On opère comme au stade F de l'exemple 1 à partir de 720 mg du produit obtenu au stade E, ci-dessus, en utilisant 286 mg d'iodure de sodium et un cristal d'iode. On obtient 770 mg de produit recherché que l'on utilise tel quel pour le stade suivant.

### Stade G : (6R(3(E), 6α,7β (Z))) iodure de 1-(3-(7-(((((5-fluoro 3,4-bis((2-méthoxyéthoxy)méthoxy) phényl) méthoxy) imino)(2-((triphénylméthyl)amino) 4-thiazolyl) acétyl) amino) 2-(((4-méthoxyphényl) méthoxy) carbonyl) 8-oxo-5-thia-1-azabicyclo(4.2.0)oct-2-en-3-yl)-2-propényl)-quinoléinium.

On agite 1 heure à température ambiante 770 mg du produit obtenu au stade F ci-dessus avec 0,43 ml de quinoléine redistillée. On précipite par ajout d' éther éthylique, essore, lave à l'éther et sèche sous pression réduite. On recueille 820 mg de produit que l'on chromatographie sur silice en éluant avec un mélange chlorure de méthylène-méthanol 96-04. On recueille 231mg de produit recherché.
Spectre R.M.N. CDCl₃
3,78- 3,93(s) : CH₃ de OMEM ; 3,42(m)-3,54(m) et 3,72 à 5,22 : les CH₂ de OMEM et CH₂-O-N= ; 6,86(d,mobile) : CO-NH ; 6,48 (s) : H thiazole ; 7,28 : trityl ; 4,98(d) : N-CH-CH-S ; 5,84(d) : N-CH-CH-S ; 6,70 à 6,97 : H aromatiques ; 5,28 : CH₂-Φ ; 3,52(d)-3,61(d) : S-CH₂- ; 6,42(dt) : CH propényl ; 6,03(dd) et 6,17(dd) : CH₂ propényl ; 8,14 à 10,4(d) : 7H quinoléine.

### Stade H : Sel interne de (6R(3(E),6α,7β(Z))) 1-3-(7-(((2-amino-4-thiazolyl)(((-3,4-dihydroxy-5-fluorophényl) méthoxy) imino) acétyl) amino)-2-carboxy-8-oxo-5-thia-1-azabicyclo (4.2.0)oct-2-en-3-yl)-2-propényl)-quinoléinium.

On opère comme au stade H de l'exemple 1 à partir de 120 mg du produit obtenu au stade G, en utilisant 1,2 ml d'acide trifluoroacétique à 10 % d'anisole, on recueille ainsi 31 mg de produit recherché.
Spectre R.M.N. Diméthylsulfoxyde
6,59(m) : H aromatique, 4,92(s) : -CH₂-O-N= ; 9,66(d,mob.) : NH ; 6,72(s) : H thiazole ; 5,17(d,) : N-CH-CH-S ; 5,78(m) : N-CH-CH-S ; 3,52-3,74(AB) : S-CH₂- ; 6,98(d,j=15,5) : CH propényl ; 6,38 : l'autre CH propényl ; 5,90(m) : CH₂ propényl ; 8,27 à 9,57 : 7H quinoléine.

En plus des produits décrits ci-dessus dans les exemples, les produits répondant à la formule ci-dessous et résultant des combinaisons des différentes valeurs des substituants représentées dans les tableaux qui suivent, constituent des produits pouvant être obtenus selon l'invention.

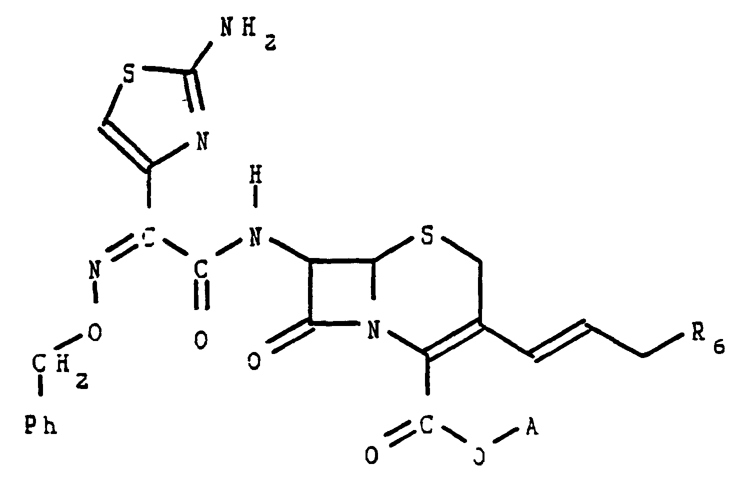

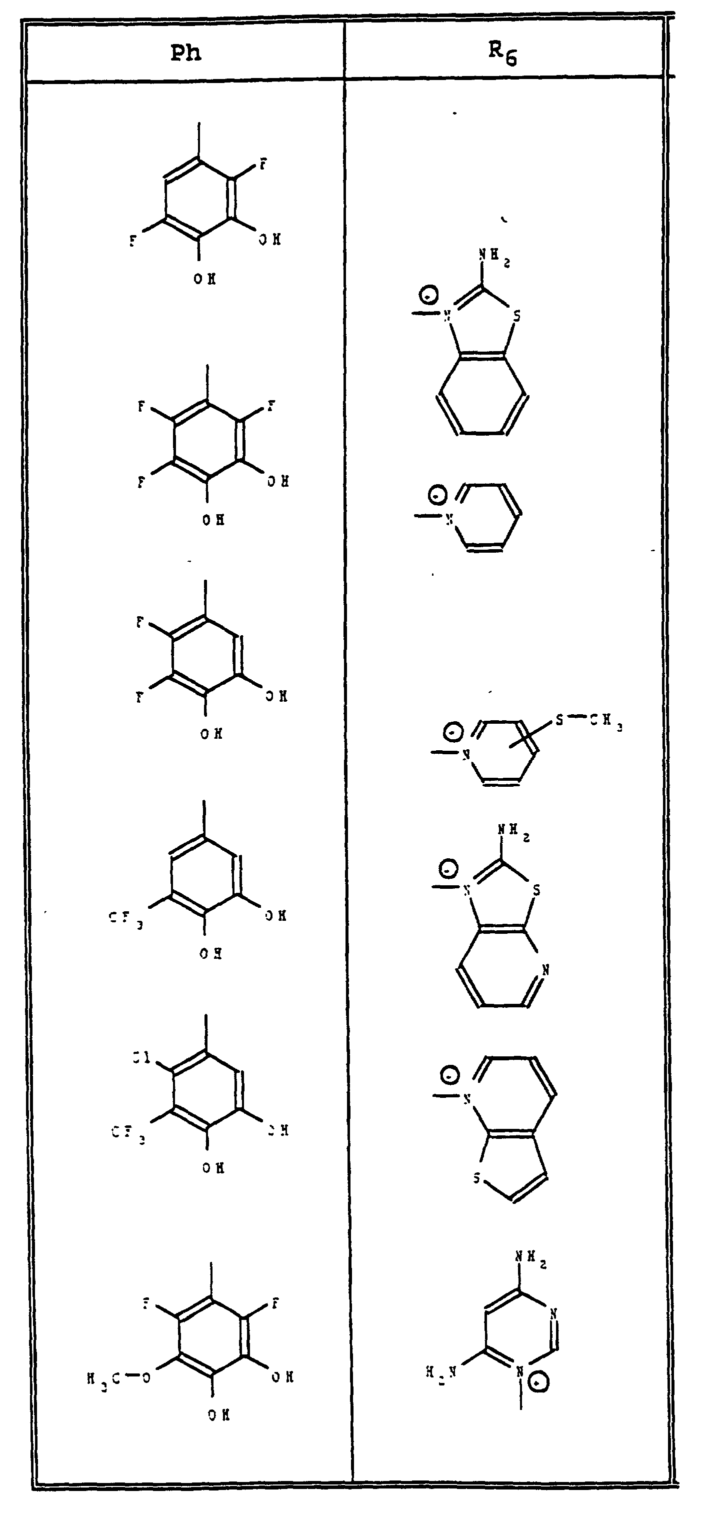

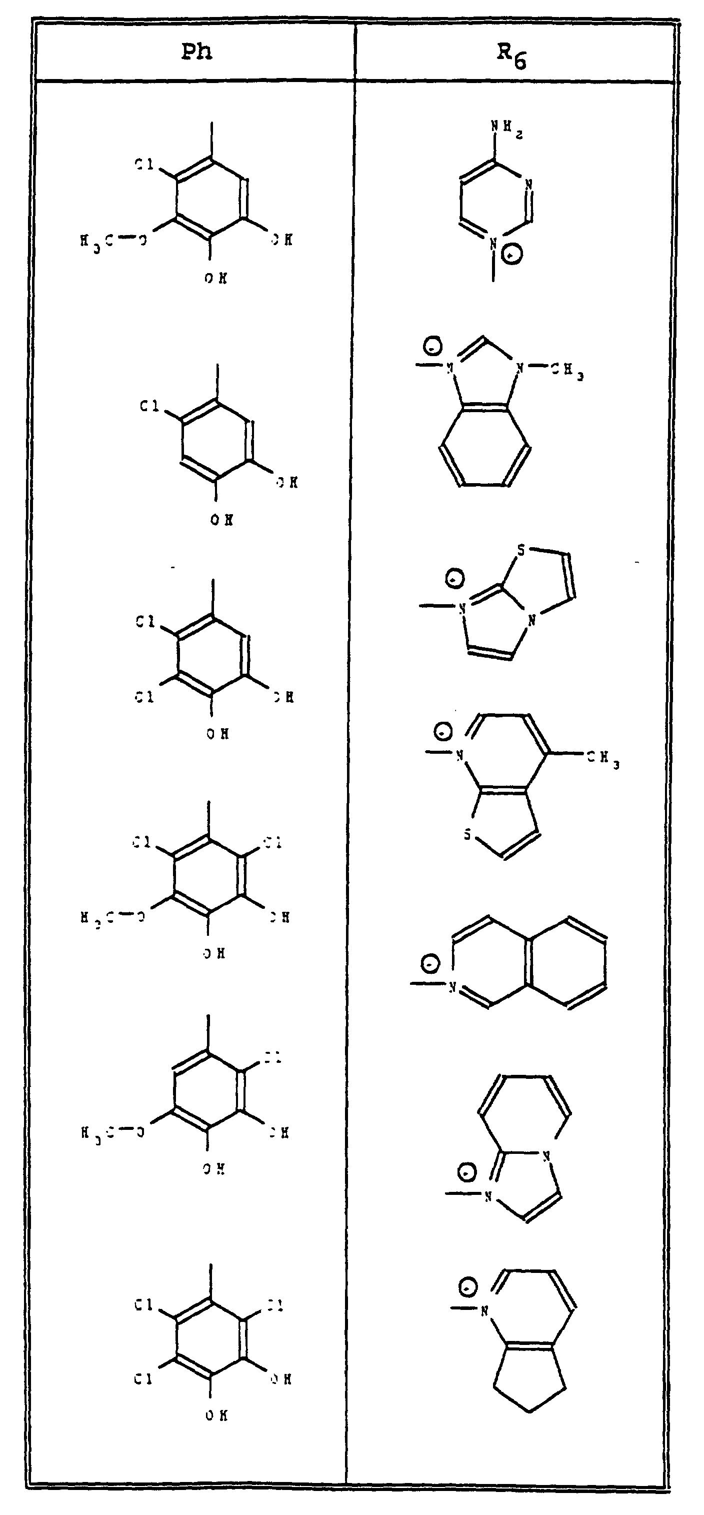

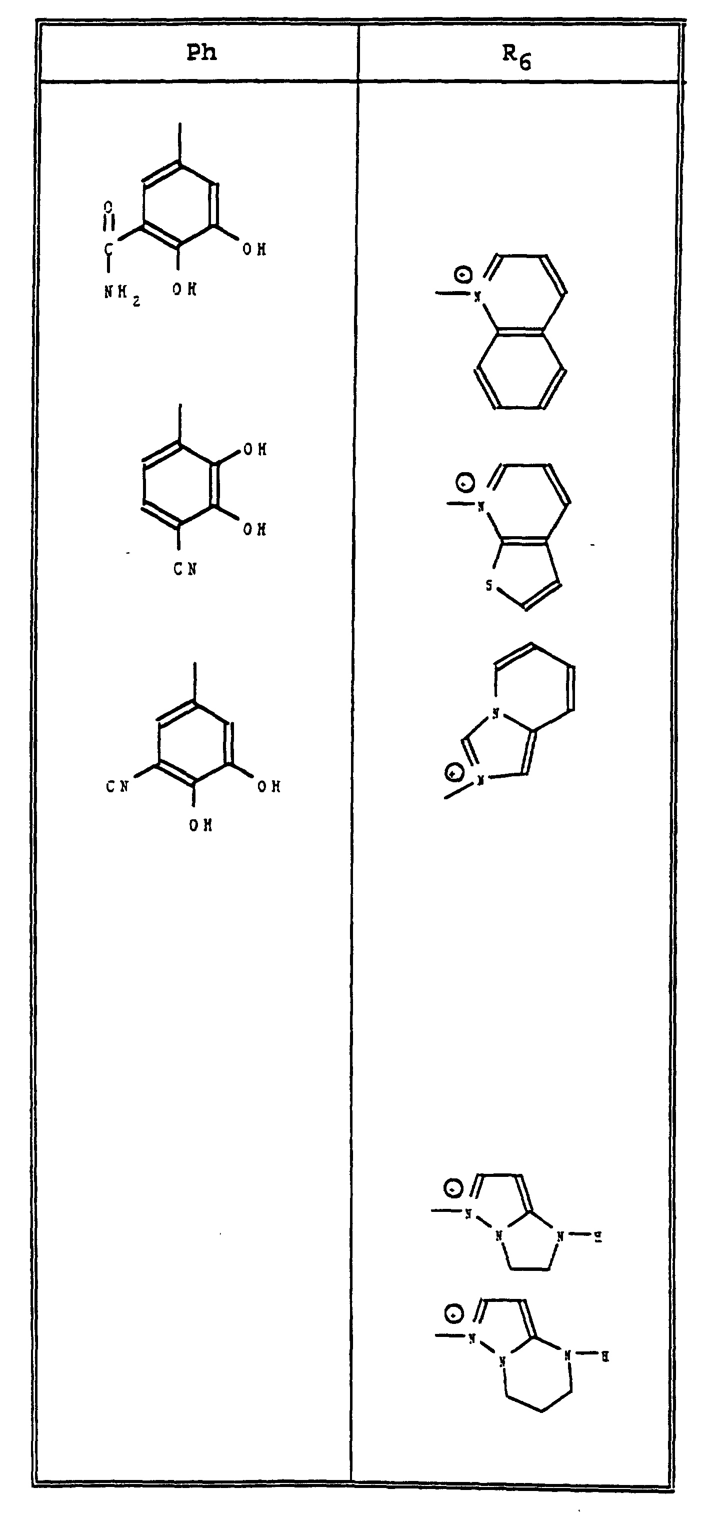

### EXEMPLE 8 :

**On a réalisé des préparations pour injections de formule :**
- Produit de l'exemple 1 500 mg
- Excipient aqueux stérile q.s.p. 5 cm³

### ETUDE PHARMACOLOGIQUE DES PRODUITS DE L'INVENTION

### Activité in vitro, méthode des dilutions en milieu solide.

On prépare une série de boîtes dans lesquelles on répartit une même quantité de milieu nutritif stérile, contenant des quantités croissantes du produit à étudier puis chaque boîte est ensemencée avec plusieurs souches bactériennes.

Après incubation de 24 heures en étuve à 37°C, l'inhibition de la croissance est appréciée par l'absence de tout développement bactérien ce qui permet de déterminer les concentrations minimales inhibitrices (CMI) exprimées en microgrammes/cm³.

Les résultats sont exprimés sous forme de moyennes géométriques de toutes les CMI obtenues, pour un groupe de souches particulier.

On a obtenu les résultats suivants (tableau) :

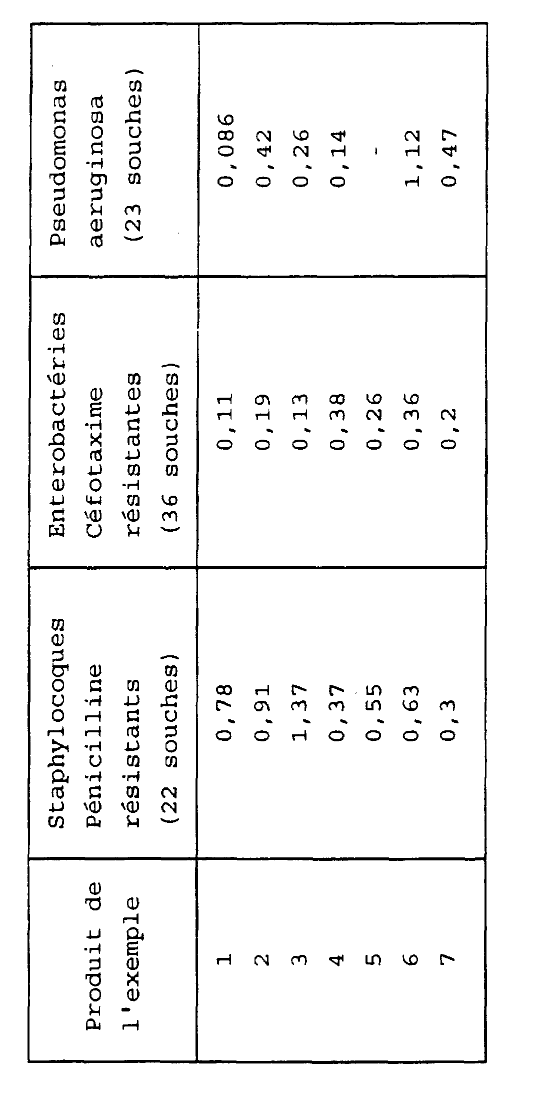

## Revendications

1. Les produits de formule générale (I) 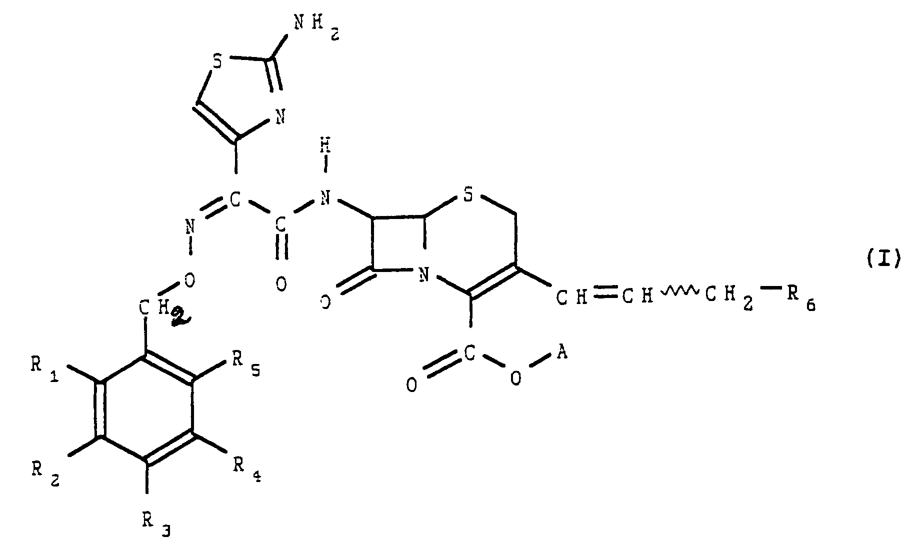 isomère syn, sous forme de sels internes ou de sels avec les acides minéraux ou organiques ou avec les bases, formule dans laquelle :
R₁, R₂, R₃ et R₅, identiques ou différents, représentent un atome d'hydrogène, un atome d'halogène ou un radical choisi dans le groupe constitué par les radicaux hydroxy, alkyle renfermant de 1 à 4 atomes de carbone éventuellement substitué par un ou plusieurs atomes d'halogène, alkyloxy renfermant de 1 à 4 atomes de carbone, mercapto, alkylthio renfermant de 1 à 4 atomes de carbone, nitro, cyano, amino, alkylamino renfermant de 1 à 4 atomes de carbone, dialkylamino renfermant de 2 à 8 atomes de carbone, carbamoyle, (alkylamino) carbonyle renfermant de 2 à 5 atomes de carbone, (dialkylamino) carbonyle renfermant de 3 à 9 atomes de carbone, carboxy, alcoxycarbonyle renfermant de 2 à 5 atomes de carbone, acyloxy renfermant de 1 à 8 atomes de carbone et 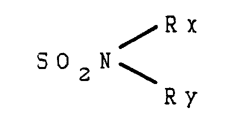 dans lequel Rx et Ry identiques ou différents representent un atome d'hydrogène ou un radical alkyle renfermant de 1 à 4 atomes de carbone,
R₄ représente un radical hydroxy ou un radical acyloxy renfermant de 1 à 8 atomes de carbone,
A représente un atome d'hydrogène, un équivalent de métal alcalin, alcalino-terreux, de magnésium, d'ammonium ou d'une base organique aminée, ou A représente le reste d'un groupement ester facilement clivable, ou bien CO₂A représente CO₂^{⊖}, le trait ondulé signifie que le groupement CH₂R₆ peut se trouver dans la position E ou Z et R₆ représente, sous forme d'ammonium quaternaire, un des radicaux suivants : 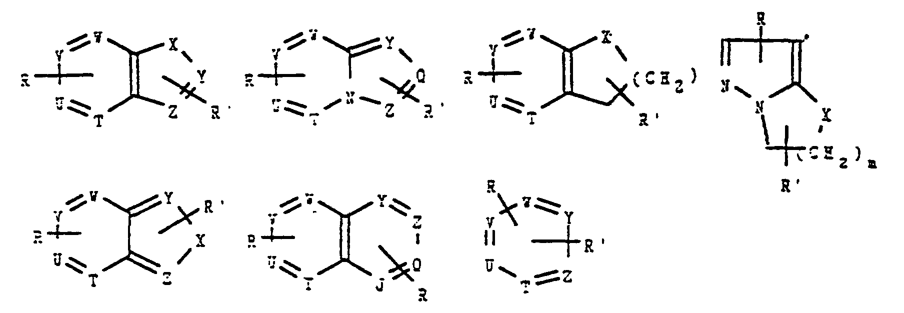 dans lesquels m est égal à 1, 2 ou 3, X représente CH₂, NH, O ou S ;
Q, J, Y, T, U, V, W et Z, identiques ou différents, représentent indépendamment les uns des autres CH ou
N, étant entendu que chacun de ces radicaux cycliques contient de 1 à 5 hétéroatomes, qu'au moins un de ces hétéroatomes est l'atome d'azote et que ces radicaux cycliques peuvent être substitués par un ou plusieurs radicaux R ou R' ;
R et R', identiques ou différents, représentent un atome d'hydrogène, un radical alkyle renfermant de 1 à 4 atomes de carbone, un radical alkyloxy renfermant de 1 à 4 atomes de carbone, un atome d'halogène, un radical cyano, un radical CO₂-Q₁, CO-NQ₁(Q₂), NQ₁(Q₂), SO₂-NQ₁(Q₂), CS-NH₂, NH-CO-Q₁, CH=N-OH, CH=N-O-Q₁, CH₂-CN, CH₂-S-Q₁, S-Q₁, dans lesquels Q₁ et Q₂, identiques ou différents, représentent un atome d'hydrogène ou un radical alkyle renfermant de 1 à 4 atomes de carbone.

2. Les produits de formule générale (I) telle que définie à la revendication 1, dans laquelle R₆ représente un des radicaux suivants :

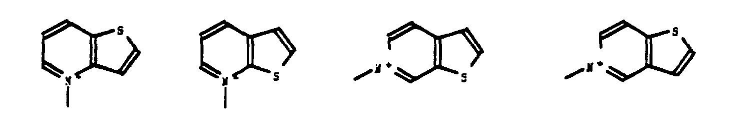

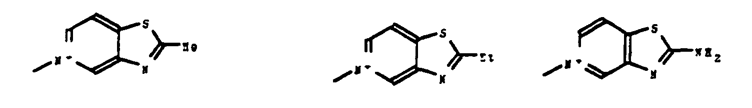

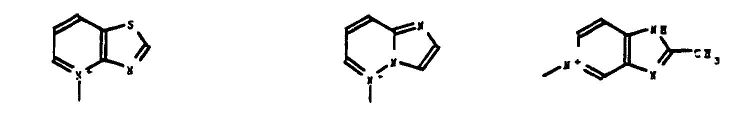

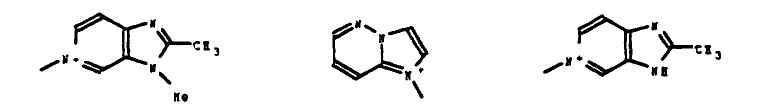

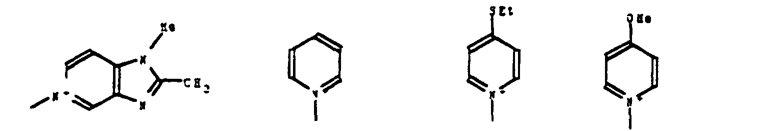

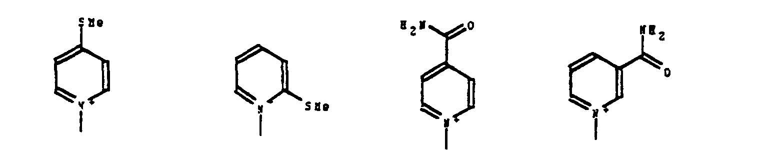

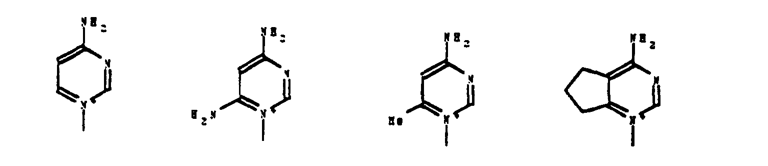

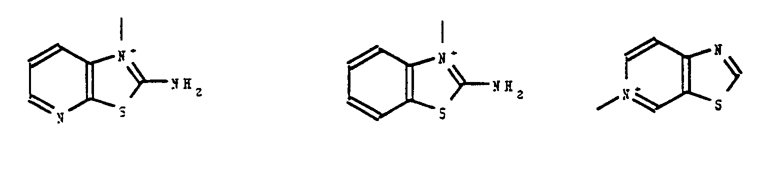

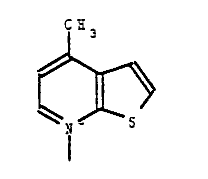

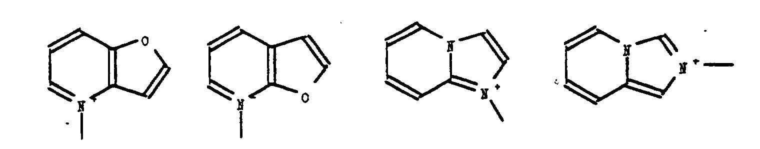

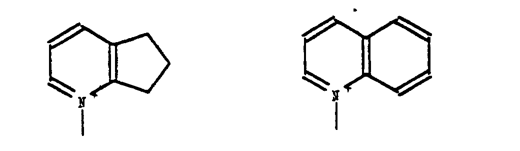

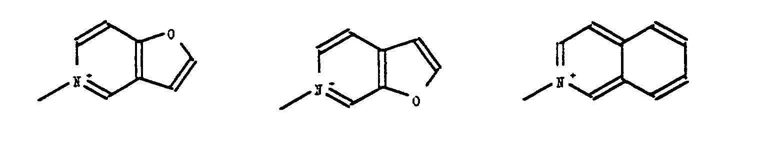

3. Les produits de formule générale (I) telle que définie à la revendication 1 ou 2, dans laquelle R₆ représente le radical quinoléinium, isoquinoléinium, 4-(méthylthio) pyridinium, thièno[2,3-b]pyridinium, imidazo(1,2-a)pyridinium ou le radical 6,7-dihydro-5H-1-pyrindinium.

4. Les produits de formule générale (I) telle que définie à l'une quelconque des revendications 1, 2 ou 3, dans laquelle R₃ et R₄ représentent chacun un radical hydroxy.

5. Les produits de formule générale (I) telle que définie à l'une quelconque des revendications 1, 2, 3 ou 4, dans laquelle R₂ et R₅ représentent chacun un atome de chlore ou de fluor.

6. Les produits de formule générale (I) telle que définie à l'une quelconque des revendications 1, 2, 3 ou 4, -dans laquelle R₂ représente un atome de fluor.

7. Les produits de formule générale (I) telle que définie à l'une quelconque des revendications 1, 2, 3 ou 4, dans laquelle R₂ représente un radical méthoxy et l'un de R₁ ou R₅ représente un atome de chlore.

8. Les produits de formule générale (I) selon la revendication 1, dont les noms suivent :
- le sel interne de (6R-(3-(E) 6alpha, 7béta-(Z))) 1-(3-(7-(((2-amino 4-thiazolyl) ((2,5-difluoro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium,
- le sel interne de (6R-(3-(E) 6alpha, 7béta-(Z))) 1-(3-(7-(((2-amino 4-thiazolyl) ((2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium,
- le sel interne de (6R-(3-(E) 6alpha, 7béta-(Z))) 1-(3-(7-(((2-amino 4-thiazolyl) ((2,5-difluoro 3,4-dihydroxyphényl) méthoxy) imino) acétyl) amino) 2-carboxy 8-oxo 5-thia 1-azabicyclo[4,2,0]oct-2-èn-3-yl) 2-propényl) imidazo(1,2-a) pyridinium,
- le sel interne de (6R-(3-(E) 6alpha, 7béta-(Z))) 1-(3-(7-(((2-amino 4-thiazolyl) (((2,5-dichloro 3,4-dihydroxyphényl) méthoxy) imino) acétyl) amino)2-carboxy 8-oxo 5-thia 1-azabicyclo[4,2,0]oct-2-èn-3-yl) 2-propényl) imidazo(1,2-a) pyridinium,
- le sel interne de (6R-(3-(E) 6alpha, 7béta-(Z))) 1-(3-(7-(((2-amino 4-thiazolyl) (((2,5-difluoro 3,4-dihydroxyphényl) méthoxy) imino) acétyl) amino) 2-carboxy 8-oxo 5-thia 1-azabicyclo[4,2,0]oct-2-èn-3-yl) 2-propényl) 6,7-dihydro 5H--1-pyrindinium,
- le sel interne de (6R-(3(E), 6alpha, 7béta(Z))) 1-(3-(7-(((2-amino 4-thiazolyl) ((2,5-dichloro 3,4-dihydroxyphényl) méthoxy) imino) acétyl) amino) 2-carboxy 8-oxo 5-thia 1-azabicyclo[4,2,0]oct-2-èn-3-yl) 2-propényl) 6,7-dihydro 5H-1-pyrindinium,
- le sel interne de (6R-(3(E), 6alpha, 7béta(Z))) 1-(3-(7-(((2-amino 4-thiazolyl) ((5-fluoro 3,4-dihydroxyphényl) méthoxy) imino) acétyl) amino) 2-carboxy 8-oxo 5-thia 1-azabicyclo[4,2,0]oct-2-èn-3-yl) 2-propényl) quinoléinium.

9. Procédé de préparation des produits de formule (I) telle que définie à la revendication 1, **caractérisé en ce que** l'aldéhyde aromatique de formule (II) : 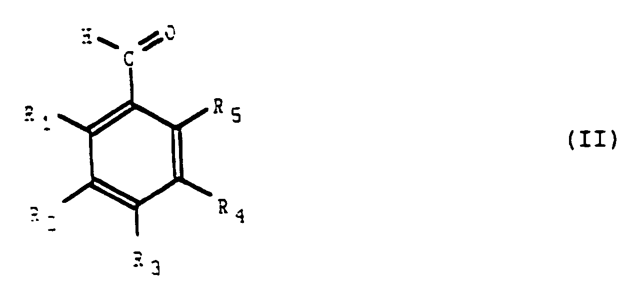 dans laquelle R₁, R₂, R₃, R₄ et R₅ sont tels que définis à la revendication 1, est si nécessaire protégé en ses fonctions réactives et transformé ainsi en aldéhyde aromatique de formule (IIₚ) : 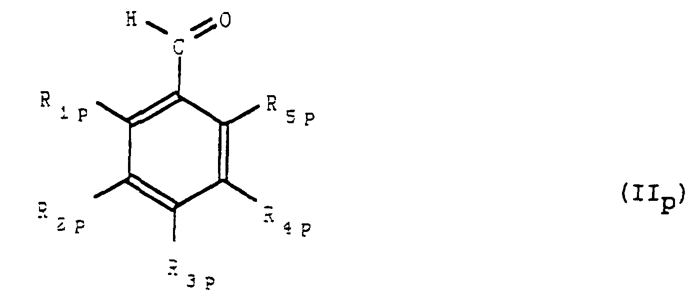 dans laquelle R₁ₚ, R₂ₚ, R₃ₚ, R₄ₚ et R₅ₚ représentent respectivement les valeurs de R₁, R₂, R₃, R₄ et R₅ telles que définies précédemment ou une fonction réactive protégée, ledit aldéhyde de formule (IIₚ) est traité par un agent réducteur, pour obtenir l'alcool de formule (III) : 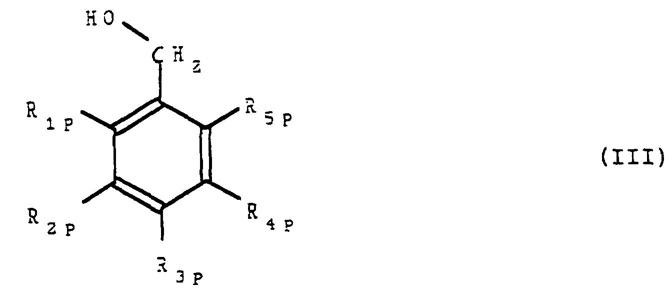 alcool de formule (III) que l'on traite par le N-hydroxy phtalimide, le cas échéant en présence d'un agent activant, pour obtenir le dérivé de formule (IV) : 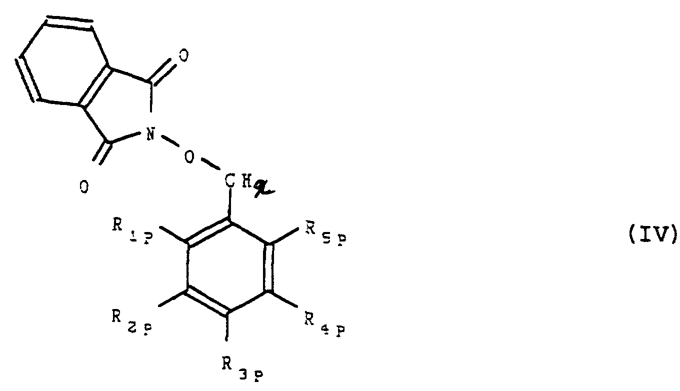 que l'on hydrolyse en hydroxylamine O-substituée de formule (V) : 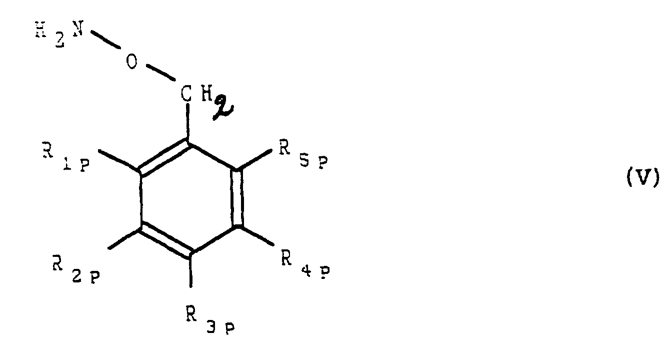 que l'on condense avec un dérivé de l'acide 2-(2-amino thiazol-4-yl) 2-oxo acétique de formule (VI) 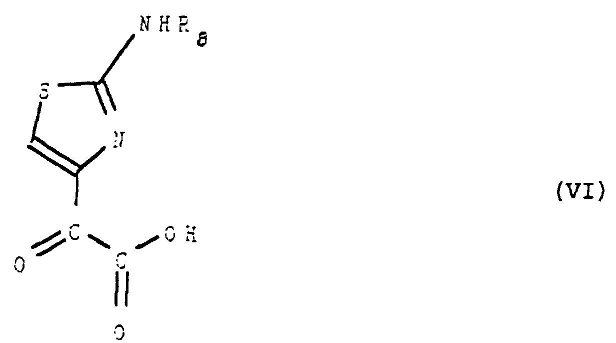 dans laquelle R₈ représente un atome d'hydrogène ou un groupe protecteur de la fonction amine, pour former le dérivé de l'acide alpha-alkoxyimino acétique "syn" de formule (VII) : 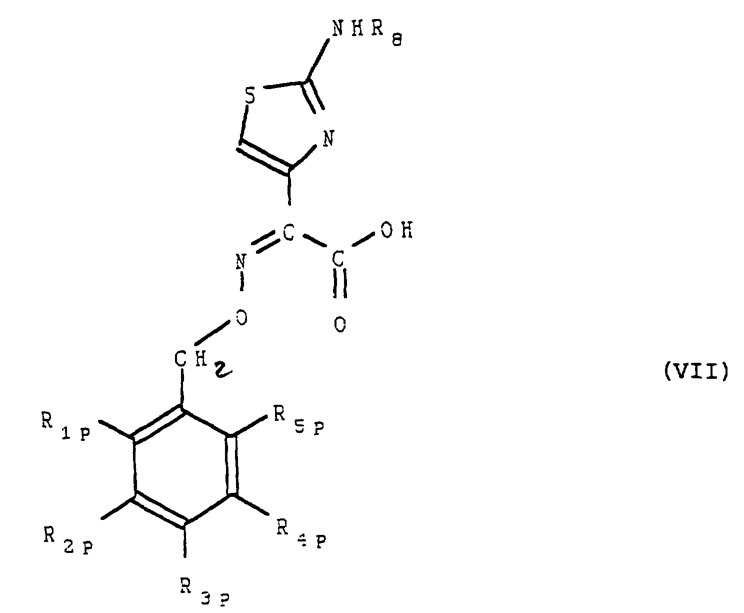 dont on prépare le cas échéant, un dérivé fonctionnel, produit de formule (VII) ou dérivé fontionnel que l'on amidifie avec un ester du chlorhydrate de l'acide 7-amino 3-(3-halo 1-propényl) 8-oxo 5-thia 1-azabicyclo[4,2,0]oct-2-èn-2-carboxylique de formule (VIII) 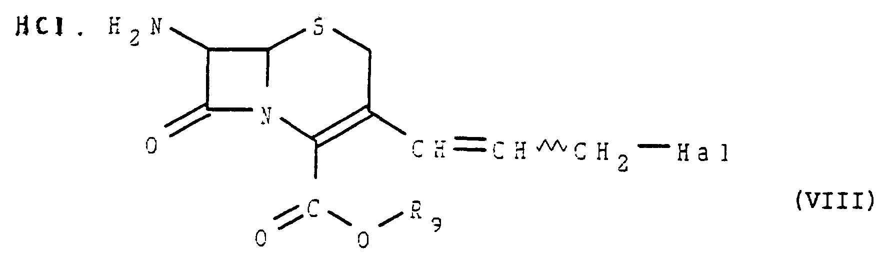 dans laquelle R₉ représente le reste d'un ester aisément clivable, pour conduire au dérivé de l'acide 7-(N-substitué amido) 3-(3-halo 1-propényl) 8-oxo 5-thia 1-azabicyclo[4,2,0]oct-2-èn-2-carboxylique de formule (IX) : 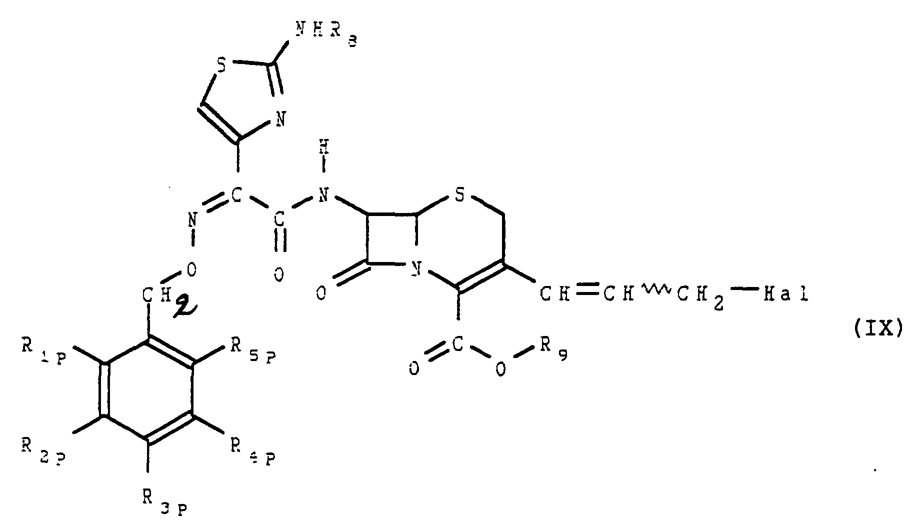 que l'on transforme, le cas échéant, en analogue 3-(3-iodo propényle) de formule (X) : 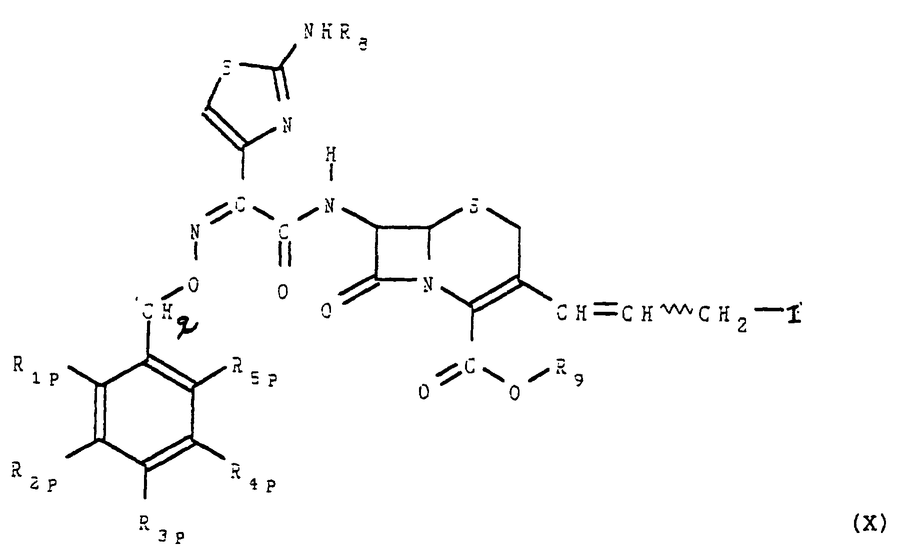 que l'on traite avec une base de formule R₆ pour obtenir le produit de formule (XI) : 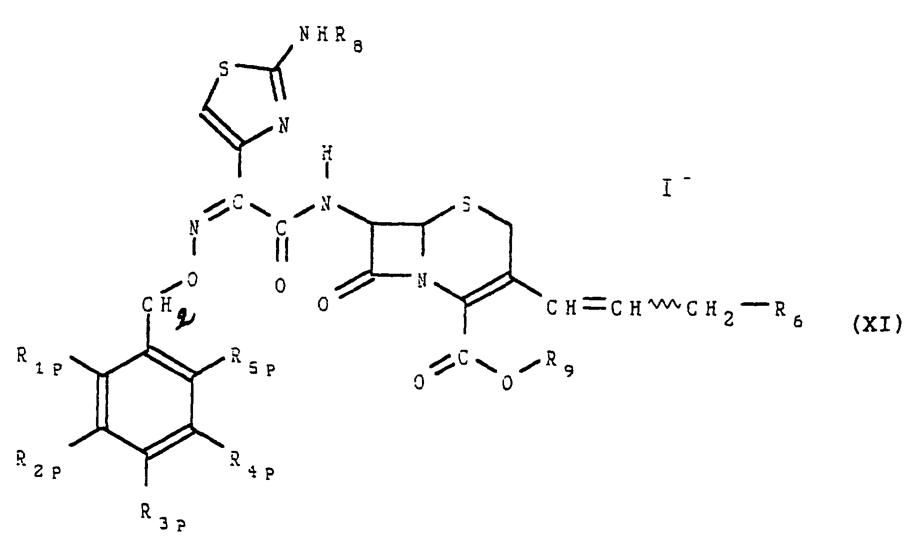 produit de formule (XI) à partir duquel le cas échéant, l'on isole les isomères (E) ou (Z) ou transforme les isomères (Z) en isomères (E), produit de formule (XI) que l'on soumet le cas échéant, à une ou plusieurs des réactions suivantes dans un ordre approprié :
a) coupure par hydrolyse ou par action de la thiourée de tout ou partie des groupements protecteuts esters et des groupements de protection du radical amino ou des radicaux hydroxyles,
b) estérification ou salification par une base du radical carboxylique,
c) salification par un acide du radical amino,
d) séparation des produits sous forme de mélange R,S en R et S.

10. Variante du procédé selon la revendication 9, **caractérisé en ce que** l'hydroxylamine O-substituée de formule (V) est condensée au produit de formule (XII) : 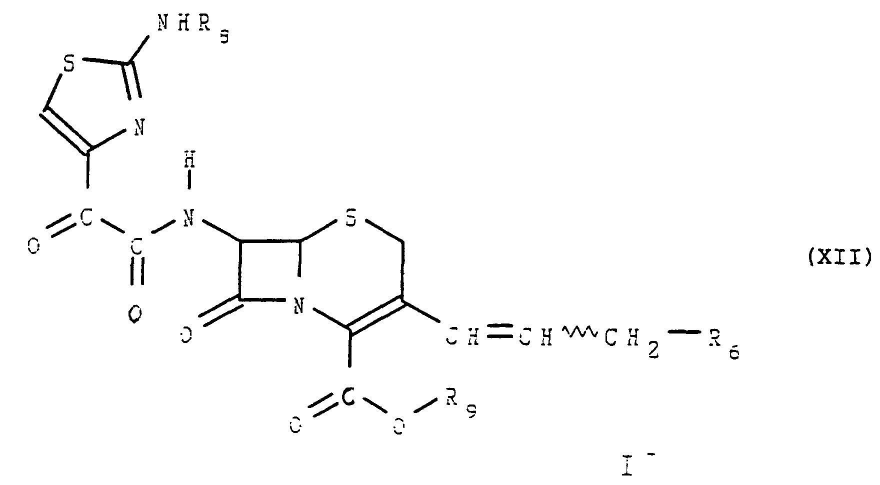 pour conduire au produit de formule (XI) telle que définie à la revendication 9.

11. A titre de médicaments, les produits répondant à la formule (I) telle que définie à la revendication 1, dans laquelle R₁, R₂, R₃, R₄, R₅, R₆ et A sont tels que définis à la revendication 1 ainsi que leurs sels d'acides, pharmaceutiquement acceptables.

12. A titre de médicaments, les produits tels que définis à l'une quelconque des revendications 2 à 8, ainsi que leurs sels d'acides, pharmaceutiquement acceptables.

13. Compositions pharmaceutiques contenant, à titre de principe actif, au moins un médicament selon l'une des revendications 11 ou 12.

14. A titre de produits industriels nouveaux, les produits de formule (IV) : 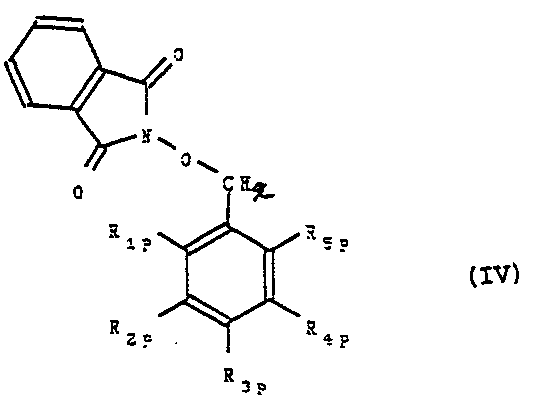 dans laquelle R₁ₚ, R₂ₚ, R₃ₚ, R₄ₚ et R₅ₚ sont définis comme à la revendication 9.

15. A titre de produits industriels nouveaux, les produits de formule (V) : 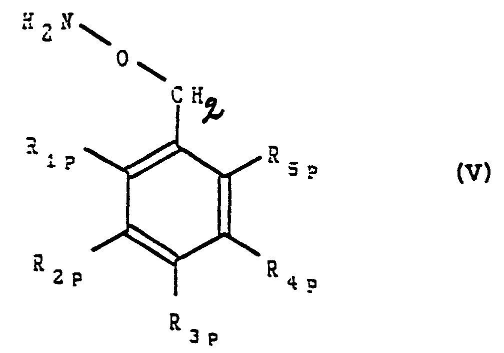 dans laquelle R₁ₚ, R₂ₚ, R₃ₚ, R₄ₚ et R₅ₚ sont définis comme à la revendication 9.

16. A titre de produits industriels nouveaux, les produits de formule (VII) : 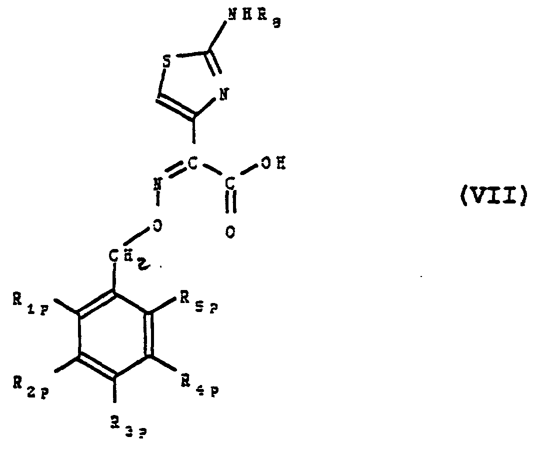 dans laquelle R₁ₚ, R₂ₚ, R₃ₚ, R₄ₚ, R₅ₚ et R₈ sont définis comme à la revendication 9.

17. A titre de produits industriels nouveaux, les produits de formule (IX) : 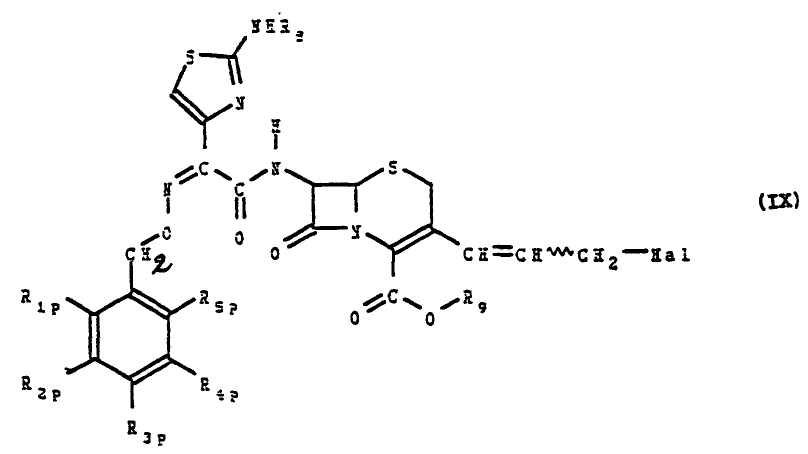 dans laquelle R₁ₚ, R₂ₚ, R₃ₚ, R₄ₚ, R₅ₚ, R₈ et R₉ sont définis comme à la revendication 9 et Hal représente un atome d'halogène.

18. A titre de produits industriels nouveaux selon la revendication 17, les produits de formule (X) 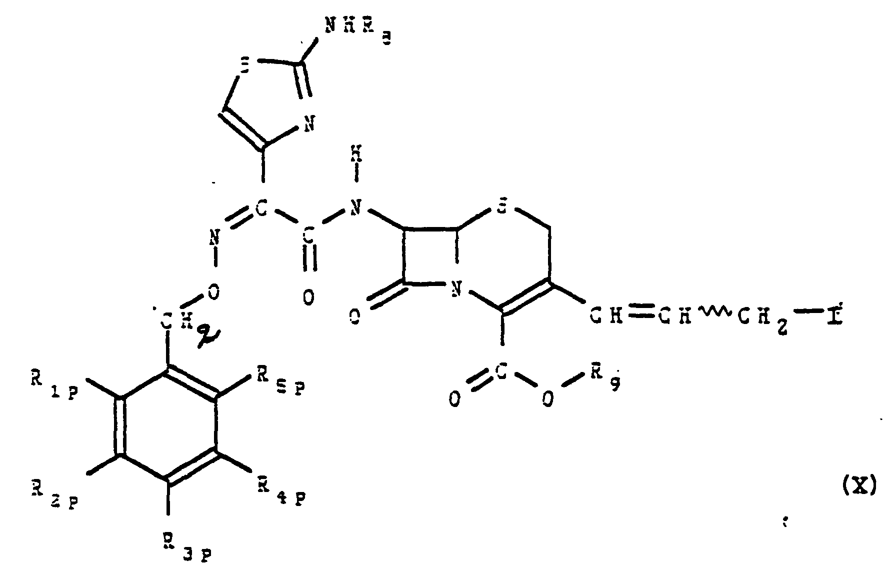 dans laquelle R_{1p,} R₂ₚ, R₃ₚ, R₄ₚ, R₅ₚ, R₈ et R₉ sont définis comme à la revendication 9.

19. A titre de produits industriels nouveaux, les produits de formule (XI) : 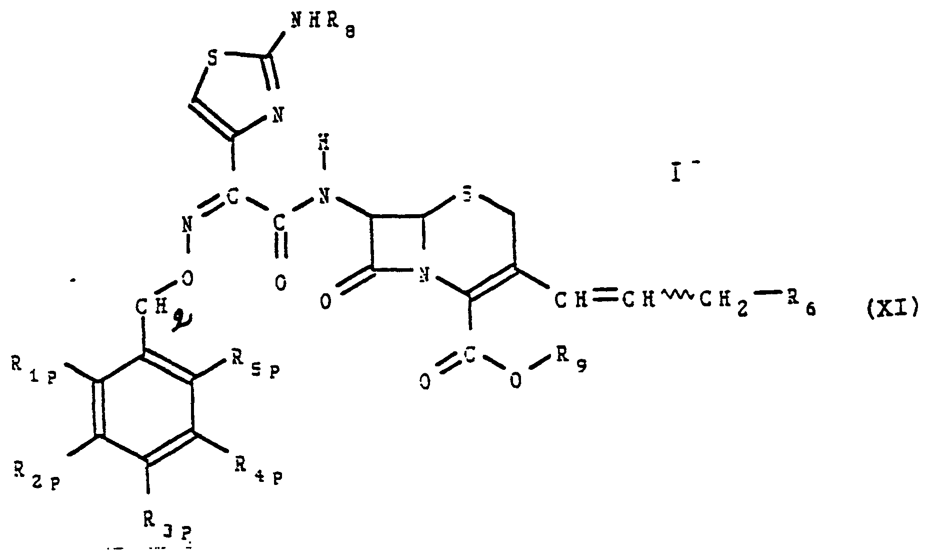 dans laquelle R₁ₚ, R₂ₚ, R₃ₚ, R₄ₚ, R₅ₚ, R₈ et R₉ sont définis comme à la revendication 9 et R₆ est défini comme à la revendication 1.

## Patentansprüche

1. Verbindungen der allgemeinen Formel (I): 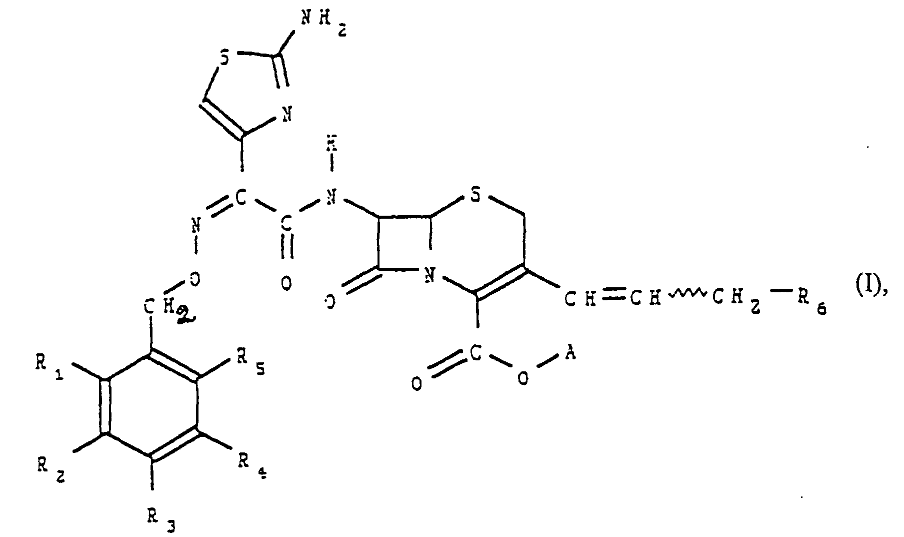 syn-Isomer, in Form der inneren Salze oder in Form von Salzen mit anorganischen oder organischen Säuren oder mit Basen, wobei in der Formel:
die Gruppen R₁, R₂, R₃ und R₅, die identisch oder voneinander verschieden sind, ein Wasserstoffatom, ein Halogenatom oder eine Gruppe bedeuten, die unter den folgenden Gruppen ausgewählt ist: Hydroxy, Alkylgruppen mit 1 bis 4 Kohlenstoffatomen, die gegebenenfalls mit einem oder mehreren Halogenatomen substituiert sind, Alkyloxy mit 1 bis 4 Kohlenstoffatomen, Mercapto, Alkylthio mit 1 bis 4 Kohlenstoffatomen, Nitro, Cyano, Amino, Alkylamino mit 1 bis 4 Kohlenstoffatomen, Dialkylamino mit 2 bis 8 Kohlenstoffatomen, Carbamoyl, (Alkylamino)carbonyl mit 2 bis 5 Kohlenstoffatomen, (Dialkylamino)carbonyl mit 3 bis 9 Kohlenstoffatomen, Carboxy, Alkoxycarbonyl mit 2 bis 5 Kohlenstoffatomen, Acyloxy mit 1 bis 8 Kohlenstoffatomen oder 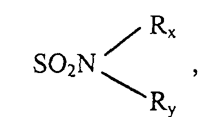 worin die Gruppen Rₓ und R_{y}, die identisch oder voneinander verschieden sind, ein Wasserstoffatom oder eine Alkylgruppe mit 1 bis 4 Kohlenstoffatomen bedeuten;
R₄ eine Hydroxygruppe oder eine Acyloxygruppe mit 1 bis 8 Kohlenstoffatomen bedeutet;
A ein Wasserstoffatom oder ein Äquivalent von Alkalimetallen, Erdalkalimetallen, Magnesium, Ammonium oder einer aminierten organischen Base bedeutet, oder A bedeutet den Rest einer leicht abspaltbaren Estergruppe oder die Gruppe CO₂A bedeutet CO₂⁻;
die Schlangenlinie bedeutet, daß sich die Gruppe CH₂R₆ in E- oder Z-Stellung befinden kann; und
R₆ in Form einer quartären Ammoniumverbindung eine der folgenden Gruppen bedeutet: 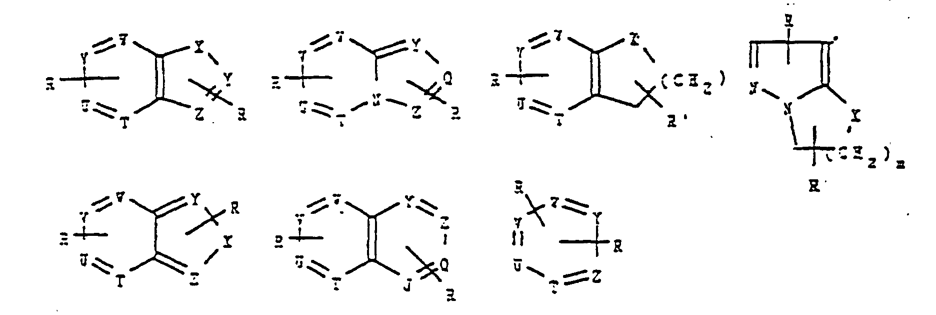 worin bedeuten:
m 1, 2 odes 3;
die Gruppe X CH₂, NH, O oder S; und
die Gruppen Q, J, Y, T, U, V, W und Z, die identisch oder voneinander verschieden sind, unabhängig voneinander CH oder N;
mit der Maßgabe, daß alle diese cyclischen Gruppen 1 bis 5 Heteroatome aufweisen, mindestens ein Heteroatom Stickstoff bedeutet und die cyclischen Gruppen mit einer oder mehreren Gruppen R oder R' substituiert sein können;
die Gruppen R und R', die identisch oder voneinander verschieden sind, bedeuten ein Wasserstoffatom, Alkyl mit 1 bis 4 Kohlenstoffatomen, Alkyloxy mit 1 bis 4 Kohlenstoffatomen, Halogen, Cyano, CO₂-Q₁, CO-NQ₁(Q₂), NQ₁(Q₂), SO₂-NQ₁(Q₂), CS-NH₂, NH-CO-Q₁, CH=N-OH, CH=N-O-Q₁, CH₂-CN, CH₂-S-Q₁ und S-Q₁, worin Q₁ und Q₂, die identisch oder voneinander verschieden sind, Wasserstoff oder eine Alkylgruppe mit 1 bis 4 Kohlenstoffatomen bedeuten.

2. Verbindungen der allgemeinen Formel (I) nach Anspruch 1, wobei R₆ eine der folgenden Gruppen bedeutet:

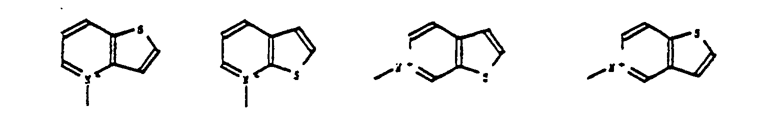

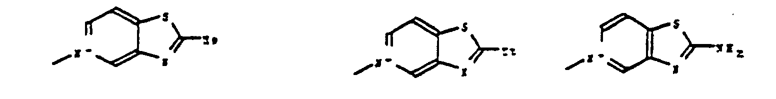

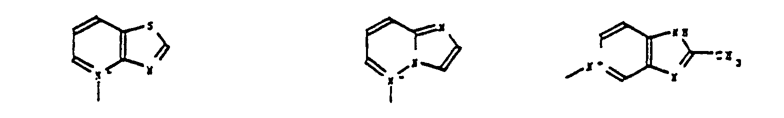

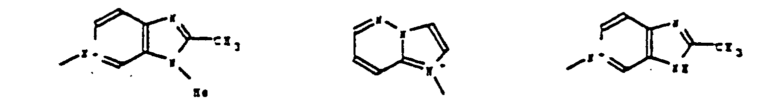

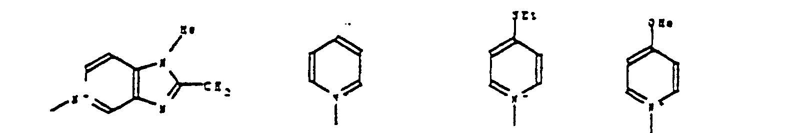

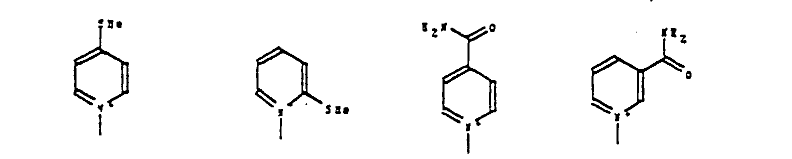

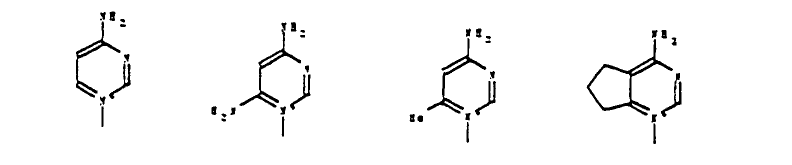

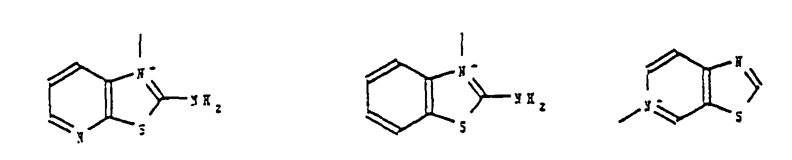

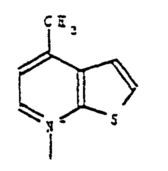

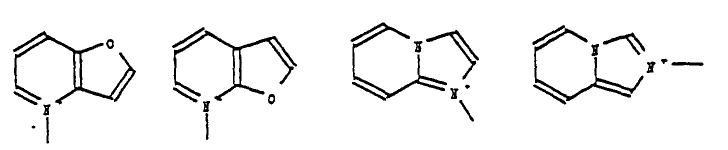

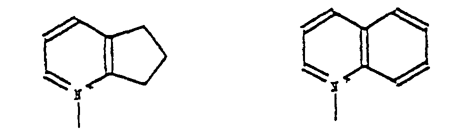

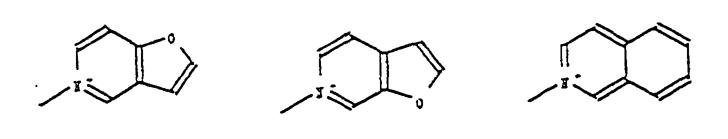

3. Verbindungen der allgemeinen Formel (I) nach Anspruch 1 oder 2, worin die Gruppe R₆ die Gruppen Chinolinium, Isochinolinium, 4-(Methylthio)-pyridinium, Thieno[2,3-b]pyridinium, Imidazo(1,2-a)pyridinium oder 6,7-Dihydro-5H-1-pyridiniumbedeutet.

4. Verbindungen der allgemeinen Formel (I) nach einem der Ansprüche 1, 2 oder 3, worin R₃ und R₄ jeweils Hydroxy bedeuten.

5. Verbindungen der allgemeinen Formel (I) nach einem der Ansprüche 1, 2, 3 oder 4, worin R₂ und R₅ jeweils ein Chloratom oder ein Fluoratom bedeuten.

6. Verbindungen der allgemeinen Formel (I) nach einem der Ansprüche 1, 2, 3 oder 4, worin R₂ ein Fluoratom bedeutet.

7. Verbindungen der allgemeinen Formel (I) nach einem der Ansprüche 1, 2, 3 oder 4, worin R₂ Methoxy und eine der Gruppen R₁ oder R₅ ein Chloratom bedeutet.

8. Verbindungen der allgemeinen Formel (I) nach Anspruch 1 mit den folgenden Bezeichnungen:
- inneres Salz von (6R-(3-(E) 6alpha, 7beta-(Z)))-1-(3-(7-(((2-Amino-4-thiazolyl)-((2,5-difluor-3,4-dihydroxyphenyl)methoxy)imino)acetyl)-amino)-2-carboxy-8-oxo-5-thia-1-azabicyclo[4,2,0]oct-2-en-3-yl)-2-propenyl)-chinolinium;
- inneres Salz von (6R-(3-(E) 6alpha, 7beta-(Z)))-1-(3-(7-(((2-Amino-4-thiazolyl)-((2,5-dichlor-3,4-dihydroxyphenyl)methoxy)imino)acetyl)-amino)-2-carboxy-8-oxo-5-thia-1-azabicyclo[4,2,0]oct-2-en-3-yl)-2-propenyl)-chinolinium;
- inneres Salz von (6R-(3-(E) 6alpha, 7beta-(Z)))-1-(3-(7-(((2-Amino-4-thiazolyl)-((2,5-difluor-3,4-dihydroxyphenyl)methoxy)imino)acetyl)-amino)-2-carboxy-8-oxo-5-thia-1-azabicyclo[4,2,0]oct-2-en-3-yl)-2-propenyl)-imidazo(1,2-a)-pyridinium;
- inneres Salz von (6R-(3-(E) 6alpha, 7beta-(Z)))-1-(3-(7-(((2-Amino-4-thiazolyl)-((2,5-dichlor-3,4-dihydroxyphenyl)methoxy)imino)acetyl)-amino)-2-carboxy-8-oxo-5-thia-1-azabicyclo[4,2,0]oct-2-en-3-yl)-2-propenyl)-imidazo(1,2-a)-pyridinium;
- inneres Salz von (6R-(3-(E) 6alpha, 7beta-(Z)))-1-(3-(7-(((2-Amino-4-thiazolyl)-((2,5-difluor-3,4-dihydroxyphenyl)methoxy)imino)acetyl)-amino)-2-carboxy-8-oxo-5-thia-1-azabicyclo[4,2,0]oct-2-en-3-yl)-2-propenyl)-6,7-dihydro-5H-1-pyridinium;
- inneres Salz von (6R-(3-(E) 6alpha, 7beta-(Z)))-1-(3-(7-(((2-Amino-4-thiazolyl)-((2,5-dichlor-3,4-dihydroxyphenyl)methoxy)imino)acetyl)-amino)-2-carboxy-8-oxo-5-thia-1-azabicyclo[4,2,0]oct-2-en-3-yl)-2-propenyl)-6,7-dihydro-5H-1-pyridinium;
- inneres Salz von (6R-(3-(E) 6alpha, 7beta-(Z)))-1-(3-(7-(((2-Amino-4-thiazolyl)-((5-fluor-3,4-dihydroxyphenyl)methoxy)-imino)acetyl)amino)-2-carboxy-8-oxo-5-thia-1-azabicyclo[4,2,0]oct-2-en-3-yl)-2-propenyl)-chinolinium.

9. Verfahren zur Herstellung der Verbindungen der Formel (I) nach Anspruch 1, **dadurch gekennzeichnet, daß** der aromatische Aldehyd der Formel (II): 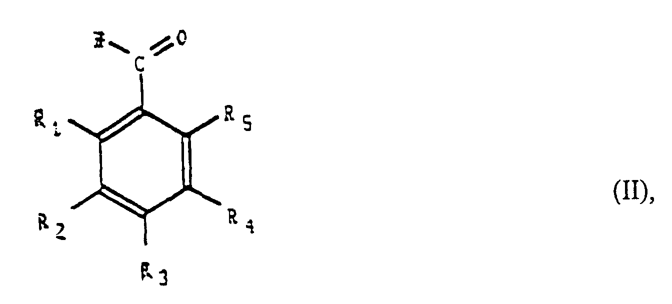
worin R₁, R₂, R₃, R₄ und R₅ die in Anspruch 1 definierten Bedeutungen aufweisen, erforderlichenfalls an den reaktiven Gruppen geschützt wird und so in den aromatischen Aldehyden der Formel (II)ₚ überführt wird: 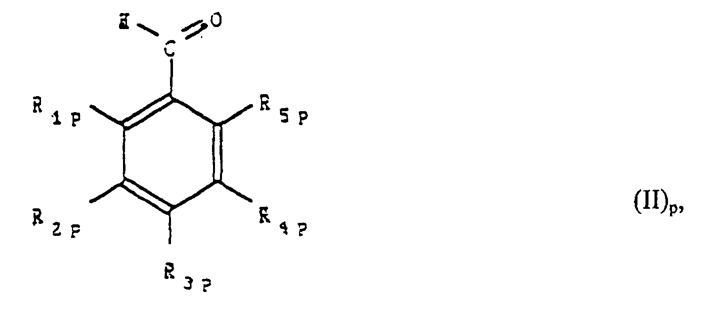
worin R₁ₚ, R₂ₚ, R₃ₚ, R₄ₚ und R₅ₚ die in Anspruch 1 definierten Gruppen R₁, R₂, R₃, R₄ bzw. R₅ oder eine reaktive Gruppe mit Schutzgruppe bedeuten;
der Aldehyd der Formel (II)ₚ mit einem Reduktionsmittel umgesetzt wird, wodurch der Alkohol der Formel (III) erhalten wird: 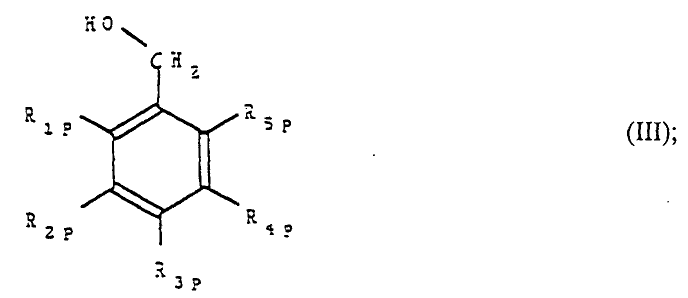
der Alkohol der Formel (III) gegebenenfalls in Gegenwart eines Aktivators mit N-Hydroxyphthalimid umgesetzt wird, wodurch das Derivat der Formel (IV) gebildet wird: 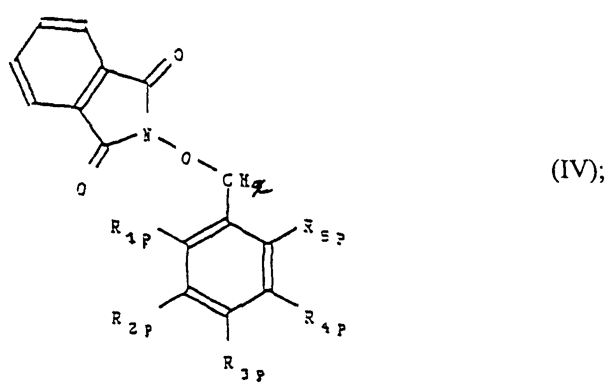
das Derivat der Formel (IV) zu dem O-substituierten Hydroxylamin der Formel (V) hydrolysiert wird: 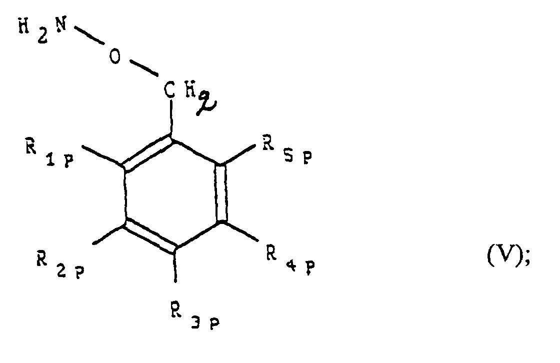
die Verbindung der Formel (V) mit einem (2-Amino-thiazol-4-yl)-2-oxoessigsäurederivat der Formel (VI) kondensiert wird: 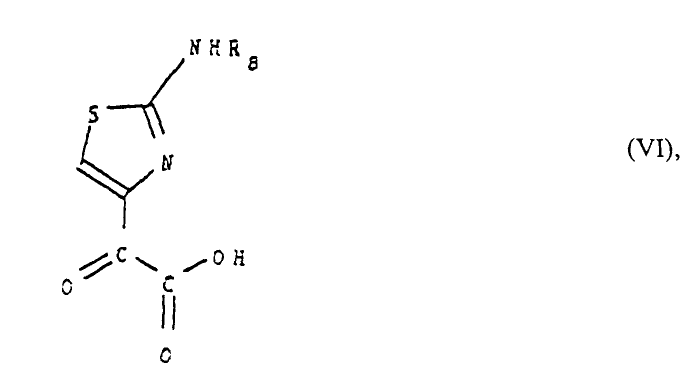
worin R₈ ein Wasserstoffatom oder eine Schutzgruppe für die Aminogruppe bedeutet, wodurch das "syn" alpha-Alkoxyimino-essigsäurederivat der Formel (VII) gebildet w 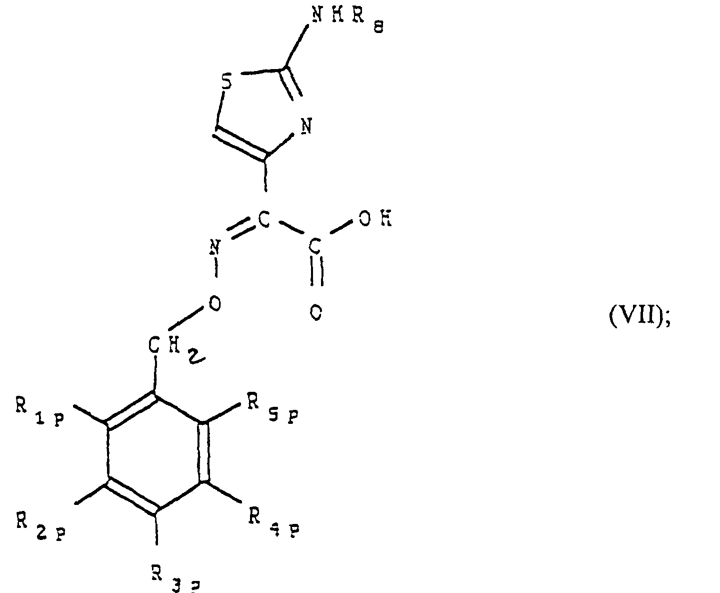 wobei gegebenenfalls ein funktionelles Derivat hergestellt wird;
die Verbindung der Formel (VII) oder ein funktionelles Derivat mit einem 7-Amino-3-(3-halogen-1-propenyl)-8-oxo-5-thia-1-azabicyclo[4,2,0]-oct-2-en-2-carbonsäureester-Hydrochlorid der Formel (VIII) amidiert wird: 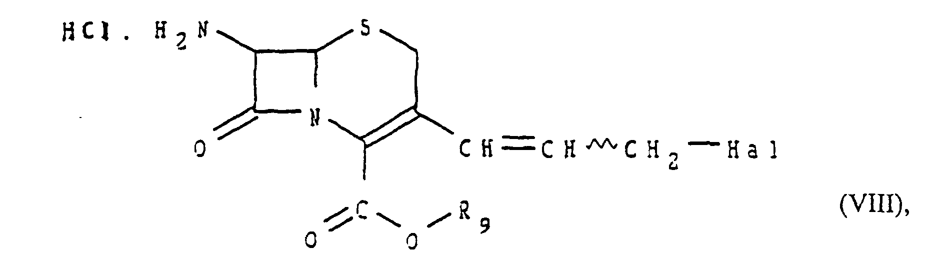
worin die Gruppe R₉ eine Gruppe bedeutet, die leicht von dem Ester abgespalten werden kann, wodurch das 7-(N-substituiertes Amido)-3-(3-halogen-1-propenyl)-8-oxo-5-thia-1-azabicyclo[4,2,0]oct-2-en-2-carbonsäurederivat der Formel (IX) gebildet wird: 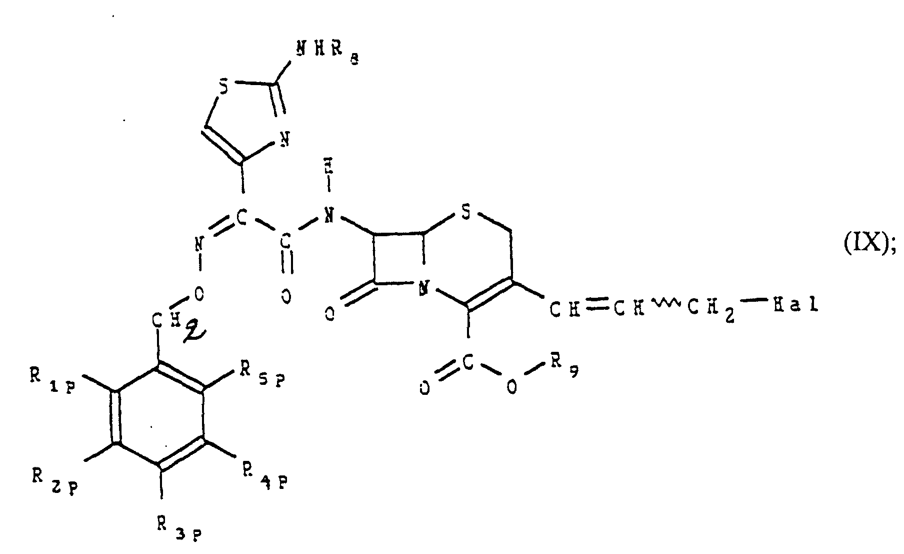
die Verbindung der Formel (IX) gegebenenfalls in die analoge 3-(3-Iodpropenyl)-Verbindung der Formel (X) umgewandelt wird: 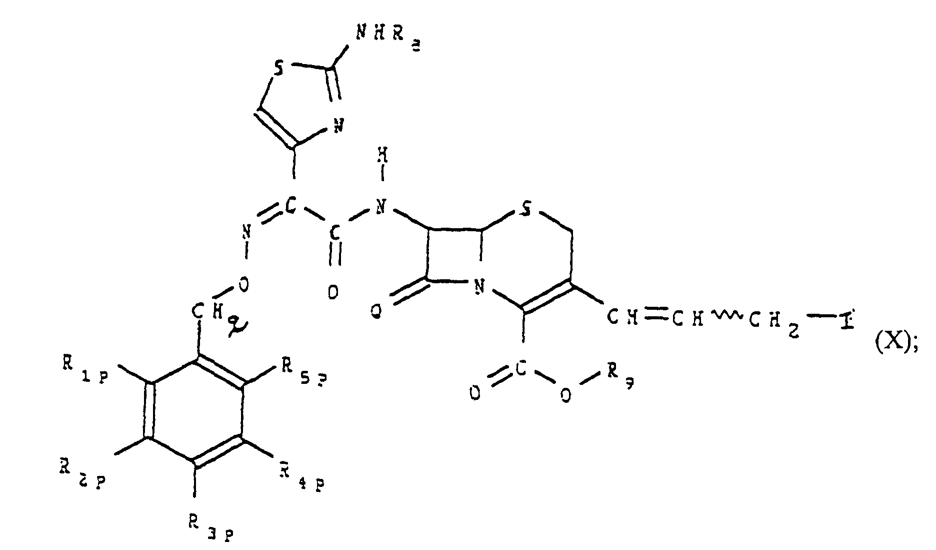
die Verbindung der Formel (X) mit einer Base der Formel R₆ umgesetzt wird, wodurch die Verbindung der Formel (XI) hergestellt wird: 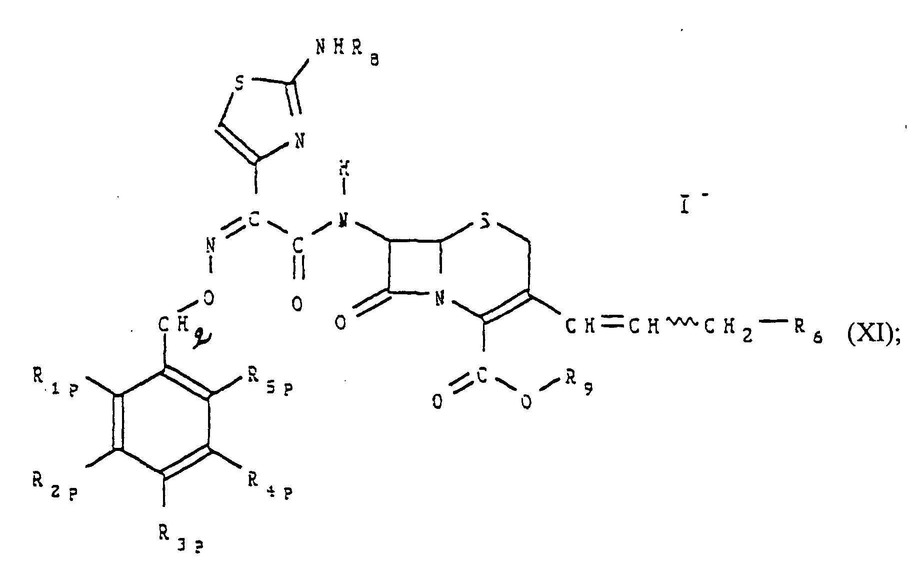
aus die Verbindung der Formel (XI) gegebenenfalls die (E)- oder (Z)-Isomere abgetrennt werden oder die (Z)-Isomere in (E)-Isomere überführt werden;
und die Verbindung der Formel (XI) in beliebiger Reihenfolge gegebenenfalls einer oder mehreren der folgenden Umsetzungen unterzogen wird:
a) hydrolytische Spaltung oder Spaltung durch Einwirkung von Thioharnstoff aller oder eines Teils der Esterschutzgruppen oder der Schutzgruppen der Aminogruppe oder der Hydroxygruppen;
b) Veresterung der Carboxygruppe oder Salzbildung mit der Carboxygruppe und einer Base;
c) Salzbildung mit der Aminogruppe und einer Säure;
d) Trennung der Verbindungen in Form des Gemisches R,S in R oder S.

10. Verfahren nach Anspruch 9, **dadurch gekennzeichnet, daß** das O-substituierte Hydroxylamin der Formel (V) mit der Verbindung der Formel (XII) kondensiert wird: 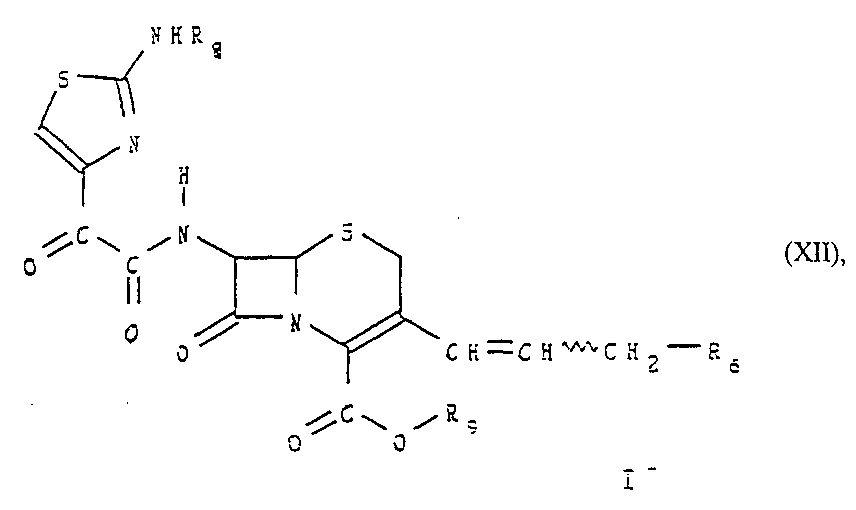 wodurch die Verbindung der Formel (XI) nach Anspruch 9 hergestellt wird.

11. Verbindungen der Formel (I) nach Anspruch 1, worin die Gruppen R₁, R₂, R₃, R₄, R₅, R₆ und A die in Anspruch 1 angegebenen Bedeutungen aufweisen, und ihre pharmazeutisch akzeptablen Säuresalze als Arzneimittel.

12. Verbindungen nach einem der Ansprüche 2 bis 8 sowie ihre pharmazeutisch akzeptablen Säuresalze als Arzneimittel.

13. Pharmazeutische Zusammensetzungen, die als Wirkstoff mindestens ein Arzneimittel nach einem der Ansprüche 11 oder 12 enthalten.

14. Verbindungen der Formel (IV) als neue technische Produkte:
worin die Gruppen R₁ₚ, R₂ₚ, R₃ₚ, R₄ₚ und R₅ₚ die in Anspruch 9 angegebenen Bedeutungen aufweisen.

15. Verbindungen der Formel (V) als neue technische Produkte: 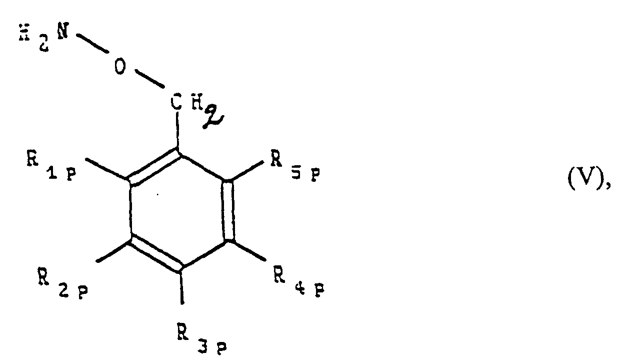 worin die Gruppen R₁ₚ, R₂ₚ, R₃ₚ, R₄ₚ und R₅ₚ die in Anspruch 9 angegebenen Bedeutungen aufweisen.

16. Verbindungen der Formel (VII) als neue technische Produkte: 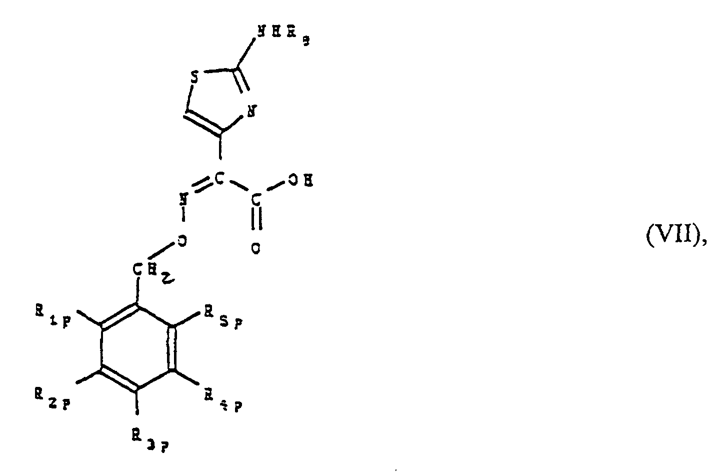 worin die Gruppen R₁ₚ, R₂ₚ, R₃ₚ, R₄ₚ, R₅ₚ und R₈ die in Anspruch 9 angegebenen Bedeutungen aufweisen.

17. Verbindungen der Formel (IX) als neue technische Produkte: 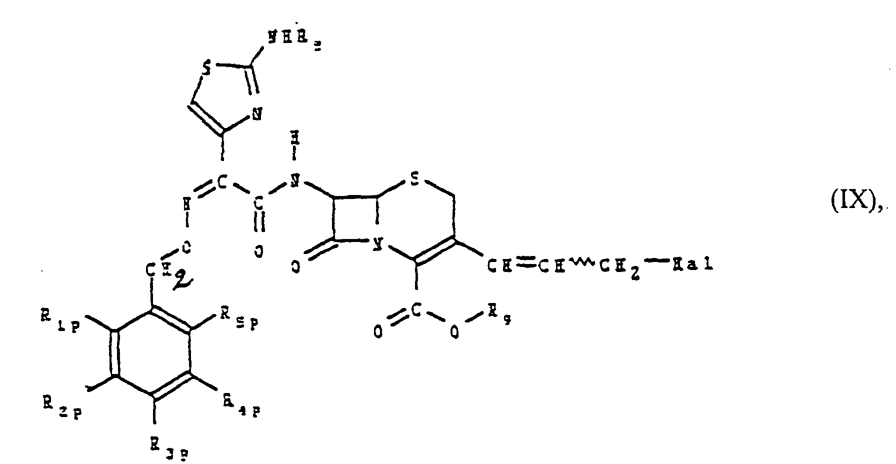 worin die Gruppen R₁ₚ, R₂ₚ, R₃ₚ, R₄ₚ, R₅ₚ, R₈ und R₉ die in Anspruch 9 angegebenen Bedeutungen aufweisen und Hal Halogen bedeutet.

18. Verbindungen der Formel (X) als neue technische Produkte gemäß Anspruch 17: 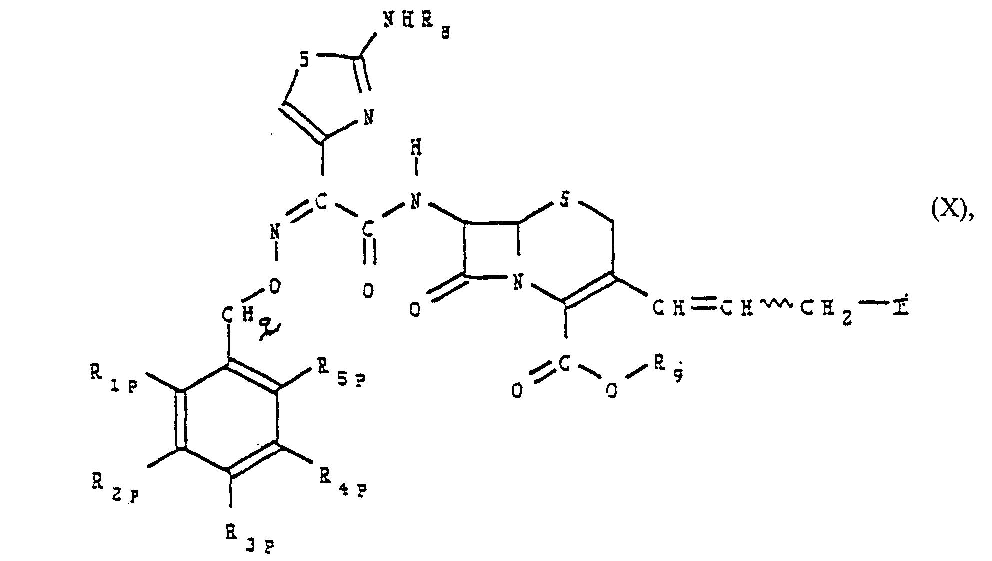 worin die Gruppen R₁ₚ, R₂ₚ, R₃ₚ, R₄ₚ, R₅ₚ, R₈ und R₉ die in Anspruch 9 angegebenen Bedeutungen aufiveisen.

19. Verbindungen der Formel (XI) als neue technische Produkte: 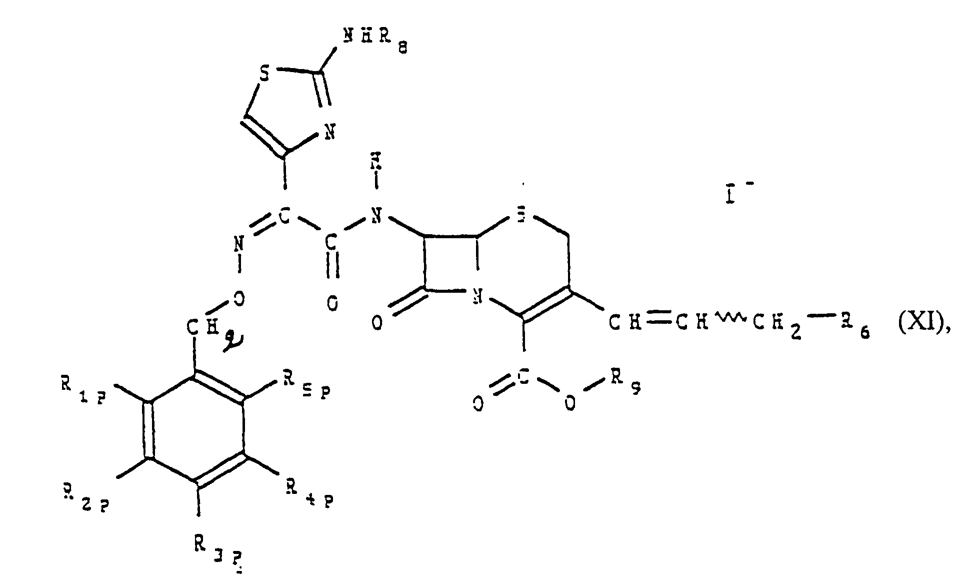 wobei die Gruppen R₁ₚ, R₂ₚ, R₃ₚ, R₄ₚ, R₅ₚ, R₈ und R₉ die in Anspruch 9 angegebenen Bedeutungen aufweisen und R₆ in Anspruch 1 definiert ist.

## Claims

1. The products of general formula (I): 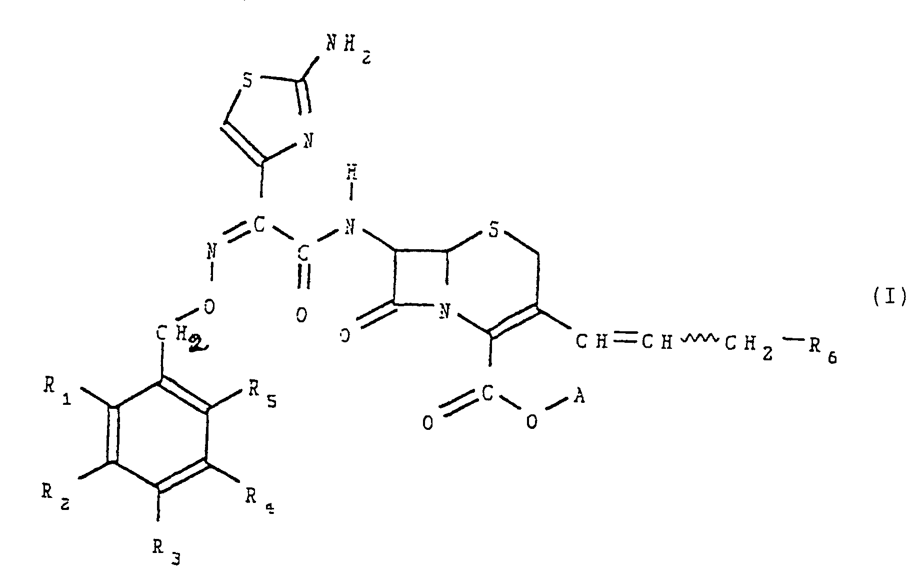
syn isomer, in the form of internal salts or salts with mineral or organic acids or with bases, in which formula: R₁, R₂, R₃ and R₅, identical or different, represent a hydrogen atom, a halogen atom or a radical chosen from the group constituted by the following radicals: hydroxy, alkyl containing 1 to 4 carbon atoms optionally substituted by one or more halogen atoms, alkyloxy containing 1 to 4 carbon atoms, mercapto, alkylthio containing 1 to 4 carbon atoms, nitro, cyano, amino, alkylamino containing 1 to 4 carbon atoms, dialkylamino containing 2 to 8 carbon atoms, carbamoyl, (alkylamino) carbonyl containing 2 to 5 carbon atoms, (dialkylamino) carbonyl containing 3 to 9 carbon atoms, carboxy, alkoxycarbonyl containing 2 to 5 carbon atoms, acyloxy containing 1 to 8 carbon atoms and 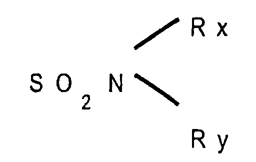 in which Rx and Ry, identical or different, represent a hydrogen atom or an alkyl radical containing 1 to 4 carbon atoms,
R₄ represents a hydroxy radical or an acyloxy radical containing 1 to 8 carbon atoms,
A represents a hydrogen atom, an equivalent of an alkali or alkaline-earth metal, magnesium, ammonium or an aminated organic base, or A represents the remainder of an easily cleavable ester group, or CO₂A represents CO₂⁻, the dotted line means that the CH₂R₆ group can be in the E or Z position and R₆ represents, in the quaternary ammonium form, one of the following radicals: 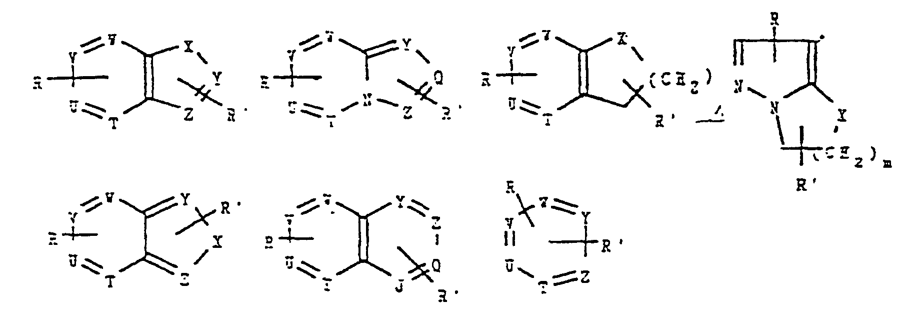 in which m is equal to 1, 2 or 3, X represents CH₂, NH, O or S;
Q, J, Y, T, U, V, W and Z, identical or different, represent independently of each other CH or N, it being understood that each of these cyclic radicals contains 1 to 5 heteroatoms, that at least one of these heteroatoms is the nitrogen atom and that these cyclic radicals can be substituted by one or more R or R' radicals;
R and R', identical or different, represent a hydrogen atom, an alkyl radical containing 1 to 4 carbon atoms, an alkyloxy radical containing 1 to 4 carbon atoms, a halogen atom, a cyano radical, a CO₂-Q₁, CO-NQ₁(Q₂), NQ₁(Q₂), SO₂-NQ₁(Q₂), CS-NH₂, NH-CO-Q₁, CH=N-OH, CH=N-O-Q₁, CH₂-CN, CH₂-S-Q₁, S-Q₁ radical, in which Q₁ and Q₂, identical or different, represent a hydrogen atom or an alkyl radical containing 1 to 4 carbon atoms.

2. The products of general formula (I) as defined in claim 1, in which R₆ represents one of the following radicals:

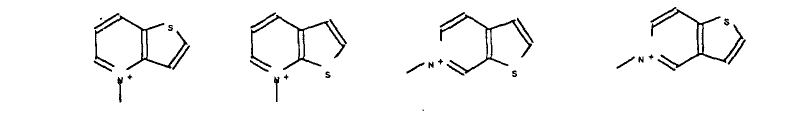

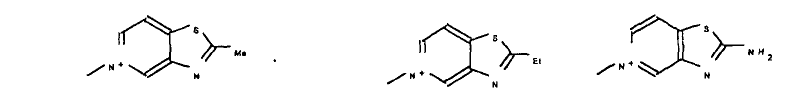

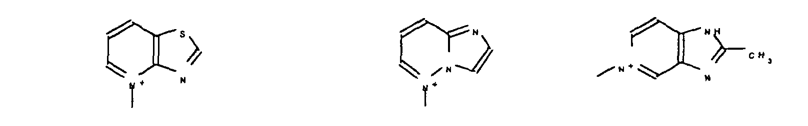

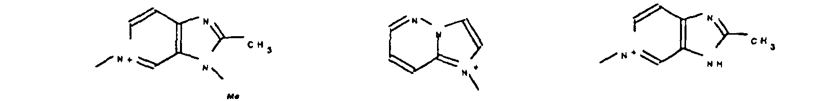

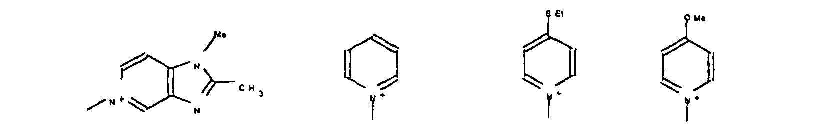

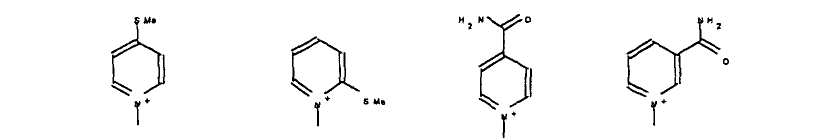

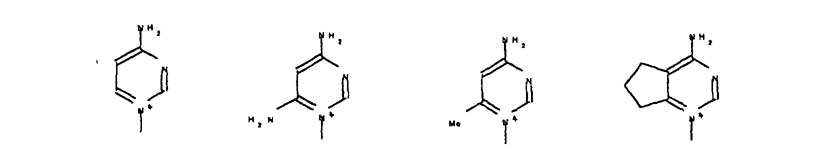

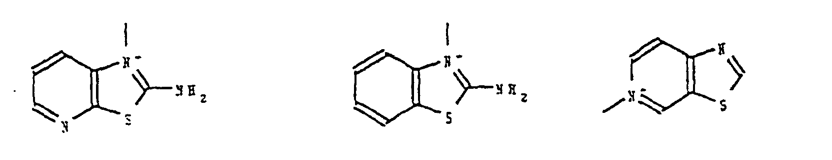

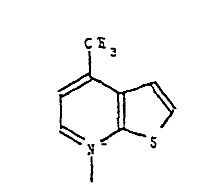

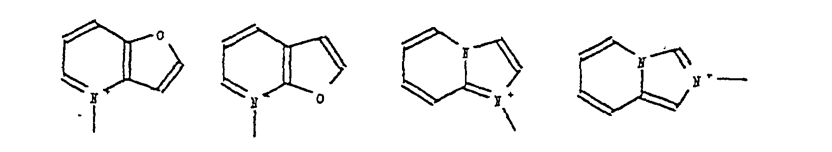

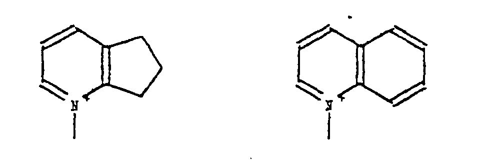

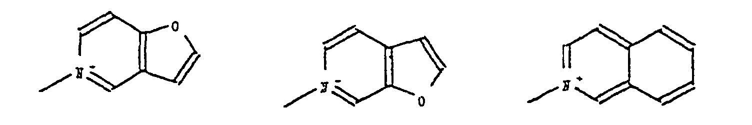

3. The products of general formula (I) as defined in claim 1 or 2, in which R₆ represents the quinolinium, isoquinolinium, 4-(methylthio) pyridinium, thieno[2,3-b]pyridinium, imidazo(1,2-a)pyridinium radical or the 6,7-dihydro-5H-1-pyrindinium radical.

4. The products of general formula (I) as defined in any one of claims 1, 2 or 3, in which R₃ and R₄ each represent a hydroxy radical.

5. The products of general formula (I) as defined in any one of claims 1, 2, 3 or 4, in which R₂ and R₅ each represent a chlorine or fluorine atom.

6. The products of general formula (I) as defined in any one of claims 1, 2, 3 or 4, in which R₂ represents a fluorine atom.

7. The products of general formula (I) as defined in any one of claims 1, 2, 3 or 4, in which R₂ represents a methoxy radical and one of R₁ or R₅ represents a chlorine atom.

8. The products of general formula (I) according to claim 1 the names of which follow:
- the internal salt of (6R-(3-(E) 6alpha, 7beta-(Z))) 1-(3-(7-(((2-amino 4-thiazolyl) ((2,5-difluoro 3,4-dihydroxyphenyl) methoxy) imino) acetyl) amino) 2-carboxy 8-oxo 5-thia 1-azabicyclo[4,2,0]oct-2-en-3-yl) 2-propenyl) quinolinium,
- the internal salt of (6R-(3-(E) 6alpha, 7beta-(Z))) 1-(3-(7-(((2-amino 4-thiazolyl) ((2,5-dichloro 3,4-dihydroxyphenyl) methoxy) imino) acetyl) amino) 2-carboxy 8-oxo 5-thia 1-azabicyclo[4,2,0]oct-2-en-3-yl) 2-propenyl) quinolinium,
- the internal salt of (6R-(3-(E) 6alpha, 7beta-(Z))) 1-(3-(7-(((2-amino 4-thiazolyl) ((2,5-difluoro 3,4-dihydroxyphenyl) methoxy) imino) acetyl) amino) 2-carboxy 8-oxo 5-thia 1-azabicyclo[4,2,0]oct-2-en-3-yl) 2-propenyl) imidazo(1,2-a) pyridinium,
- the internal salt of (6R-(3-(E) 6alpha, 7beta-(Z))) 1-(3-(7-(((2-amino 4-thiazolyl) (((2,5-dichloro 3,4-dihydroxyphenyl) methoxy) imino) acetyl) amino) 2-carboxy 8-oxo 5-thia 1-azabicyclo[4,2,0]oct-2-en-3-yl) 2-propenyl) imidazo(1,2-a) pyridinium,
- the internal salt of (6R-(3-(E) 6alpha, 7beta-(Z))) 1-(3-(7-(((2-amino 4-thiazolyl) (((2,5-difluoro 3,4-dihydroxyphenyl) methoxy) imino) acetyl) amino) 2-carboxy 8-oxo 5-thia 1-azabicyclo[4,2,0]oct-2-en-3-yl) 2-propenyl) 6,7-dihydro 5H-1-pyrindinium,
- the internal salt of (6R-(3(E), 6alpha, 7beta(Z))) 1-(3-(7-(((2-amino 4-thiazolyl) ((2,5-dichloro 3,4-dihydroxyphenyl) methoxy) imino) acetyl) amino) 2-carboxy 8-oxo 5-thia 1-azabicyclo[4,2,0]oct-2-en-3-yl) 2-propenyl) 6,7-dihydro 5H-1-pyrindinium,
- the internal salt of (6R-(3(E), 6alpha, 7beta(Z))) 1-(3-(7-(((2-amino 4-thiazolyl) ((5-fluoro 3,4-dihydroxyphenyl) methoxy) imino) acetyl) amino) 2-carboxy 8-oxo 5-thia 1-azabicyclo[4,2,0]oct-2-en-3-yl) 2-propenyl) quinolinium.

9. Process for the preparation of the products of formula (I) as defined in claim 1, **characterized in that** the aromatic aldehyde of formula (II): 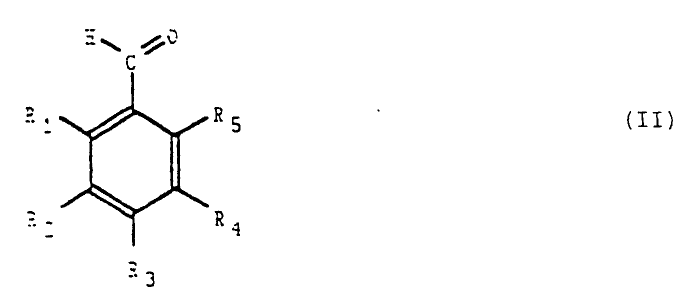
in which R₁, R₂, R₃, R₄ and R₅ are as defined in claim 1, if necessary its reactive functions are protected and thus converted to an aromatic aldehyde of formula (IIₚ): 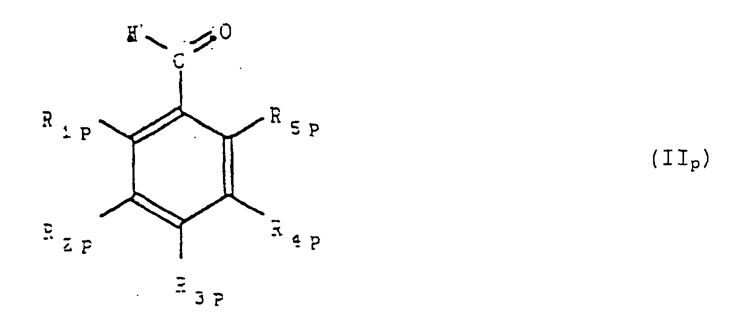
in which R₁ₚ, R₂ₚ, R₃ₚ, R₄ₚ and R₅ₚ respectively represent the values of R₁, R₂, R₃, R₄ and R₅ as defined previously or a protected reactive function, said aldehyde of formula (IIₚ) is treated with a reducing agent, in order to obtain the alcohol of formula (III): 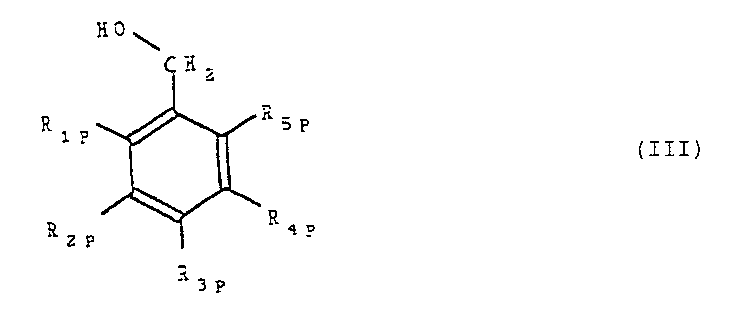
which alcohol of formula (III) is treated with N-hydroxy phthalimide, if appropriate in the presence of an activating agent, in order to obtain the derivative of formula (IV): 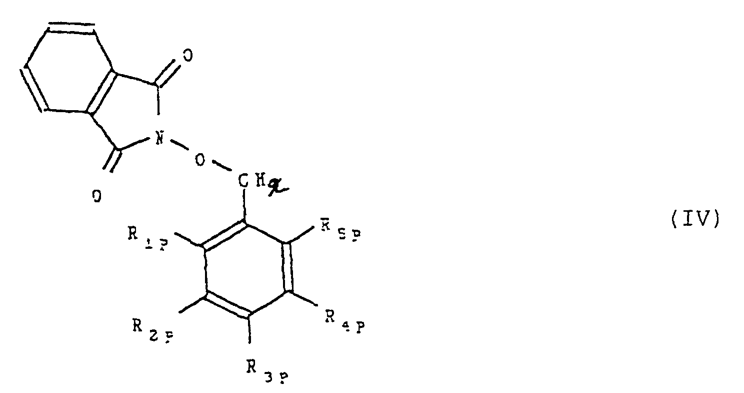
which is hydrolyzed to the 0-substituted hydroxylamine of formula (V): 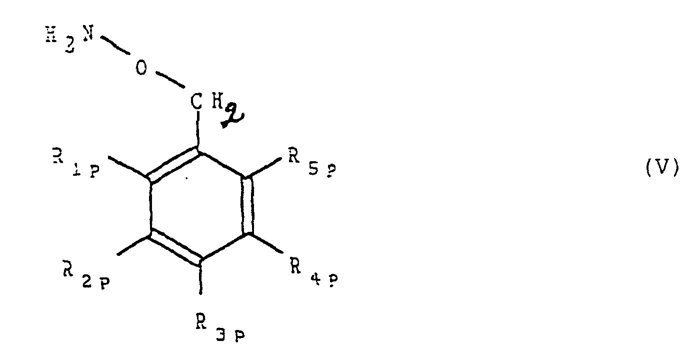
which is condensed with a derivative of 2-(2-amino thiazol-4-yl) 2-oxo acetic acid of formula (VI): 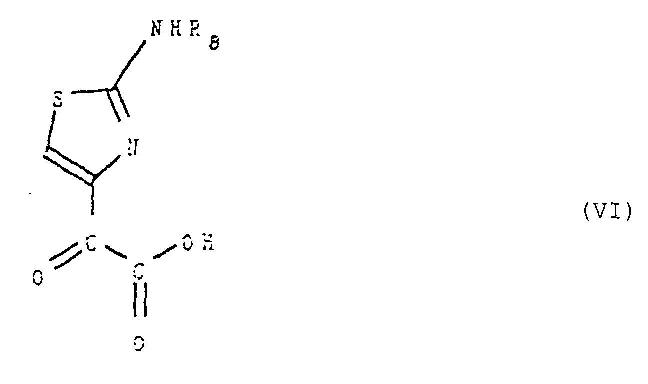
in which R₈ represents a hydrogen atom or a protective group of the amine function, in order to form the derivative of "syn" alpha-alkoxyimino acetic acid of formula (VII): 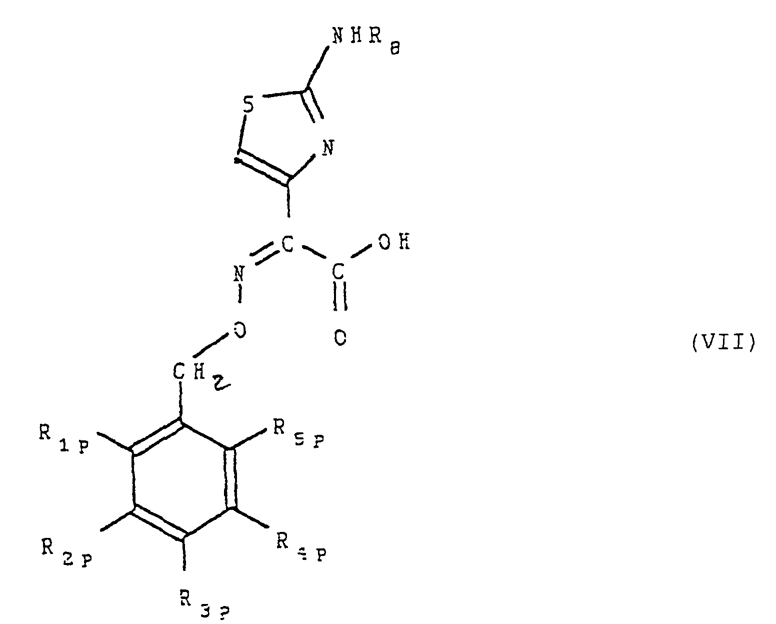
of which there is prepared if appropriate, a functional derivative, a product of formula (VII) or a functional derivative which is amidified with an ester of the hydrochloride of 7-amino 3-(3-halo 1-propenyl) 8-oxo 5-thia 1-azabicyclo[4,2,0]oct-2-en-2-carboxylic acid of formula (VIII): 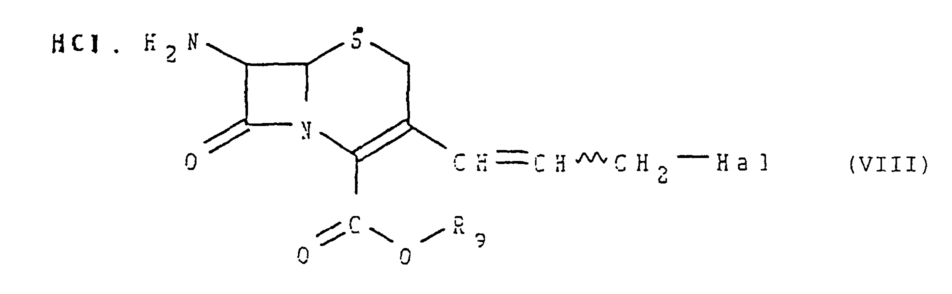
in which R₉ represents the remainder of an easily cleavable ester, in order to produce the derivative of the 7-(N-substituted amido) 3-(3-halo 1-propenyl) 8-oxo 5-thia 1-azabicyclo[4,2,0]oct-2-en-2-carboxylic acid of formula (IX): 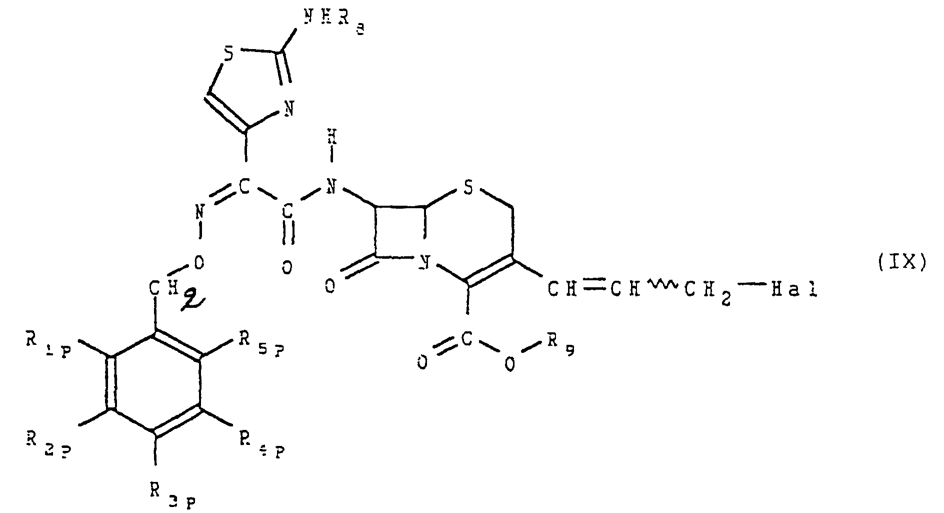
which is converted, if appropriate, to the analogous 3-(3-iodo propenyl) of formula (X): 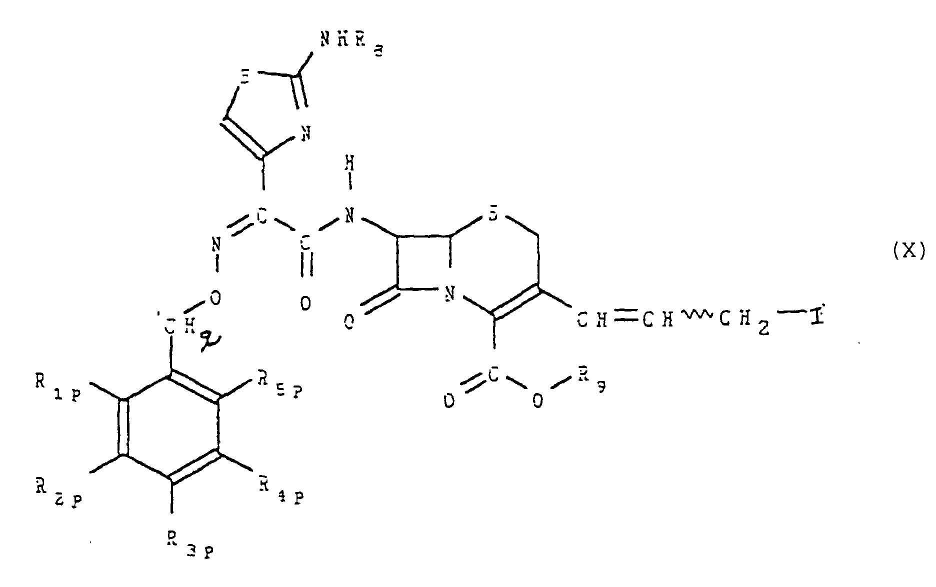
which is treated with a base of formula R₆ in order to obtain the product of formula (XI): 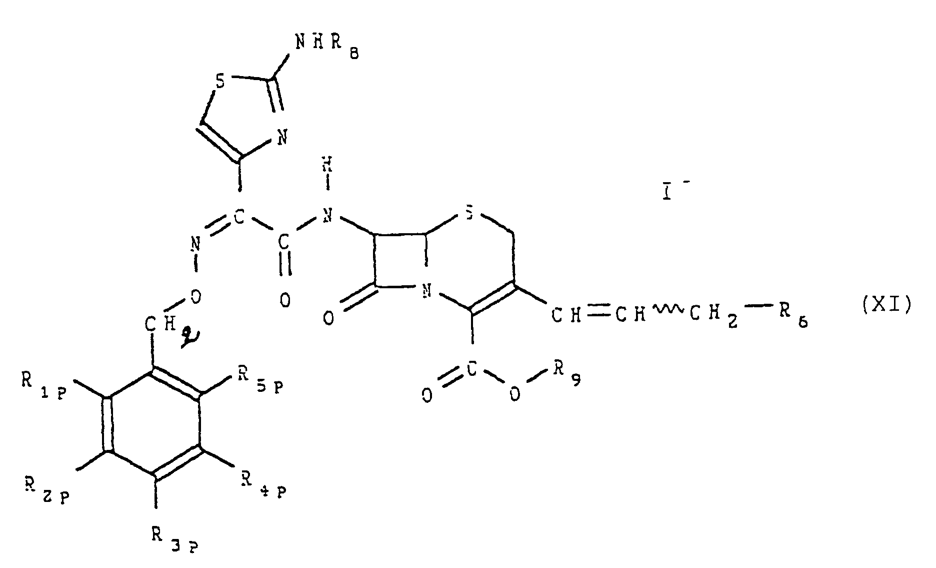
from which product of formula (XI) if appropriate, the (E) or (Z) isomers are isolated or the (Z) isomers are converted to (E) isomers, which product of formula (XI) is subjected if appropriate, to one or more of the following reactions in an appropriate order:
a) cleavage by hydrolysis or by the action of the thiourea of all or part of the ester protective groups and of the protective groups of the amino radical or of the hydroxyl radicals,
b) esterification or salification by a base of the carboxylic radical,
c) salification by an acid of the amino radical,
d) separation of the products in the form of an R,S mixture to R and S.

10. Variant of the process according to claim 9, **characterized in that** the 0-substituted hydroxylamine of formula (V) is condensed with the product of formula (XII): 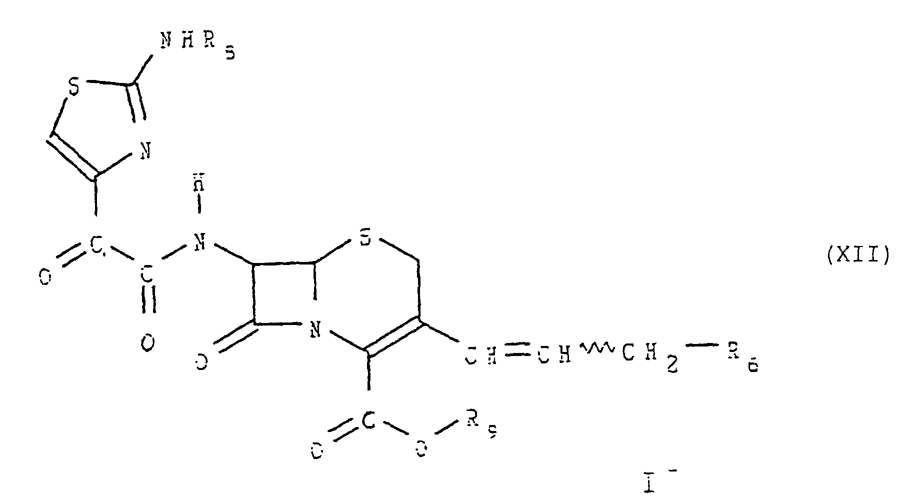 in order to produce the product of formula (XI) as defined in claim 9.

11. As medicaments, the products corresponding to formula (I) as defined in claim 1, in which R₁, R₂, R₃, R₄, R₅, R₆ and A are as defined in claim 1, as well as their pharmaceutically acceptable acid salts.

12. As medicaments, the products as defined in any one of claims 2 to 8, as well as their pharmaceutically acceptable acid salts.

13. Pharmaceutical compositions containing at least one medicament according to one of claims 11 or 12, as active ingredient.

14. As new industrial products, the products of formula (IV): 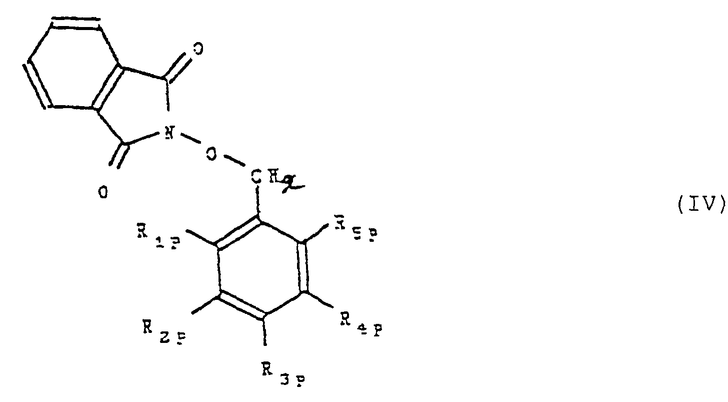 in which R₁ₚ, R₂ₚ, R₃ₚ, R₄ₚ and R₅ₚ are as defined in claim 9.

15. As new industrial products, the products of formula (V): 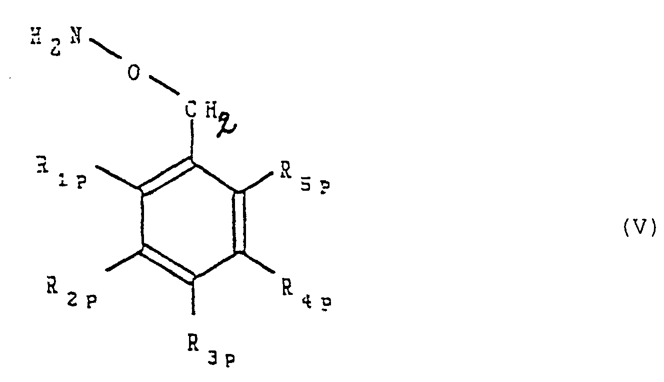 in which R₁ₚ, R₂ₚ, R₃ₚ, R₄ₚ and R₅ₚ are as defined in claim 9.

16. As new industrial products, the products of formula (VII): 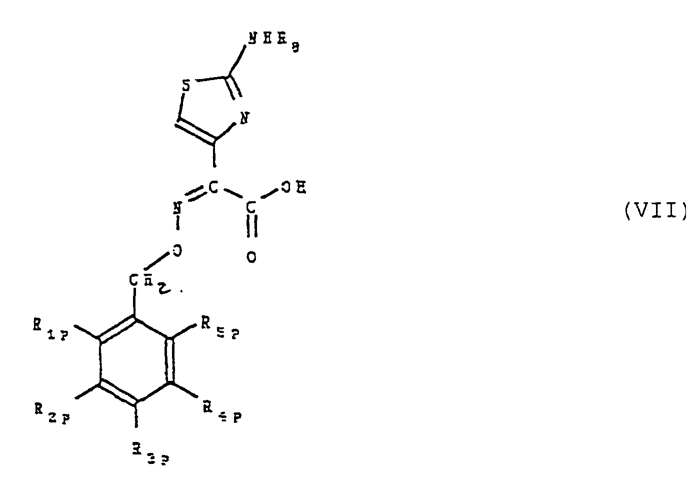 in which R₁ₚ, R₂ₚ, R₃ₚ, R₄ₚ, R₅ₚ and R₈ are as defined in claim 9.

17. As new industrial products, the products of formula (IX): 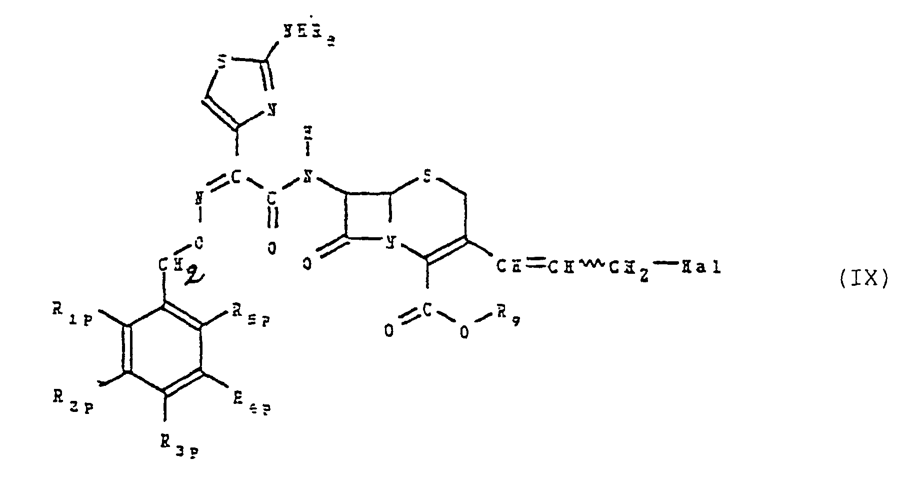 in which R₁ₚ, R₂ₚ, R₃ₚ, R₄ₚ, R₅ₚ, R₈ and R₉ are as defined in claim 9 and Hal represents a halogen atom.

18. As new industrial products according to claim 17, the products of formula (X): 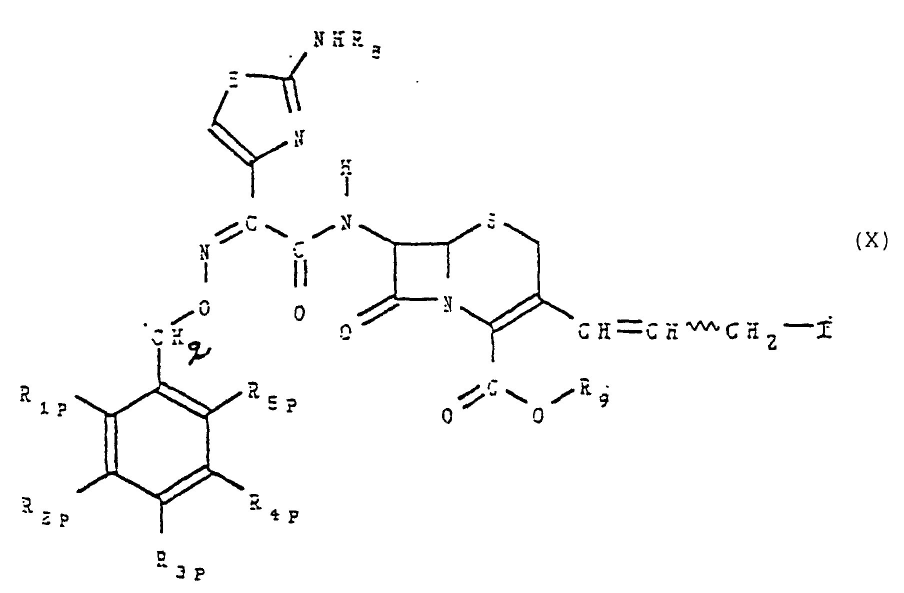 in which R₁ₚ, R₂ₚ, R₃ₚ, R₄ₚ, R₈ and R₉ are as defined in claim 9.

19. As new industrial products, the products of formula (XI): 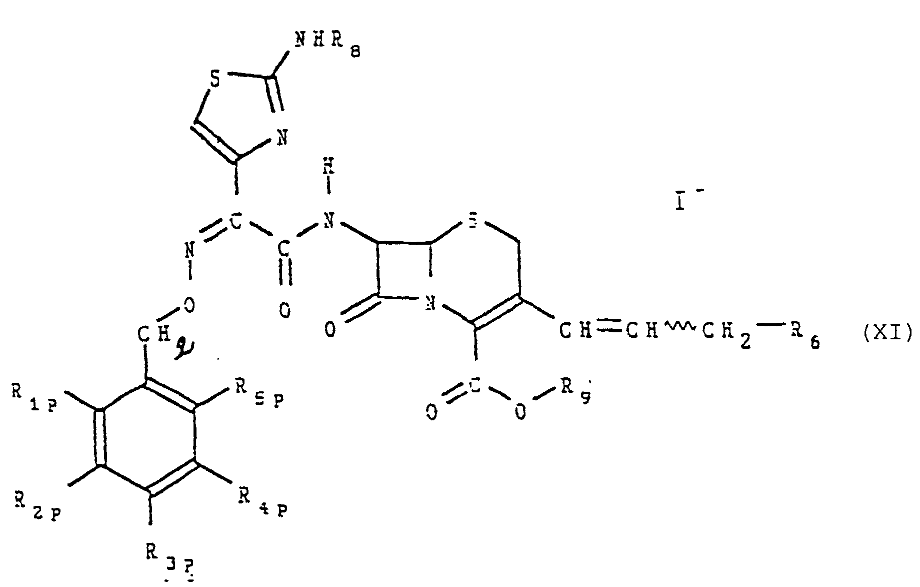 in which R₁ₚ, R₂ₚ, R₃ₚ, R₄ₚ, R₈ and R₉ are as defined in claim 9 and R₆ is as defined in claim 1.
